(19) Europäisches Patentamt
European Patent Office
Office européen des brevets

(11) EP 0 656 348 B1

# (12) EUROPÄISCHE PATENTSCHRIFT

(45) Veröffentlichungstag und Bekanntmachung des Hinweises auf die Patenterteilung:
**03.05.2000 Patentblatt 2000/18**

(21) Anmeldenummer: **94118645.4**

(22) Anmeldetag: **26.11.1994**

(51) Int. Cl.$^7$: **C07C 257/18**, C07C 271/62, C07C 259/18, A61K 31/22, C07D 211/46, C07D 211/58, C07D 207/12, C07F 9/24, C07D 401/12, C07D 211/34, A61K 31/395, C07D 309/12, C07D 213/82, C07D 413/06, C07D 271/06, C07D 413/10, C07D 217/02, C07C 323/42

## (54) Essigsäurederivate als Arzneimittel

Aceric acid derivatives as medicaments

Dérivés d'acide acérique comme médicaments

(84) Benannte Vertragsstaaten:
**AT BE CH DE DK ES FR GB GR IE IT LI LU MC NL PT SE**
Benannte Erstreckungsstaaten:
**LT SI**

(30) Priorität: **03.12.1993 CH 360993**
**25.10.1994 CH 319894**

(43) Veröffentlichungstag der Anmeldung:
**07.06.1995 Patentblatt 1995/23**

(73) Patentinhaber:
**F. HOFFMANN-LA ROCHE AG**
**4002 Basel (CH)**

(72) Erfinder:
- **Alig, Leo**
  **CH-4303 Kaiseraugst (CH)**
- **Hadvary, Paul**
  **CH-4105 Biel-Benken (CH)**
- **Hürzeler Müller, Marianne**
  **CH-4658 Däniken (CH)**
- **Müller, Marcel**
  **CH-4402 Frenkendorf (CH)**
- **Steiner, Beat**
  **CH-4112 Bättwil (CH)**
- **Weller, Thomas**
  **CH-4051 Basle (CH)**

(74) Vertreter: **Mahé, Jean et al**
**F.Hoffmann-La Roche AG**
**Patent Department (PLP),**
**124 Grenzacherstrasse**
**4070 Basel (CH)**

(56) Entgegenhaltungen:
**EP-A- 0 372 486** **EP-A- 0 381 033**
**EP-A- 0 505 868** **EP-A- 0 632 020**
**EP-A- 0 641 770** **WO-A-94/15913**
**WO-A-95/01336**

- **JOURNAL OF MEDICINAL CHEMISTRY, Bd. 35, Nr. 23, 13.November 1992 WASHINGTON US, Seiten 4393-4407, LEO ALIG ET AL. 'Low molecular weight, non-peptide fibrinogen receptor antagonists'**

Bemerkungen:
Die Akte enthält technische Angaben, die nach dem Eingang der Anmeldung eingereicht wurden und die nicht in dieser Patentschrift enthalten sind.

EP 0 656 348 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft neue Essigsäurederivate, Verfahren zu deren Herstellung, pharmazeutische Präparate, die solche Verbindungen enthalten, sowie die Verwendung dieser Verbindungen bei der Herstellung pharmazeutischer Präparate.

Die Erfindung betrifft insbesondere Essigsäurederivate der Formel

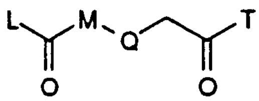

I

worin

L eine Gruppe der Formeln $L^1$ bis $L^5$:

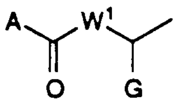

$L^1$

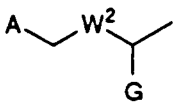

$L^2$

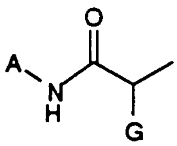

$L^3$

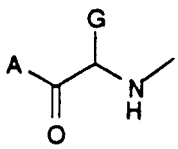

$L^4$

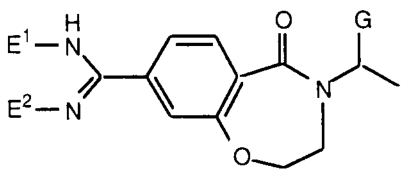

$L^5$

wobei eine in der Gruppe L und/oder zwischen den Gruppen L und M enthaltene nicht amidartig gebundene Carbonylgruppe auch als Oxim vorliegen kann,

A eine Gruppe der Formeln $A^1$ bis $A^4$:

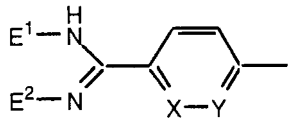

A[1]

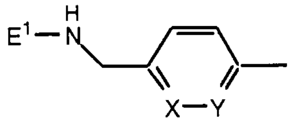

A[2]

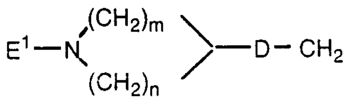

A[3]

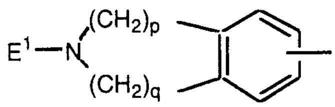

A[4]

| | |
|---|---|
| $E^1$ und $E^2$ | H, nieder-Alkyl, OH, nieder-Alkoxy, nieder-Alkoxy-nieder alkyl, Carboxy-niederalkyl, P(O)(O-nieder-Alkyl)$_2$, C(O)OR$^1$, OC(O)R$^1$, OC(O)OR$^1$ oder C(O)SR$^1$, wobei zumindest eins von $E^1$ und $E^2$ H ist oder |
| $E^1$ und $E^2$ | zusammen mit den N-Atomen, an die sie gebunden sind, eine (5,5-Dimethyl- oder 5-Oxo)-4,5-dihydro-1,2,4-oxadiazol-3-ylgruppe sind, |
| $R^1$ | nieder-Alkoxy-niederalkyl, nieder-Alkyl, durch OH, COOH, nieder-Alkoxycarbonyl, nieder-Alkanoyloxy, nieder-Alkenoyl oxy, durch gegebenenfalls (durch nieder-Alkanoyloxy) substituiertes Benzoyloxy oder durch nieder-Alkyl-CONH substituiertes nieder-Alkyl, oder gegebenenfalls (durch Halogen oder nieder-Alkoxy) substituiertes und gegebenenfalls über nieder-Alkylen gebundenes Phenyl, oder gegebenenfalls durch O unterbrochenes Cycloalkyl, |
| eins von X und Y | CH und das andere CH, C-nieder-Alkyl, C-nieder-Alkoxy oder N, |
| D | eine Gruppe $(CH_2)_s$ oder $(CH_2)_tO$, |
| s | 1 bis 4, |
| m und n | 0 bis 5 und |
| t | 0 bis 3, jedoch m + n 1 bis 5 und jedes von m+ t und n + t mindestens 1, |
| p und q | 0 bis 5, jedoch p + q 2 bis 5, |
| $W^1$ | $CH_2$, nieder Alkyl-CH, nieder-Alkyl-OC(O)CH, NH, nieder-Alkyl-N oder nieder-Alkoxy-niederalkyl-N, |
| $W^2$ | O, NH, Acyl-N oder nieder Alkyl-OC(O)-N, |
| G | H oder gegebenenfalls durch OH, SH, $CONH_2$, CONH-nieder-Alkyl, nieder-Alkylthio, Aryl, $NH_2$, |

NH-$R^a$, N($R^a$,$R^b$) oder $OR^a$ substituiertes nieder-Alkyl,

$R^a$ und $R^b$ nieder-Alkyl, nieder-Alkoxy-niederalkyl, Acyl oder nieder-Alkoxycarbonyl,

M über das N-Atom an die Ketogruppe gebundenes 1,4-Piperidinylen oder gegebenenfalls durch nieder-Alkyl, nieder-Alkoxy, $OCH_2COOH$ oder $OCH_2COO$-nieder-Alkyl substituiertes 1,4-Phenylen,

Q O, $CH_2$, NH, Acyl-N oder nieder-Alkyl OC(O)N ist,

T $NH_2$, NH-nieder-alkyl, NH-nieder-Alkyl-COOH, NH-nieder-Alkyl-COO-nieder Alkyl, durch nieder-Alkoxy, COOH, COO-nieder-Alkyl, nieder-Alkyl-COO oder nieder-Alkyl-OCOO substituiertes nieder-Alkyloxy oder nieder-Alkenyloxy oder eine Gruppe OT',

T' H, nieder-Alkyl, gegebenenfalls über nieder-Alkylen gebundenes Phenyl oder Pyridyl oder gegebenenfalls über nieder-Alkylen gebundenes und gegebenenfalls durch O, NH oder NCOO-nieder-Alkyl unterbrochenes Cycloalkyl ist, mit den Massgaben, dass

a)

T' von H, nieder-Alkyl und Phenyl-niederalkyl verschieden ist, falls

L eine Gruppe der Formel

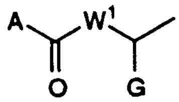

$L^1$

A eine Gruppe der Formel

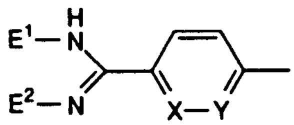

$A^1$

eins von $E^1$ und $E^2$ Wasserstoff und das andere Wasserstoff, tert-Butoxycarbonyl oder Benzyloxycarbonyl,

eins von X und Y CH und das andere CH oder N und

$W^1$ NH, nieder-Alkyl-N oder nieder-Alkoxy-niederalkyl-N ist,

G die obige Bedeutung hat,

M über das N-Atom an die Ketogruppe gebundenes 1,4-Piperidinylen, und

Q O ist und dass

b)

T' von H, nieder-Alkyl, Phenyl und Phenyl-niederalkyl, verschieden ist, falls

L eine Gruppe der Formel $L^{11}$, $L^{31}$ oder $L^{41}$:

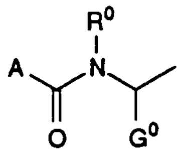

$L^{11}$

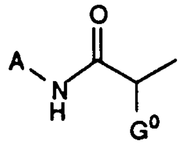

$L^{31}$

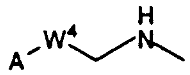

$L^{41}$

A eine Gruppe der Formel

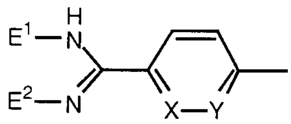

$A^1$

| | |
|---|---|
| eins von $E^1$ und $E^2$ | Wasserstoff und das andere Wasserstoff, tert-Butoxycarbonyl oder Benzyloxycarbonyl, |
| eins von X und Y | CH und das andere CH, C-nieder-Alkyl, C-nieder-Alkoxy oder N, |
| $R^o$ und $G^o$ | H oder nieder-Alkyl, |
| $W^4$ | C=O oder C=NOH, |
| M | gegebenenfalls durch nieder-Alkyl, nieder-Alkoxy, $OCH_2COOH$ oder $OCH_2COO$-nieder-Alkyl substituiertes 1,4-Phenylen und |
| Q | O, $CH_2$ oder NH ist, |
| c) | A nur dann eine Gruppe der Formel $A^3$ sein kann, wenn L eine Gruppe der Formel $L^1$ und M 1,4-Phenylen ist, und |
| d) | G nicht Wasserstoff ist, wenn L eine Gruppe der Formel $L^1$ und A eine Gruppe der Formel $A^3$ oder $A^4$ ist, |

sowie Hydrate oder Solvate und physiologisch verwendbare Salze davon.

Die Essigsäurederivate der Formel I unterscheiden sich strukturell von denjenigen in EP-A-0 381 033, EP-A-0 505 868, EP-A-0 641 770 und J. Med. Chem. 35, 4393-4407, 1992, sowie von den Dipiperidinderivaten in EP-A-0 706 999.

Im Rahmen der vorliegenden Erfindung bezeichnet "nieder" geradkettige oder verzweigte Gruppen mit 1 bis 6, vorzugsweise 1 bis 4 C-Atomen. So sind Methyl, Aethyl, Propyl, Isopropyl, n-, s- und t-Butyl und Hexyl Beispiele für nieder-Alkyl; Methoxy und Aethoxy für nieder-Alkoxy; Acetyl und Propionyl für nieder-Alkanoyl. Methacryloyl ist ein Beispiel für nieder-Alkenoyl und Pentenyloxy für nieder-Alkenyloxy.

Beispiele von Substituenten einer Phenylgruppe $R^1$ sind Halogen und nieder-Alkoxy. Weitere Beispiele von Gruppen $R^1$ sind Benzyl und gegebenenfalls durch O unterbrochenes $C_{3-6}$-Cycloalkyl, wie Tetrahydropyranyl. Beispiele von Substituenten einer in $R^1$ enthaltenen Benzoylgruppe sind nieder-Alkanoyloxygruppen, wie Acetoxy.

Beispiele von gegebenenfalls über nieder-Alkylen gebundene und gegebenenfalls durch O, NH oder NCOO-nieder-Alkyl unterbrochene Cycloalkylgruppen T' sind solche mit 3 bis 6 C-Atomen im cyclischen Anteil, wie Cyclopropylmethyl, Cyclohexyl, Tetrahydropyranyl, Piperidinyl und N-(t-Butoxycarbonyl)piperidinyl.

Beispiele von Gruppen G sind Methyl, Isopropyl, Benzyl, p-Hydroxybenzyl, $CH_2SH$, $CH_2OH$, 1-Hydroxyäthyl und dergleichen. Ferner ist G beispielsweise eine gegebenenfalls durch OH, SH, nieder-Alkylthio, Aryl, $NH_2$, NH-$R^a$, N($R^a$, $R^b$) oder $OR^a$ substituierte nieder-Alkylgruppe, worin $R^a$ und $R^b$ nieder-Alkyl, nieder-Alkoxy-niederalkyl, Acyl oder nieder-Alkoxy-carbonyl sind. Eine nieder-Alkylgruppe G kann ferner durch $CONH_2$ oder CONH-nieder-Alkyl substituiert sein. Das obige Aryl ist z.B. Phenyl oder durch OH, $NH_2$, NH-$R^a$, N($R^a$, $R^b$) oder $OR^a$ substituiertes Phenyl. Das obige Acyl ist z.B. nieder-Alkanoyl, Aroyl oder Heteroaroyl, wobei Aroyl eine über CO gebundene, wie oben definierte Arylgruppe, wie Benzoyl oder nieder-Alkanoyloxybenzoyl, und Hetereoaroyl eine über CO gebundene, z.B. 5 bis 6 gliedrige, O oder NH enthaltende heteroaromatische Gruppe, wie Furoyl ist.

Die Verbindungen der Formel I können solvatisiert, insbesondere hydratisiert sein. Die Hydratisierung kann im Zuge des Herstellungsverfahrens erfolgen oder allmählich als Folge hygroskopischer Eigenschaften einer zunächst wasserfreien Verbindung der Formel I auftreten.

Beispiele von physiologisch verwendbaren Salzen der Verbindungen der Formel I sind Salze mit physiologisch verträglichen Mineralsäuren, wie Salzsäure, Schwefelsäure oder Phosphorsäure; oder mit organischen Säuren, wie Methansulfonsäure, Essigsäure, Trifluoressigsäure, Zitronensäure, Fumarsäure, Maleinsäure, Weinsäure, Bernsteinsäure oder Salicylsäure. Die Verbindungen der Formel I mit freier Carboxygruppe können auch Salze mit physiologisch verträglichen Basen bilden. Beispiele solcher Salze sind Alkalimetall-, Erdalkalimetall-, Ammonium- und Alkylammoniumsalze, wie das Na-, K-, Ca- oder Tetramethylammoniumsalz. Die Verbindungen der Formel I können auch in Form von Zwitterionen vorliegen.

Die Verbindungen der Formel I, die ein oder mehrere asymmetrische C-Atome enthalten, können als Enantiomere, als Diastereomere oder als Gemische davon, z.B. als Racemate, vorliegen.

Die erfindungsgemässen Verbindungen können in folgende Gruppen aufgeteilt werden:

a) diejenigen, worin L eine Gruppe $L^1$, in der A eine Gruppe $A^1$ ist und der Formel

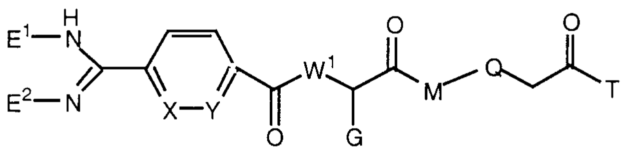

I-A

worin $E^1$, $E^2$, X, Y, $W^1$, G, M, Q und T obige Bedeutung haben,

b) diejenigen, worin L eine Gruppe $L^1$, in der A eine Gruppe $A^3$ ist und der Formel

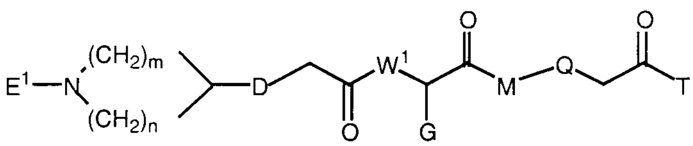

I-B

worin $E^1$, m, n, D, $W^1$, G, Q und T obige Bedeutung haben und M 1,4-Phenylen ist, mit der Massgabe, dass G nicht Wasserstoff ist,

c) diejenigen, worin L eine Gruppe $L^1$, in der A eine Gruppe $A^2$ ist und der Formel

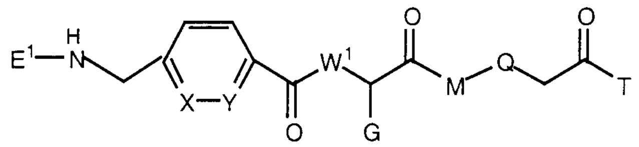 I-C

worin $E^1$, X, Y, $W^1$, G, M, Q und T obige Bedeutung haben, insbesondere worin Q O und T OH oder nieder-Alkoxy ist,

d) diejenigen, worin L eine Gruppe $L^1$, in der A eine Gruppe $A^4$ ist und der Formel

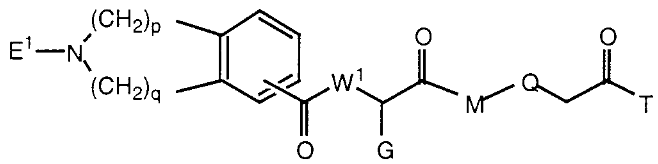 I-D

worin $E^1$, p, q, $W^1$, G, M, Q und T obige Bedeutung haben, insbesondere worin M 1,4-Phenylen, Q O und T nieder-Alkoxy ist, mit der Massgabe, dass G nicht Wasserstoff ist,

e) diejenigen, worin L eine Gruppe $L^2$, in der A eine Gruppe $A^1$ ist und der Formel

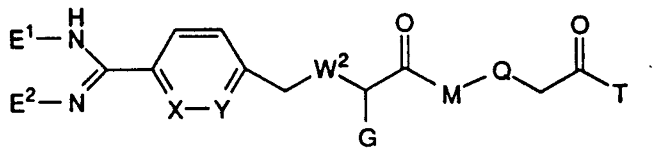 **I-E**

worin $E^1$, $E^2$, X, Y, $W^2$, G, M, Q und T obige Bedeutung haben, insbesondere worin M über das N-Atom an die Ketogruppe gebundenes 1,4-Piperidinylen, Q O und T nieder-Alkoxy ist,

f) diejenigen, worin L eine Gruppe $L^3$, in der A eine Gruppe $A^1$ ist und der Formel

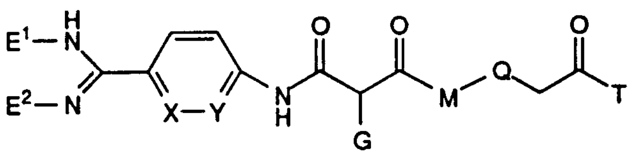 **I-F**

worin $E^1$, $E^2$, X, Y, G, M, Q und T obige Bedeutung haben, insbesondere worin M über das N-Atom an die Ketogruppe gebundenes 1,4-Piperidinylen, Q O und T nieder-Alkoxy ist,

g) diejenigen, worin L eine Gruppe $L^4$, in der A eine Gruppe $A^1$ ist und der Formel

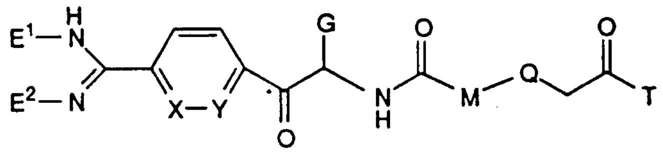

I-G

worin $E^1$, $E^2$, X, Y, G, M, Q und T obige Bedeutung haben, insbesondere worin M 1,4-Phenylen, Q O und T nieder-Alkoxy ist,

h) diejenigen, worin L eine Gruppe $L^5$ ist und der Formel

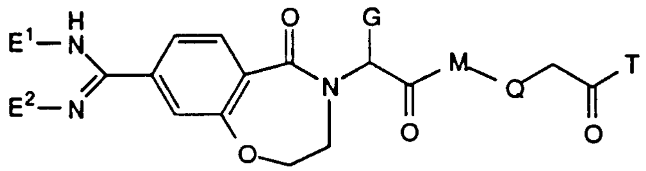

I-H

worin $E^1$, $E^2$, G, M, Q und T obige Bedeutung haben, insbesondere worin M über das N-Atom an die Ketogruppe gebundenes 1,4-Piperidinylen, Q O und T nieder-Alkoxy ist.

Beispiele von Essigsäurederivaten der vorliegenden Erfindung sind diejenigen, worin L eine Gruppe der Formel

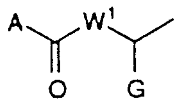

$L^1$

A eine Gruppe $A^1$, $A^2$ oder $A^{30}$:

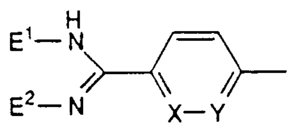

$A^1$

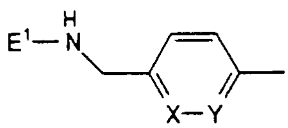

$A^2$

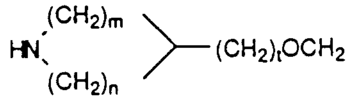

$A^{30}$

eins von $E^1$ und $E^2$ H und das andere H, nieder-Alkyl, OH, nieder-Alkoxy, nieder-Alkoxy-niederalkyl, Carboxy-niederalkyl, $PO(O\text{-nieder-Alkyl})_2$, $C(O)OR^1$ oder $OC(O)OR^1$,

$R^1$ nieder-Alkoxy-niederalkyl, nieder-Alkyl, durch OH, COOH oder nieder-Alkanoyloxy substituiertes nieder-Alkyl, oder gegebenenfalls substituiertes und gegebenenfalls über nieder-Alkylen gebundenes Phenyl, oder gegebenenfalls durch O unterbrochenes Cycloalkyl,

eins von X und Y CH und das andere CH oder N,

m und n 0 bis 5 und t0 bis 3, jedoch m + n 1 bis 5 und jedes von m + t und n + t mindestens 1,

$W^1$ $CH_2$, nieder-Alkyl-OCOCH, NH, nieder-Alkyl-N oder nieder-Alkoxy-niederalkyl-N,

G und M die gleiche Bedeutung wie im Anspruch 1 haben,

Q Sauerstoff,

T eine Gruppe OT'' und

T'' H, nieder-Alkyl, nieder-Alkoxy-niederalkyl oder gegebenenfalls über nieder-Alkylen gebundenes und gegebenenfalls durch O unterbrochenes Cycloalkyl ist, mit den Massgaben, dass

a)

T'' von H, nieder-Alkyl und Phenyl-niederalkyl verschieden ist, falls

A eine Gruppe der Formel

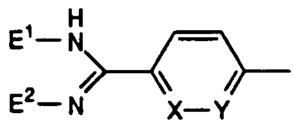

$A^1$

$E^1$ und $E^2$ Wasserstoff, tert-Butoxycarbonyl oder Benzyloxycarbonyl sind,

X, Y, G und Q die obige Bedeutung haben,

$W^1$ NH, nieder-Alkyl-N oder nieder-Alkoxy-niederalkyl-N und

M über das N-Atom an die Ketogruppe gebundenes 1,4-Piperidinylen ist und dass

b)

T'' von H, nieder-Alkyl, Phenyl und Phenyl-niederalkyl, verschieden ist, falls

L eine Gruppe der Formel $L^{11}$:

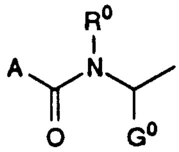

$L^{11}$

A eine Gruppe der Formel

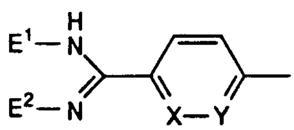

$A^1$

$E^1$ und $E^2$ Wasserstoff, tert-Butoxycarbonyl oder Benzyloxycarbonyl sind,

X, Y und Q die obige Bedeutung haben,

$R^o$ und $G^o$ H oder nieder-Alkyl und

M gegebenenfalls durch nieder-Alkyl, nieder-Alkoxy, $OCH_2COOH$ oder $OCH_2COO$-nieder-Alkyl substituiertes 1,4-Phenylen ist,

c) A nur dann eine Gruppe als Formel $A^{30}$ sein kann, wenn M 1,4-Phenylen ist,

und physiologisch verwendbare Salze davon.

Bevorzugte Verbindungen der Formel I sind diejenigen,

worin eins von $E^1$ und $E^2$ H und das andere H, OH, $C(O)OR^1$ oder $OC(O)OR^1$ ist und/oder

worin $R^1$ nieder-Alkyl, wie Aethyl, Butyl oder Isobutyl, nieder-Alkoxyniederalkyl, wie Methoxyäthyl, durch Benzoyloxy oder nieder-Alkanoyloxy substituiertes nieder-Alkyl, wie Benzoyloxymethyl, Acetoxymethyl, Acetoxyäthyl oder Pivaloyloxymethyl, oder Phenyl ist und/oder

worin eins von X und Y CH und das andere CH oder N und/oder worin $W^1$ NH oder $CH_2$ und/oder worin Q O oder $CH_2$ ist und/oder

worin G H, nieder-Alkyl, wie Methyl oder Aethyl, oder nieder-Alkoxycarbonylamino-niederalkyl, wie Aethoxycarbonylaminopropyl ist und/oder

worin M über das N-Atom an die Ketogruppe gebundenes 1,4-Piperidinylen, 1,4-Phenylen oder durch $OCH_2COO$-nieder-Alkyl, wie Methoxycarbonylmethoxy, substituiertes 1,4-Phenylen ist und/oder

worin T nieder-Alkoxy, wie Methoxy, Aethoxy, Isopropoxy, Isobutoxy, tert-Butoxy oder Hexyloxy, nieder-Alkoxy-niederalkoxy, wie Methoxyäthoxy, durch COO-nieder-Alkyl substituiertes nieder-Alkenyloxy, wie 2-Isobutoxycarbonyl-2-pentenyloxy, durch nieder-Alkyl-COO substituiertes nieder-Alkoxy, wie Pivaloyloxymethoxy, durch nieder-Alkyl-OCOO substituiertes nieder-Alkoxy, wie 1-Isopropoxycarbonyloxy-äthoxy, gegebenenfalls durch O unterbrochenes Cycloalkyloxy, wie Tetrahydropyranyloxy, über nieder-Alkylenoxy gebundenes Pyridyl, wie 3- oder 4-Pyridylmethoxy, oder über nieder-Alkylenoxy gebundenes und gegebenenfalls durch NCOO-nieder-Alkyl unterbrochenes Cycloalkyl, wie 1-tert-Butoxycarbonyl-3 oder 4-piperidylmethoxy, ist.

Beispiele solcher Verbindungen sind die aus der Gruppe der folgenden:

(S)-4-[2-[4-[Imino-2-(methoxy-ethoxycarbonylamino)-methyl]-benzoylamino]-propionyl]-phenoxyessigsäureethylester,
(Z)-(R,S)-4-[2-[4-[Amino-hydroxyimino-methyl]-benzoylamino]-propionyl]-phenoxyessigsäureethylester,
(S)-4-[2-[4-(Ethoxycarbonylamino-imino-methyl)-benzoylamino]-propionyl]-phenoxyessigsäure-tetrahydropyran-4-yl-ester,
(Z)-(R,S)-4-[2-[4-[Amino-ethoxycarbonyloximino-methyl]-benzoylamino]-propionyl]-phenoxyessigsäureethylester,
(S)-4-[2-[4-(Imino-phenoxycarbonylamino-methyl)-benzoylamino]-propionyl]-phenoxyessigsäureethylester,
(S)-4-[2-[4-[Imino-(2-methoxy-ethoxy-carbonylamino)-methyl]-benzoylamino]-propionyl]-phenoxyessigsäure-2-methoxy-ethylester,
(Z)-(S)-4-[2-[4-(Amino-hydroxyimino-methyl)-benzoylamino]-propionyl]-phenoxyessigsäureethylester,
(E/Z)-(S)-1-[2-[4-(Amino-ethoxycarbonylimino-methyl)-benzoylamino]-propionyl]-piperidin-4-yloxyessigsäure-isopropylester,
(E/Z)-(S)-1-[2-[4-(Amino-hydroxyimino-methyl)-benzoylamino]-propionyl]-piperidin-4-yloxyessigsäure-isopropylester,
[1-[4-[4-(Ethoxycarbonylamino-imino-methyl)-phenyl]-4-oxo-butyryl]-piperidin-4-yloxy]-essigsäureisopropylester,
(RS)-[1-[4-[4-(Isobutoxycarbonylamino-imino-methyl)-phenyl]-2-methyl-4-oxo-butyryl]-piperidin-4-yloxy]-essigsäure-isopropylester und insbesondere
(E/Z)-(S)-1-[2-[4-(Amino-hydroxyimino-methyl)-benzoylamino]-propionyl]-piperidin-4-yloxyessigsäureethylester

sowie die aus der Gruppe der folgenden:

(Z)-(S)-[1-[2-[4-(Amino-hydroxyimino-methyl)-benzoylamino]-propionyl]-piperidin-4-yloxy]essigsäure-(R/S)-1-isopropoxycarbonyloxy-ethylester,
(R)-(E)/(Z)-[1-[4-[4-(Amino-hydroxyimino-methyl)-phenyl]-2-methyl-4-oxo-butyryl]-piperidin-4-yloxy]-essigsäure-pyridin-3-ylmethyl-ester,
(R)-(E)/(Z)-[1-[4-[4-(Amino-hydroxyimino-methyl)-phenyl]-2-methyl-4-oxo-butyryl]-piperidin-4-yloxy]-essigsäure-pyridin-4-ylmethyl-ester,
(E)- oder (Z)-(RS)-3-[1-[(R)-4-[4-(amino-hydroxyimino-methyl)-phenyl]-2-methyl-4-oxo-butyryl]-piperidin-4-yloxyacetoxymethyl]-piperidin-1-carbonsäure-tert-butylester,
(R)-[1-[4-[4-(Benzoyloxymethoxycarbonylamino-imino-methyl)-phenyl]-2-methyl-4-oxo-butyryl]-piperidin-4-yloxy]-essigsäure-ethylester,
(R)-[1-[4-[4-(Imino-pivaloyloxymethoxycarbonylamino-methyl)-phenyl]-2-methyl-4-oxo-butyryl]-piperidin-4-yloxy]-essigsäure-ethylester,
(E)- oder (Z)-(R)-4-[1-[4-[4-(Amino-hydroxyimino-methyl)-phenyl]-2-methyl-4-oxo-butyryl]-piperidin-4-yloxyacetoxymethyl]-piperidin-1- carbonsäure-tert-butylester,
(S)-[4-[2-[4-[(2-Acetoxy-ethoxy-carbonylimino)-amino-methyl]-benzoylamino]-propionyl]-phenoxy]-essigsäure-ethylester und
(S)-4-[2-(4-Ethoxycarbonylmethoxy-phenyl)-1-methyl-2-oxo-ethylcarbamoylmethoxy]-piperidin-1-carbonsäure-acetoxymethylester.

Weitere Beispiele von Verbindungen der Formel I sind folgende:

(S)-4-[2-[4-(Amino-imino-methyl)-benzoylamino]-propionyl]-phenoxyessigsäurecyclopropylmethylester,
(S)-4-[2-[4-(Amino-imino-methyl)-benzoylamino]-propionyl]-phenoxyessigsäurecyclohexylester,
(S)-4-[2-[4-(Amino-imino-methyl)-benzoylamino]-3-hydroxy-propionyl]-phenoxyessigsäureethylester,
(S)-4-[2-[4-(Amino-imino-methyl)-benzoylamino]-3-(4-methoxy-phenyl)-propionyl]-phenoxyessigsäure,
(2S,3R)-4-[2-[4-(Amino-imino-methyl)-benzoylamino]-3-hydroxy-butyryl]-phenoxyessigsäureethylester,
(S)-4-[2-[4-(Amino-imino-methyl)-benzoylamino]-propionyl]-phenoxyessigsäure-2-methoxy-ethylester,
(S)-4-[2-[4-(Amino-imino-methyl)-benzoylamino]-3-acetoxy-propionyl]-phenoxyessigsäureethylester,
(R,S)-4-[2-[4-(Ethoxycarbonylamino-imino-methyl)-benzoylamino]-3-methylsulfanyl-propionyl]-phenoxyessigsäureethylester,
(S)-4-[2-[4-(Diethoxyphosphorylamino-imino-methyl)-benzoylamino]-propionyl]-phenoxyessigsäureethylester,
(E/Z)-(S)-1-[2-[4-(Amino-ethoxycarbonylimino-methyl)-benzoylamino]-3-(4-ethoxycarbonyloxy-phenyl)-propionyl]-piperidin-4-yloxyessigsäure-isopropylester,
2-Amino-benzoesäure-(E/Z)-(S)-4-[2-[4-(amino-ethoxycarbonyliminomethyl)-benzoylamino]-3-(4-isopropoxycarbonylmethoxy-piperidin-1-yl)-3-oxo-propyl]-phenylester,
Furan-2-carbonsäure-(E/Z)-(S)-4-[2-[4-(amino-ethoxycarbonyliminomethyl)-benzoylamino]-3-(4-isopropoxycarbonylmethoxy-piperidin-1-yl)-3-oxo-propyl]-phenylester,
2-Acetoxy-benzoesäure-(E/Z)-(S)-4-[2-[4-(amino-ethoxycarbonyliminomethyl)-benzoylamino]-3-(4-isopropoxycarbonylmethoxy-piperidin-1-yl)-3-oxo-propyl]-phenylester,
(S)-1-[2-(5-Amino-imino-methyl-pyridin-2-ylcarbonylamino)-propionyl]-piperidin-4-yloxy-essigsäure-tetrahydropyran-4-ylester,
(S)-1-[2-(5-Aminomethyl-pyridin-2-ylcarbonylamino)-3-(4-methoxyphenyl)-propionyl]-piperidin-4-yloxyessigsäure-isopropylester.

Die erfindungsgemässen Essigsäurederivate lassen sich dadurch herstellen, dass man

a) in einer Verbindung der Formel

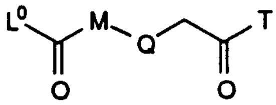

II

worin $L^o$ eine Gruppe einer der Formeln $L^{10}$ bis $L^{50}$

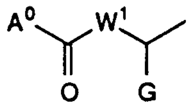

$L^{10}$

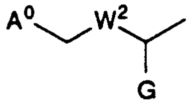

$L^{20}$

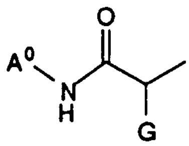

$L^{30}$

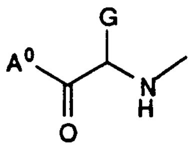

$L^{40}$

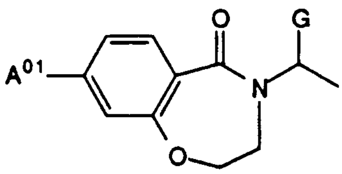

$L^{50}$

in der $A^o$ eine eine geschützte Amino- oder Amidinogruppe enthaltende Gruppe A ist und A, $W^1$, $W^2$, G, M, Q und T obige Bedeutung haben und $A^{01}$ eine geschützte Amidinogruppe ist,
die geschützte Amino- oder Amidinogruppe spaltet, oder

b) in einer Verbindung der Formel III

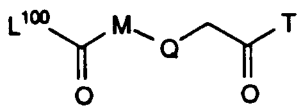

III

worin $L^{100}$ eine Gruppe einer der Formeln $L^{101}$ bis $L^{501}$

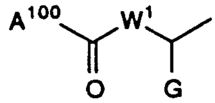

L101

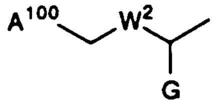

L201

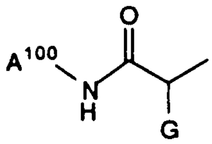

L301

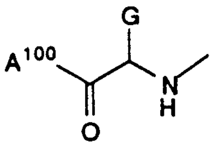

L401

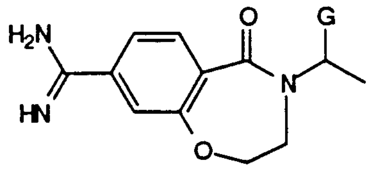

L501

in der $A^{100}$ eine eine freie Amidinogruppe enthaltende Gruppe A ist und A, $W^1$, $W^2$, G, M, Q und T obige Bedeutung haben,
oder in einem Salz davon die freie Amidinogruppe in eine durch eine Gruppe $E^1$ oder $E^2$ substituierte Amidinogruppe umwandelt oder

c) in einer Verbindung der Formel IV

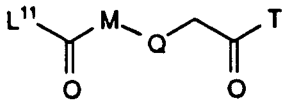

IV

worin $L^{11}$ eine Gruppe einer der Formeln $L^{111}$ bis $L^{115}$

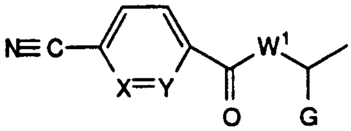

L111

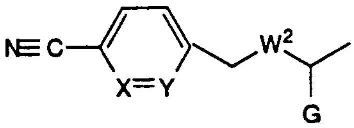

L112

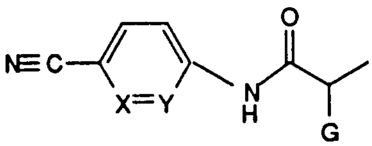

L113

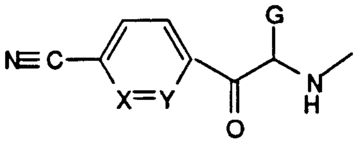

L114

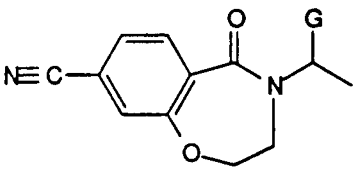

L115

in der X, Y, $W^1$, $W^2$, G, M, Q und T obige Bedeutung haben,
die in $L^{11}$ enthaltene Cyangruppe in eine gegebenenfalls durch $E^1$ oder $E^2$ substituierte Amidinogruppe umwandelt oder

d) ein Amin der Formel

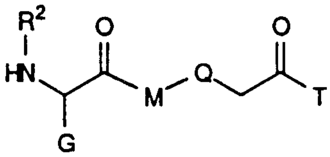

V

worin $R^2$ H, nieder-Alkyl oder nieder-Alkoxy-niederalkyl ist und G, M, Q und T obige Bedeutung haben,

mit einer Säure A-COOH oder einem funktionellen Derivat davon umsetzt, und
e) gewünschtenfalls eine in einer Verbindung der Formel I enthaltene reaktionsfähige Gruppe funktionell abwandelt und
f) gewünschtenfalls eine Verbindung der Formel I in ein physiologisch verträgliches Salz oder ein Salz einer Verbindung der Formel I in die freie Säure oder Base überführt.

Beispiele von in den Verbindungen II enthaltenen spaltbaren geschützten Amino- bzw. Amidinogruppen $A^o$

sind NH-Boc und NH-Z bzw. C(NH)NH-Boc, C(N-Boc)N$(Boc)_2$, C(N-Boc)NH-Boc und C(NH)NH-Z. Durch Boc geschützte Amino- und Amidinogruppen lassen sich z.B. mit einer Säure, wie Ameisensäure oder Trifluoressigsäure, gewünschtenfalls in einem Lösungsmittel, wie Dichlormethan, bei einer Temperatur bis zu 40°C, vorzugsweise bei Raumtemperatur spalten. Eine in einer Gruppe $A^o$ enthaltene Hydroxygruppe kann auch durch eine Tri-niederalkyl-silanylgruppe, wie tert-Butyl-dimethyl-silanyl, geschützt sein. Solche Gruppen kann man mittels Tetrabutylammoniumfluorid in einem Aether, wie Diäthyläther und/oder THF, bei einer Temperatur bis zu 40°C, vorzugsweise bei Raumtemperatur spalten.

Die Umwandlung b) einer in der Verbindung III oder einem Salz davon, z.B. dem Trifluoroacetat, enthaltenen freien Amidinogruppe in eine durch eine Gruppe $E^1$ oder $E^2$ substituierte Amidinogruppe kann man in einem Lösungsmittel, wie Dichlormethan, gewünschtenfalls in Gegenwart einer Base, wie $NaHCO_3$ oder $Na_2CO_3$, mit einer Verbindung der Formel $R^1$OC(O)Cl oder ClP(O)(O-nieder-Alkyl$)_2$ durchführen.

Zur Umwandlung der Cyangruppe in eine gegebenenfalls durch $E^1$ oder $E^2$ substituierte Amidinogruppe nach der Verfahrensvariante c) kann man ein Nitril der Formel IV in Pyridin und Triäthylamin mit $H_2S$ umsetzen und eine erhaltene durch Thiocarbamoyl $H_2$NC(S)- substituierte Verbindung z.B. in Aceton mit Methyljodid bei Siedetemperatur in die entsprechende durch Methylthioformimidoyl HN=C($SCH_3$)- substituierte Verbindung methylieren. Durch Umsetzung letzterer Verbindung mit einer Verbindung E-$NH_2$, worin E H, nieder-Alkyl oder nieder-Alkoxy-niederalkyl ist, oder mit einem Säureadditionssalz davon, wie das Hydrochlorid oder das Acetat, in einem Lösungsmittel, wie THF oder Methanol, unter Erhitzen, zweckmassigerweise bis auf den Siedepunkt des Reaktionsgemisches, erhält man das entsprechende Amidin der Formel I. Bei der Umsetzung eines Nitrils IV mit Hydroxylaminhydrochlorid in einem Lösungsmittel, wie Methanol oder DMSO, in Gegenwart einer Base, wie Natriummethanolat oder Triethylamin, entsteht die entsprechende Verbindung I, worin die Gruppe A (in L) eine hydroxylierte Amidinogruppe enthält.

Die Umsetzung d) lässt sich zweckmässigerweise mit einem Salz, z.B. dem Hydrochlorid, des Amins V in Gegenwart einer Base, wie Pyridin, in einem Lösungsmittel, wie einem Aether, bei einer Temperatur bis zu 40°C, vorzugsweise bei Raumtemperatur, bewerkstelligen.

Eine in einem nach Variante a) bis d) erhaltenen Ester der Formel I enthaltene tert-Butoxycarbonylgruppe $COOT^1$ kann man mittels einer Säure, wie Ameisensäure, zu einer Carboxygruppe spalten.

Als funktionelle Abwandlungen von reaktionsfähigen Gruppen nach der Verfahrensvariante e) sind (1) die Spaltung von nieder-Alkoxycarbonylgruppen, wie $COOT^1$; (2) die Spaltung von z.B. in der Gruppe G (in L) enthaltenen Aethergruppen; (3) die Umwandlung einer in einer Gruppe $A^1$ (in L) oder $L^5$ enthaltenen unsubstituierten Amidinogruppe ($E^1$=$E^2$=H) in eine substituierte Amidinogruppe; (4) die Umwandlung einer in einer Gruppe $A^1$ (in L) oder $L^5$ enthaltenen Hydroxygruppe (eins von $E^1$ und $E^2$ ist OH und das andere H) in eine $R^1$OC(O)O-gruppe.

Diese Umwandlungen lassen sich in an sich bekannter Weise durchführen, beispielsweise die Spaltung (1) in einem Lösungsmittel, wie einem wässrigen nieder-Alkanol, z.B. wässrigem Methanol oder Aethanol, mit einer Base, wie Natriumhydroxyd; die Spaltung (2) von Aethergruppen, wie die tert-Butoxygruppe, in Dichlormethan mittels Trifluoressigsäure; die Umwandlungen (3) und (4) in einem Lösungsmittel, wie Dichlormethan, in Gegenwart von $NaHCO_3$ oder $Na_2CO_3$, mittels einer Verbindung der Formel $R^1$OC(O)Cl oder in DMF in Gegenwart von Triethylamin mittels $R^1$OC(O)O-p-$NO_2C_6H_5$.

Weitere Umwandlungen, wie die Veresterung einer Carboxygruppe in einer Säure der Formel I (T = OH), die Veresterung einer in der Gruppe G (in L) enthaltenen Hydroxygruppe zur Aminobenzoyloxy-, Furoyloxy-, Acetoxy- oder Acetoxybenzoyloxygruppe, die Abspaltung der Hydroxygruppe aus einer in der Gruppe A (in L) enthaltenen hydroxylierten Amidinogruppe und die Umwandlung einer in der Gruppe L und/oder zwischen L und M enthaltenen nicht amidartig gebundenen Gruppe O=C= in die Gruppe HON=C= können in an sich bekannter Weise wie in den Beispielen im Detail beschrieben bewerkstelligt werden.

Eine in der Gruppe A (in L) enthaltene hydroxylierte Amidinogruppe kann man in Aceton mittels Ameisensäure unter Erwärmen bzw. in Gegenwart von Methylmorpholin in Dichlormethan mittels Triphosgen unter Abkühlung in die (5,5-Dimethyl- bzw. 5-Oxo)-4,5-dihydro-1,2,4-oxadiazol-3-ylgruppe umwandeln. Die Umwandlung einer in der Gruppe A (in L) enthaltenen Amidinogruppe in eine durch -C(O)$SR^1$ substituierte Amidinogruppe lässt sich in Dichlormethan in Gegenwart von $NaHCO_3$ mittels entsprechendem Chlorothioformat ClC(O)$SR^1$ bewerkstelligen.

Eine in einer Gruppe der Formel L enthaltene N-unsubstituierte Gruppe $A^3$ oder $A^4$, worin $E^1$ H ist, kann man zunächst mittels Acrylnitril in Aethanol in die entsprechende durch 2-Cyanoäthyl N-substituierte Gruppe $A^3$ oder $A^4$, worin $E^1$ für 2-Cyanoäthyl steht, und letztere Gruppe $A^3$ oder $A^4$ in Dichlormethan mittels m-Chlorbenzoesäure in die entsprechende N-Hydroxy substituierte Gruppe $A^3$ oder $A^4$, worin $E^1$ OH ist, umwandeln.

Die Verbindungen der Formel II lassen sich in an sich bekannter Weise herstellen. So kann man diejenigen, worin L eine N-haltige Gruppe $L^{10}$ ist und Q für O steht und der Formel

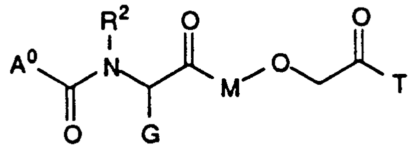

II-A

worin $A^o$, G, M und T obige Bedeutung haben und $R^2$ H, nieder-Alkyl, nieder-Alkoxy-niederalkyl ist,

ausgehend von den Verbindungen der Formel VI

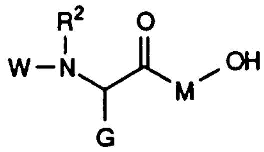

VI

worin W eine Schutzgruppe wie Boc, ist und $R^2$, G und M obige Bedeutungen haben,

über die Verbindungen der Formel

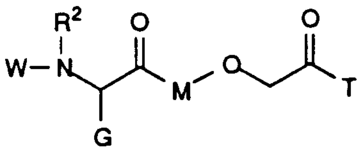

V-A

herstellen. Die Reaktion VI→V-A kann man mittels eines Bromids $BrCH_2C(O)T$ in Gegenwart einer Base, wie $K_2CO_3$, in einem Lösungsmittel, wie DMF, durchführen. Durch Abspaltung der Schutzgruppe W in der Verbindung der Formel V-A erhält man eine Verbindung der Formel V. Diese oder ein Säureadditionssalz davon, z.B. das Hydrochlorid, wird dann mit einem funktionellen Derivat einer Säure $A^oCOOH$, z.B. dem Säurechlorid, in Gegenwart einer Base, wie N-Methylmorpholin, in einem Lösungsmittel, wie THF, in die Verbindung II-A umgesetzt.

Die Verbindungen VI, worin M gegebenenfalls substituiertes 1,4-Phenylen ist, sind bekannt oder können in an sich bekannter Weise hergestellt werden, z.B. ausgehend von den Verbindungen VII

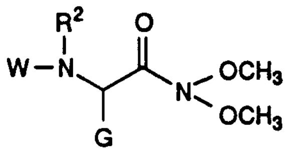

VII

über diejenigen der Formel VI-A

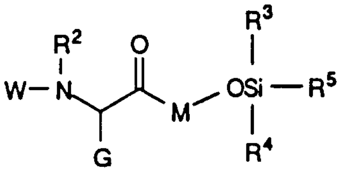

VI-A

worin $R^3$, $R^4$ und $R^5$ nieder-Alkyl sind. Die Reaktion VII→VI-A kann man mittels eines Bromids Br-M-OSi($R^3$,$R^4$,$R^5$) in einem Lösungsmittel, wie THF, bei niedriger Temperatur, z.B. -78°C in Gegenwart von n-Butyllithium in Hexan durchführen. Nach Abspaltung der Silanylgruppe, z.B. in einem Lösungsmittel, wie Diäthyläther, mittels Tetrabutylammoniumfluorid in THF erhält man die Verbindung VI.

Die Verbindungen der Formel V-A, worin M 1,4-Piperidinylen ist, kann man durch Umsetzung einer Säure der Formel VIII mit einem Amin der Formel IX herstellen:

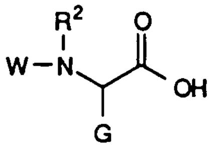

VIII

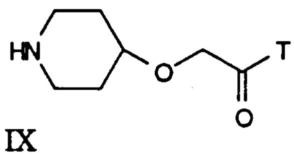

IX

Solche Verbindungen V-A lassen sich über die Verbindungen V in die entsprechenden Verbindungen II, worin M 1,4-Piperidinylen ist, überführen.

Nitrile der Formel IV sind z.B. durch Umsetzung von den Verbindungen der Formel V-A (nach Abspaltung der Schutzgruppe W) mit einem Säurechlorid

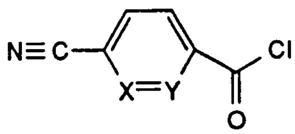

X

erhältlich.

Nitrile der Formel IV, worin M 1,4-Piperidinylen ist, kann man auch durch Umsetzung einer z.B. mit 2-Chlor-4,6-dimethoxy-1,3,5-triazin und N-Methylmorpholin aktivierten Säure der Formel $L^{11}$-COOH mit einem Amin der Formel IX-A herstellen:

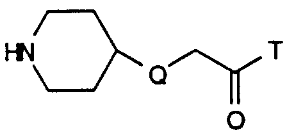

IX-A

Eine weitere Herstellungsmethode der Nitrile IV besteht darin, dass man ein Keton der Formel XI mit einem Bromid der Formel XII

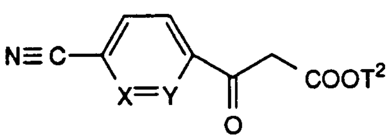

XI

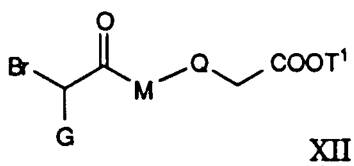

XII

worin $T^2$ nieder-Alkyl ist und X, Y, G, M und $T^1$ obige Bedeutung haben,

umsetzt. In einer Variante wird ein Säurechlorid der Formel X über einen Ester der Formel XI, worin $T^2$ tert-Butyl ist, mit dem Bromid XII in eine Verbindung der Formel XIII:

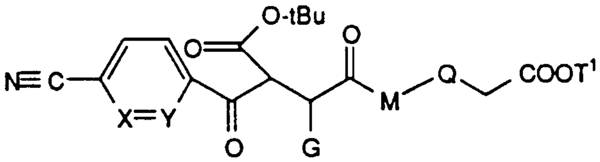

XIII

überführt und die t-Butoxycarbonylgruppe aus der Verbindung XIII abgespalten.

Durch Hydrierung eines Nitrils IV, worin $W^1$ (in $L^{11}$) NH, N-nieder-Alkyl oder nieder-Alkoxy-niederalkyl-N ist, z.B. über einem Pd/C-Katalysator in Methanol/Wasser/Essigsäure erhält man eine Verbindung der Formel I in der die Gruppe A (in L) die Formel $A^{01}$

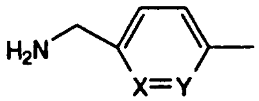

$A^{01}$

hat.

Die in $L^{11}$ durch Cyan substituierten Ausgangssäuren $L^{11}$-COOH sind durch Sandmeyer-Reaktion mit der entsprechenden durch Amino substituierten Säure erhältlich. Ausgangssäuren der Formel $L^{115}$-COOH lassen sich ausgehend von 4-Cyanosalicylsäure via Verbindungen der Formel

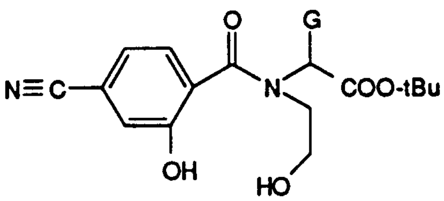

XIV

herstellen.

Zudem enthalten manche der nachfolgenden Beispiele detaillierte Angaben betreffend die Herstellung bestimmter Ausgangsmaterialien und Zwischenprodukten.

Die Verbindungen der Formel I, ihre Solvate und ihre Salze hemmen sowohl die Bindung von Fibrinogen, Fibronectin und des Willebrand-Faktors an den Fibrinogenrezeptor der Blutplättchen (Glykoprotein IIb/IIIa) als auch die Bindung derselben und weiterer adhäsiver Proteine, wie Vitronectin, Kollagen und Laminin, an die entsprechenden Rezeptoren auf der Oberfläche verschiedener Zelltypen. Die besagten Verbindungen beeinflussen somit Zell-Zell- und Zell-Matrix-Wechselwirkungen. Sie verhindern insbesondere die Entstehung von Blutplättchenthromben und können bei der Bekämpfung bzw. Verhütung von Krankheiten, wie Thrombose, Hirnschlag, Herzinfarkt, Entzündung und Arteriosklerose verwendet werden. Ferner haben diese Verbindungen einen Effekt auf Tumorzellen, indem sie deren Metastasierung hemmen. Somit können sie auch als Antitumor-Mittel eingesetzt werden. Weiter können sie die Wundheilung beschleunigen. Da sie auch den Knochenabbau verhindern, kann man sie bei der Behandlung der Osteoporose verwenden.

Die Aktivität der Verbindungen kann wie folgt nachgewiesen werden:

Nach oraler Applikation einer erfindungsgemässen Verbindung an der Maus wird das Plasma oder eine Verdünnung davon (1 Teil) mit Plättchen-reichem human Plasma (human PRP, 3 Teile) gemischt. Das Volumen an Mäuseplasma, das benötigt wird, um die ADP-induzierte Plättchenaggregation in dieser Mischung zu 50% zu hemmen, wird im Aggregometer bestimmt. Dieses Volumen ($IC_{50}$) wird durch das Total-volumen der Mischung dividiert und mit der applizierten Dosis multipliziert. Die in der nachstehenden Tabelle solchermassen extrapolierten $ID_{50}$-Werte entsprechen derjenigen Dosis der Testsubstanz, welche oral appliziert werden muss, um die ADP-induzierte ex vivo Aggregation der Plättchen in human PRP zu 50% zu hemmen.

| Produkt von Beispiel | 3 | 4 | 9 | 11 | 20 | 22 | 24 | 27 | 28 | 29 |
|---|---|---|---|---|---|---|---|---|---|---|
| $ID_{50}$ (mg/kg) | 2,7 | 2,1 | 1,4 | 3,1 | 2,4 | 1,8 | 2,3 | 4,3 | 0,3 | 1,0 |

| Produkt von Beispiel | 30 | 32 | 33 | 35 | 36 | 38 | 40 | 41 | 49 | 50 |
|---|---|---|---|---|---|---|---|---|---|---|
| $ID_{50}$ (mg/kg) | 3,9 | 0,2 | 0,6 | 0,5 | 0,5 | 2,9 | 0,6 | 0,2 | 1,2 | 1,3 |

| Produkt von Beispiel | 51 | 53 | 60 | 63 | 72 | 76 | 80 | 85 |
|---|---|---|---|---|---|---|---|---|
| $ID_{50}$ (mg/kg) | 3,9 | 1,5 | 0,3 | 1,1 | 2,7 | 0,2 | 0,7 | 0,4 |

Wie eingangs erwähnt, sind Arzneimittel, enthaltend eine Verbindung der Formel I, ein Solvat davon oder ein Salz davon, ebenfalls Gegenstand der vorliegenden Erfindung, weiterhin auch ein Verfahren zur Herstellung derartiger Arzneimittel, welches dadurch gekennzeichnet ist, dass man eine oder mehrere der besagten Verbindungen und erwünschtenfalls einen oder mehrere andere therapeutisch wertvolle Stoffe in eine galenische Darreichungsform bringt. Die Arzneimittel können enteral, z.B. oral in Form von Tabletten, Lacktabletten, Dragées, Hart- und Weichgelatinekapseln, Lösungen, Emulsionen oder Suspensionen, oder rektal, z.B. in Form von Suppositorien; oder als Spray verabreicht werden. Die Verabreichung kann aber auch parenteral, z.B. in Form von Injektionslösungen oder als Infusion, erfolgen.

Zur Herstellung von Tabletten, Lacktabletten, Dragées und Hartgelatinekapseln kann der Wirkstoff mit pharmazeutisch inerten, anorganischen oder organischen Excipientien vermischt werden. Als solche Excipientien kann man für Tabletten, Dragées und Hartgelatinekapseln z.B. Lactose, Maisstärke oder Derivate davon, Talk, Stearinsäure oder deren Salze, verwenden. Für Weichgelatinekapseln eignen sich als Excipientien z.B. vegetabile Oele, Wachse, Fette, halbfeste und flüssige Polyole; je nach Beschaffenheit des Wirkstoffes sind jedoch bei Weichgelatinekapseln überhaupt keine Excipientien erforderlich. Zur Herstellung von Lösungen und Sirupen eignen sich als Excipientien z.B. Wasser, Polyole, Saccharose, Invertzucker und Glukose; für Injektionslösungen eignen sich z.B. Wasser, Alkohole, Polyole, Glycerin und vegetabile Oele, und für Suppositorien eignen sich z.B. natürliche oder gehärtete Oele, Wachse, Fette und halbflüssige oder flüssige Polyole. Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten.

Für die Bekämpfung bzw. Verhütung der weiter oben genannten Krankheiten kann die Dosierung des Wirkstoffes innerhalb weiter Grenzen variieren und ist natürlich in jedem einzelnen Fall den individuellen Gegebenheiten anzupassen. Im allgemeinen dürfte bei oraler Verabreichung eine Dosis von etwa 0,1 bis 20 mg/kg, vorzugsweise von etwa 0,5 bis 4 mg/kg, pro Tag für den Erwachsenen angemessen sein, wobei aber die soeben angegebene obere Grenze auch überschritten werden kann, wenn sich dies als angezeigt erweisen sollte.

Beispiel 1

Zu einer Lösung von 0,5 g (S)-4-(2-tert-Butoxycarbonylamino-3-phenylpropionyl)-phenoxyessigsäureethylester in 2,5 ml Dichlormethan werden 0,2 ml Jodtrimethylsilan in 2 ml Dichlormethan zugegeben und 15 min. bei Raumtemperatur gerührt. Die Reaktionslösung wird mit 30 ml HCl in Methanol (4N) versetzt und eingeengt. Der Rückstand wird in 5 ml Pyridin gelöst und mit 275 mg p-Amidinobenzoylchlorid hydrochlorid während 24 h bei Raumtemperatur gerührt. Die Suspension wird abgenutscht, die Mutterlauge eingeengt und der Eindampfrückstand an silyliertem Kieselgel RP18 chromatographiert (THF/Wasser-Gradient). Man erhält 222 mg (S)-4-[2-[4-(Amino-imino-methyl)-benzoylamino]-3-phenyl-propionyl]-phenoxyessigsäuremethylester-hydrochlorid, $[\alpha]_D^{20}$ = + 31.1° (c = 1, Methanol).

Das Ausgangsmaterial kann wie folgt hergestellt werden:

a) Zu einer Lösung von 11,5 g p-Brom-tert-butyldimethylsilylphenol in 160 ml THF werden bei -78 °C unter Wasserausschluss 29 ml t-Butyllithium (1,4 M in Pentan) zugegeben und 15 min. bei -78 °C gerührt. Anschliessend wird die Lösung bei -78 °C mit 4 g (S)-2-tert Butoxycarbonylamino-N-methoxy-N-methyl-3-phenylpropionamid in 40 ml THF versetzt, 1 h bei -78 C gerührt und zu 300 ml 1 M Phosphorsäure gegossen. Die wässerige Phase wird mit Ether extrahiert, die Etherphasen mit ges. NaCl-Lösung gewaschen, getrocknet und eingeengt. Nach Chromatographie des Eindampfrückstands an Kieselgel (Hexan Essigester 9:1) erhält man 2,95 g (S)-[1-Benzyl-2-(4-tert-butyl-dimethyl-silanyloxy-phenyl)-2-oxo-ethyl]-carbaminsäure-tert-butylester als farbloses Öl, $[\alpha]_D^{20}$ = + 40,3° (c = 0,8, Chloroform).

b) Eine Lösung von 2,24 g des Produkts von a) in 30 ml THF wird mit 0,75 g Cäsiumfluorid in 2,5 ml Wasser 16 h bei Raumtemperatur gerührt und eingeengt. Chromatographie des Rückstands an Kieselgel (Hexan/Essigester 2:1) ergibt 2,47 g (S)-[1-Benzyl-2-(4-hydroxy-phenyl)-2-oxo-ethyl]-carbaminsäure-tert butylester, $[\alpha]_D^{20}$ = + 67,0° (c = 0,7, Chloroform).

c) Eine Suspension von 2,44 g des Produkts von b), 2,39 g Ethylbromoacetat und 2,97 g Kaliumcarbonat in 20 ml DMF wird 4 h bei Raumtemperatur gerührt, der Niederschlag abgenutscht und die Mutterlauge eingeengt. Chromatographie des Rückstands an Kieselgel (Hexan/Essigester 5:1) ergibt 7,44 g (S)-4-(2-tert-Butoxycarbonylamino-3-phenyl-propionyl)-phenoxyessigsäureethylester, $[\alpha]^{20}_D$ = + 40,6° (c = 1, Chloroform).

<u>Beispiel 2</u>

Eine Lösung von 263 mg 4-tert-Butoxycarbonylaminoacetyl-phenoxyessigsäure-2-methoxy-ethylester in 10 ml Dichlormethan und 5 ml Trifluoressigsäure wird 2 h bei Raumtemperatur gerührt und eingeengt. Der Rückstand wird in Ether suspendiert, die Kristalle abgenutscht, in 5 ml Pyridin gelöst und mit 219 mg Amidinobenzoylchlorid hydrochlorid 18 h bei Raumtemperatur gerührt. Einengen der Reaktionslösung und Chromatographie des Rückstands an silyliertem Kieselgel RP18 (THF/Wasser-Gradient) ergibt 35 mg 4-[4-(Amino-imino-methyl)-benzoylaminoacetyl]-phenoxyessigsäure-2-methoxy-ethylester, MS (EI): 414 $(M+H)^+$.

Das Ausgangsmaterial kann wie folgt hergestellt werden:

a) Eine Suspension von 476 mg (R,S)-2-Hydroxy-2-(4-hydroxyphenyl)-ethylcarbaminsäure-tert-butylester, 287 mg (2-Methoxy-ethyl)-chloroacetat und 260 mg Kaliumcarbonat in 13 ml DMF wird 2 h bei 50 °C gerührt, auf Raumtemperatur abgekühlt und mit 100 ml Wasser verdünnt. Die wässerige Phase wird mit Ether extrahiert, die Etherphasen mit ges. NaCl-Lösung gewaschen, getrocknet und eingeengt. Chromatographie des Rückstands an Kieselgel (Hexan/Essigester 1:2) ergibt 349 mg (R,S)-4-(2-tert-Butoxycarbonylamino-1-hydroxy-ethyl)-phenoxyessigsäure-2-methoxy-ethylester, MS (EI): 312 (M-57).

b) Die Oxidation von 316 mg des Produkts von a) in 10 ml Dichlormethan mit 224 mg Pyridinchlorochromat während 3 h bei Raumtemperatur ergibt nach Einengen der Reaktionslösung und Chromatographie des Rückstands an Kieselgel (Hexan/Essigester 1:1, 1:2) 284 mg 4-tert-Butoxycarbonylaminoacetyl-phenoxyessigsäure-2-methoxy-ethylester, MS (EI): 294 (M-73).

<u>Beispiel 3</u>

Eine Lösung von 550 mg (S)-4-(2-tert-Butoxycarbonylamino-propionyl)-phenoxyessigsäurecyclopropylmethylester in 10 ml Dichlormethan wird mit 0,2 ml Trimethylsilyljodid 15 min. bei 0 °C gerührt, mit 0,5 ml HCl in Dioxan (4 M) versetzt und eigeengt. Der Rückstand wird analog Beispiel 1 mit 330 mg p-Amidinobenzoylchlorid hydrochlorid umgesetzt. Man erhält nach Chromatographie des Rohprodukts an silyliertem Kieselgel RP18 (THF/Wasser-Gradient) 313 mg (S)-4-[2-[4-(Amino-imino-methyl)-benzoylamino]-propionyl]-phenoxyessigsäurecyclopropylmethylester hydrochlorid (1:0,4) hydrojodid (1:0,6), Smp. 125 °C, $[\alpha]_D^{20}$ = + 56,6° (c = 0,5, DMSO).

Das Ausgangsmaterial kann wie folgt hergestellt werden:

a) Zu einer Lösung von 43,05 g p-Brom-tert-butyldimethylsilylphenol in 150 ml THF werden bei -78 °C unter Wasserausschluss 94 ml n-Butyllithium (1,6 M in Hexan) zugetropft und 15 min. bei -78 °C gerührt. Anschliessend werden in 30 min. bei -78 °C 11,61 g (S)-2-tert-Butoxycarbonylamino-N-methoxy-N-methyl-propionamid in 150 ml THF zugegeben, 0,5 h bei -78 °C gerührt und die Reaktionslösung zu 400 ml 1 M Phosphorsäure gegossen. Die wässerige Phase wird mit Ether extrahiert, die Etherphasen mit ges. NaCl-Lösung gewaschen, getrocknet und eingeengt. Nach Chromatographie des Eindampfrückstands an Kieselgel (Hexan/Essigester 9:1) erhält man 17,85 g (S)-1-(4-tert-Butyl-dimethyl-silanyloxy-benzoyl)-ethylcarbaminsäure-tert-butylester als farbloses Öl, Rf = 0,43 (Hexan/Essigester 5:1).

b) Zu einer Lösung von 17,85 g des Produkts von a) in 180 ml Ether werden 47 ml Tetrabutylammoniumfluorid (1 M in THF) zugegeben und 1 h bei Raumtemperatur gerührt. Die Reaktionslösung wird mit 1 M Phosphorsäure

extrahiert, mit ges. NaCl-Lösung gewaschen, getrocknet und eingeengt. Man erhält 12,03 g (S)-1-(4-Hydroxy-benzoyl)-ethylcarbaminsäure-tert-butylester, Smp. 166-168 °C, $[\alpha]_D^{20}$ = + 24,9° (c = 1, Chloroform).

c) Die Umsetzung von 400 mg des Produkts von b) mit Cyclopropylmethylbromoacetat analog Beispiel 1c ergibt nach Chromatographie an Kieselgel (Hexan Essigester 3:1) 580 mg (S)-4-(2-tert-Butoxycarbonylamino-propionyl)-phenoxyessigsäurecyclopropylmethylester, $[\alpha]_D^{20}$ = + 12,4° (c = 0,9, Chloroform).

Beispiel 4

Analog Beispiel 3 werden 500 mg (S)-4-(2-tert-Butoxycarbonylamino-propionyl)-phenoxyessigsäurecyclohexylester mit 0,17 ml Trimethylsilyljodid und anschliessend mit 320 mg p-Amidinobenzoylchorid hydrochlorid umgesetzt und ergeben nach Chromatographie an silyliertem Kieselgel RP18 (THF/Wasser-Gradient) 330 mg (S)-4-[2-[4-(Amino-imino-methyl)-benzoylamino]-propionyl]-phenoxyessigsäurecyclohexylester hydrochlorid (1:0,6) hydrojodid (1:0,4), Smp. 110 °C, $[\alpha]_D^{20}$ = + 56,7° (c = 0,55, DMSO).

Das Ausgangsmaterial kann wie folgt hergestellt werden:

Die Umsetzung von 530 mg (S)-1-(4-Hydroxy-benzoyl)-ethylcarbaminsäure-tert-butylester mit 660 mg Cyclohexylbromoacetat analog Beispiel 1c ergibt nach Chromatographie an Kieselgel (Hexan Essigester 5:1) 735 mg (S)-4-(2-tert-Butoxycarbonylamino-propionyl)-phenoxyessigsäurecyclohexylester, $[\alpha]_D^{20}$ = + 12,0° (c = 0,5, Chloroform).

Beispiel 5

Wie in Beispiel 3 werden 680 mg (S)-4-(2-tert-Butoxycarbonylamino-propionyl)-phenoxyessigsäure-tetrahydropyran-4-yl-ester mit 1,7 ml Trimethylsilyljodid (1 M in Dichlormethan) und anschliessend mit 438 mg p-Amidinobenzoylchlorid hydrochlorid umgesetzt und ergeben nach Chromatographie an silyliertem Kieselgel RP18 (THF/Wasser-Gradient) 430 mg (S)-4-[2-[4-(Amino-imino-methyl)-benzoylamino]-propionyl]-phenoxyessigsäure-tetrahydropyran-4-yl-ester-hydrochlorid (1:0,8) hydrojodid (1:0,4), $[\alpha]_D^{20}$ = + 50,3° (c = 1, Dimethylsulfoxid).

Das Ausgangsmaterial kann wie folgt hergestellt werden:

a) Die Reaktion von 530 mg (S)-1-(4-Hydroxy-benzoyl)-ethylcarbaminsäure-tert-butylester mit 670 mg 4-Tetrahydropyranyl-bromoacetat analog Beispiel 1c ergibt nach Chromatographie an Kieselgel (Hexan/Essigester 2:1) 736 mg (S)-4-(2-tert-Butoxycarbonylamino-propionyl)-phenoxyessigsäure-tetra-hydropyran-4-yl-ester, $[\alpha]_D^{20}$ = + 11,1° (c = 0,7, Chloroform).

Beispiel 6

Eine Lösung von 120 mg (S)-4-[2-[4-(Amino-imino-methyl)-benzoylamino]-3-phenyl-propionyl]-phenoxyessigsäuremethylester hydrochlorid und 80 mg Natriumhydroxid in 20 ml Methanol und 5 ml Wasser wird 1 h 15 min. bei Raumtemperatur gerührt, mit 1 N HCl angesäuert und eingeengt. Chromatographie des Rückstands an silyliertem Kieselgel RP18 (THF/Wasser-Gradient) ergibt 60 mg (S)-4-[2-[4-(Amino-imino-methyl)-benzoylamino]-3-phenyl-propionyl]-phenoxyessigsäure, Smp. > 200 °C, MS (ISP): 446 $(M+H)^+$.

Beispiel 7

Die Umsetzung von 800 mg 4-[(S)-3-tert-Butoxy-2-tert-butoxycarbonylamino-propionyl]-phenoxyessigsäureethylester mit 450 mg Trimethylsilyljodid und 497 mg p-Amidinobenzoylchlorid hydrochlorid analog Beispiel 3 ergibt nach Chromatographie an silyliertem Kieselgel RP18 (THF/Wasser-Gradient) 540 mg 4-(S)-2-[4-(Amino-imino-methyl)-benzoylamino]-3-tert-butoxy-propionyl]-phenoxyessigsäureethylester-hydrochlorid (1:0,5)-hydrojodid (1:0,4), Smp. 144 °C, $[\alpha]_D^{20}$ = + 34,2° (c = 0,5, DMSO).

Das Ausgangsmaterial kann wie folgt hergestellt werden:

a) Eine Suspension von 10 g N-tert-Butoxycarbonyl-O-tert-butyl-L-serindicyclohexylammoniumsalz in 100 ml THF wird mit 5,5 ml N-Methylmorpholin, 10,3 g HBTU und 2,2 g N,O-Dimethylhydroxylamin hydrochlorid 21 h bei Raumtemperatur gerührt und eingeengt. Chromatographie des Rückstands an Kieselgel (Hexan/Essigester 1:1) ergibt 6,02 g (S)-2-tert-Butoxy-1-(N-methoxy-N-methyl-carbamoyl)-ethylcarbaminsäure-tert-butylester, $[\alpha]_D^{20}$ = + 18,6° (c = 0,65, Chloroform).

b) Eine Lösung von 3,7 g des Produkts von a) wird analog Beispiel 3a mit 7,82 g p-Brom-tert-butyldimethylsilylphenol und 26 ml tert-Butyllithium (1,4 M in Pentan) umgesetzt. Chromatographie des Rückstands an Kieselgel

(Hexan/Essigester 5:1) ergibt 3,58 g (S)-2-tert-Butoxy-1-(4-tert-butyl-dimethylsilanyloxy-benzoyl)-ethylcarbaminsäure-tert-butylester, $[\alpha]_D^{20}$ = + 28,3° (c = 0,6, Chloroform).

c) Die Abspaltung der Silylschutzgruppe von 2,5 g des Produkts von b) mit 840 mg Cäsiumfluorid erfolgt nach Beispiel 1b. Nach Chromatographie an Kieselgel (Hexan/Essigester 3:1) erhält man 1,08 g (S)-2-tert-Butoxy-1-(4-hydroxy-benzoyl)-ethylcarbaminsäure-tert-butylester, $[\alpha]_D^{20}$ = + 41,5 °(c = 1, Chloroform).

d) Die Reaktion von 1 g des Produkts von c) mit 0,5 ml Ethylbromoacetat und 1,23 g Kaliumcarbonat analog 1c ergibt nach Chromatographie an Kieselgel (Hexan/Essigester 3:1) 1,19 g 4-[(S)-3-tert-Butoxy-2-tert-butoxycarbonylamino-propionyl]-phenoxyessigsäureethylester, $[\alpha]_D^{20}$ = + 27,3° (c = 0,8, Chloroform).

Beispiel 8

Analog zu Beispiel 3 werden 778 mg (S)-4-[2-tert-Butoxycarbonylamino-3-(4-methoxy-phenyl)-propionyl]-phenoxyessigsäureethylester mit 0,28 ml Trimethylsilyljodid und 450 mg p-Amidinobenzoylchlorid umgesetzt und das Rohprodukt an silyliertem Kieselgel RP18 (THF/Wasser-Gradient) chromatographiert. Man erhält 272 mg (S)-4-[2-[4-(Amino-imino-methyl)-benzoylamino]-3-(4-methoxy-phenyl)-propionyl]-phenoxyessigsäureethylester-hydrochlorid (1:0,3) hydrojodid (1:0,3), Smp. 129 °C, $[\alpha]_D^{20}$ = + 20,0° (c = 0,6, DMSO).

Das Ausgangsmaterial kann wie folgt hergestellt werden:

a) Die Umsetzung von 3,5 g (S)-1-(N-Methoxy-N-methyl-carbamoyl)-2-(4-methoxy-phenyl)-ethylcarbaminsäure-tert-butylester mit 8,9 g p-Brom-tert-butyldimethylsilylphenol und 22,2 ml tert-Butyllithium (1,4 M in Pentan) nach Beispiel 3a und Chromatographie des Eindampfrückstands an Kieselgel (Hexan/Essigester 5:1) ergibt 3,66 g (S)-1-[4-(tert-Butyl-dimethylsilanyloxy)-benzoyl]-2-(4-methoxy-phenyl)-ethylcarbaminsäure-tert-butylester, $[\alpha]_D^{20}$ = + 23,8° (c = 0,5, Chloroform).

b) Eine Lösung von 2,5 g des Produkts von a) wird wie in Beispiel 3b) beschrieben entschützt. Nach Chromatographie an Kieselgel (Hexan/Essigester 3:1, 2:1) erhält man 1,42 g (S)-1-(4-Hydroxy-benzoyl)-2-(4-methoxy-phenyl)-ethylcarbaminsäure-tert-butylester, Smp. 133-135 °C, $[\alpha]_D^{20}$ = + 47,3° (c = 0,9, Chloroform).

c) Die Umsetzung von 1,0 g der Vorstufe mit Ethylbromoacetat nach Beispiel 1c) und Chromatographie des Rückstands an Kieselgel (Hexan/Essigester 2:1) ergibt 1,21 g (S)-4-[2-tert-Butoxycarbonylamino-3-(4-methoxy-phenyl)-propionyl]-phenoxyessigsäureethylester, Smp. 95-97 °C, $[\alpha]_D^{20}$ = + 22,6° (c = 0,5, Chloroform).

Beispiel 9

Eine Lösung von 300 mg (S)-4-[2-[4-(Amino-imino-methyl)-benzoylamino]-3-tert-butoxy-propionyl]-phenoxyessigsäureethylester-hydrochlorid (1:0,5) hydrojodid (1:0,4) in 3 ml Dichlormethan wird mit 1 ml Trifluoressigsäure versetzt, 4 h bei Raumtemperatur gerührt und eingeengt. Chromatographie des Rückstands an Kieselgel (Chloroform/Ethanol/Wasser 60:30:5) ergibt 100 mg (S)-4-[2-[4-(Amino-imino-methyl)-benzoylamino]-3-hydroxy-propionyl]-phenoxyessigsäureethylester trifluoracetat, $[\alpha]_D^{20}$ = + 52,0° (c = 0,3, Dimethylsulfoxid).

Beispiel 10

Eine Lösung von 260 mg (S)-4-[2-(4-tert-Butoxycarbonylaminomethyl-benzoylamino)-propionyl]-phenoxyessigsäureethylester in 3 ml Dichlormethan und 1,5 ml Trifluoressigsäure wird 1 h bei Raumtemperatur gerührt und eingengt. Der Rückstand entspricht 260 mg (S)-4-[2-(4-Aminomethyl-benzoylamino)-propionyl]-phenoxyessigsäureethylester, $[\alpha]_D^{20}$ = + 43,0° (c = 0,3, DMSO).

Das Ausgangsmaterial kann wie folgt hergestellt werden:

a) Die Umsetzung von 530 mg (S)-1-(4-Hydroxy-benzoyl)-ethylcarbaminsäure-tert-butylester mit Ethylbromoacetat analog Beispiel 1c ergibt nach Chromatographie an Kieselgel (Hexan Essigester 5:1) 582 mg (S)-4-(2-tert-Butoxy-carbonylamino-propionyl)-phenoxyessigsäureethylester, $[\alpha]_D^{20}$ = - 1,8° (c = 0,5, Ethanol).

b) Entschützen von 620 mg des Produkts von a) analog Beispiel 3 ergibt (S)-4-(2-Amino-propionyl)-phenoxyessigsäureethylester hydrochlorid, das als Rohprodukt weiter umgesetzt wird. Eine Lösung von 443 mg 4-tert-Butoxycarbonylaminomethyl-benzoesäure und 0,43 ml N-Methylmorpholin in 5 ml THF wird bei 0°C mit 800 mg HBTU versetzt, 1 h bei 0°C gerührt und anschliessend 0,3 ml N-Methylmorpholin und der oben erwähnten (S)-4-(2-Amino-propionyl)-phenoxyessigsäureethylester, in 6 ml THF gelöst, zugegeben. Nach 3,5 h Rühren bei Raumtemperatur wird die Lösung eingeengt und der Rückstand an Kieselgel chromatographiert (Hexan/Essigester 1:1). Man erhält 463 mg (S)-4-[2-(4-tert-Butoxycarbonylaminomethyl-benzoylamino)-propionyl]-phenoxyessigsäure-

ethylester, $[\alpha]_D^{20}$ = + 50,0° (c = 0,5, Chloroform).

Beispiel 11

Eine Lösung von 630 mg (S)-4-[2-[4-(Amino-imino-methyl)-benzoylamino]-3-(4-methoxy-phenyl)-propionyl]-phenoxyessigsäureethylester-hydrochlorid (1:0,3)-hydrojodid (1:0,3) (Beispiel 8) und 330 mg Natriumhydroxyd in 15 ml Wasser und 30 ml Ethanol wird 3 h bei Raumtemperatur gerührt und anschliesend eingeengt. Nach Chromatographie an silyliertem Kieselgel RP18 (THF/Wasser-Gradient) erhält man 32 mg (S)-4-[2-[4-(Amino-imino-methyl)-benzoylamino]-3-(4-methoxy-phenyl)-propionyl]-phenoxyessigsäure, Mikroanalyse: ber. C 65,68, H 5,30, N 8,84; gef. C 65,77, H 5,12, N 8,73.

Referenzbeispiel 12

Eine Lösung von 1 g (S)-4[2-[4-(tert-Butoxycarbonylamino-imino-methyl)-benzoylamino]-3-methyl-butyryl]-phenoxyessigsäureethylester in 10 ml Dichlormethan und 5 ml Trifluoressigsäure wird 3 h bei Raumtemperatur gerührt und eingeengt. Chromatographie an silyliertem Kieselgel RP18 (THF/Wasser-Gradient) ergibt 789 mg (S)-4-[2-[4-(Amino-imino-methyl)-benzoylamino]-3-methyl-butyryl]-phenoxyessigsäureethylester-trifluoracetat, Smp. 202 °C, $[\alpha]_D^{20}$ = + 95,5° (c = 1, DMSO).

Das Ausgangsmaterial kann wie folgt hergestellt werden:

a) Analog Beispiel 3a) werden 2,6 g (S)-2-tert-Butoxycarbonylamino-N-methoxy-N,3-dimethyl-butyramid mit 8,6 g p-Brom-tert-butyldimethylsilylphenol umgesetzt. Nach Chromatographie an 200 g Kieselgel (Hexan/Essigester 95:5) erhält man 3,0 g (S)-1-(4-tert-Butyl-dimethyl-silanyloxybenzoyl)-2-methyl-propylcarbaminsäure-tert-butylester, $[\alpha]_D^{20}$ = + 83,0° (c = 0,5, Chloroform).
b) Die Abspaltung der Schutzgruppe von 3,0 g des Produkts von a) analog Beispiel 3b) ergibt 1,88 g (S)-1-(4-Hydroxy-benzoyl)-2-methyl-propylcarbaminsäure-tert-butylester, $[\alpha]_D^{20}$ = + 107,9° (c = 0,7, Chloroform).
c) Die Reaktion von 1,88 g des Produkts von b) mit Ethylbromoacetat wie in Beispiel 1c und anschliessende Chromatographie an Kieselgel (Hexan/Essigester 5:1) ergibt 2,02 g (S)-4-(2-tert-Butoxycarbonylamino-3-methyl-butyryl)-phenoxyessigsäureethylester, $[\alpha]_D^{20}$ = + 86,1° (c = 1, Chloroform).
d) Zu einer Suspension von 7 g 4-Amidinobenzoesäure in 120 ml Dioxan und 88 ml 1 N NaOH gibt man bei 0 °C 13,96 g Di-tert-butyldicarbonat in 70 ml Dioxan und rührt anschliessend 1,5 h bei Raumtemperatur. Der Niederschlag wird abgenutscht, die Mutterlauge zweimal mit 100 ml Ether extrahiert, mit 1 N HCl auf pH 6 gestellt und eingeengt. Chromatographie des Rückstands an silyliertem Kieselgel RP18 (THF/Wasser-Gradient) ergibt 4,2 g N-tert-Butoxycarbonyl-4-amidinobenzoesäure, Smp. > 200 °C.
e) Zu einer Lösung von 1,14 g (S)-4-(2-tert-Butoxycarbonylamino-3-methyl-butyryl)-phenoxyessigsäureethylester in 10 ml wasserfreiem Dichlormethan gibt man bei 0 °C 410 µl Trimethylsilyljodid, rührt 15 min bei 0 °C, versetzt die gelbe Lösung mit 1,5 ml HCl in Dioxan (4 N) und dampft anschliessend das Lösungsmittel ab. Der Rückstand wird in 10 ml THF gelöst, mit 793 mg N-tert-Butoxycarbonyl-4-amidinobenzoesäure, 1,0 ml Triethylamin, 1,33 g HBTU versetzt und 16 h bei Raumtemperatur gerührt. Die entstandene Suspension wird eingeengt und der Rückstand an Kieselgel chromatographiert. Man erhält 1,07 g (S)-4-[2-[4-(tert-Butoxycarbonylamino-imino-methyl)-benzoylamino]-3-methyl-butyryl]-phenoxyessigsäureethylester, MS (ISP): 526 $(M+H)^+$.

Beispiel 13

In Analogie zu Beispiel 12 erhält man aus 250 mg (S)-4-[2-[4-(tert-Butoxycarbonylamino-imino-methyl)-benzoylamino]-3-[4-(2-methoxy-ethoxy)-phenyl]-propionyl]-phenoxyessigsäureethylester und Chromatographie an silyliertem Kieselgel RP18 (THF/Wasser-Gradient) 66 mg (S)-4-[2-[4-(Amino-imino-methyl)-benzoylamino]-3-[4-(2-methoxy-ethoxy)-phenyl]-propionyl]-phenoxyessigsäureethylester trifluoracetat, MS (ISP): 548 $(M+H)^+$.

Das Ausgangsmaterial kann wie folgt hergestellt werden:

a) Die Phasentransferreaktion von 2,2 g (S)-2-(4-Hydroxy-phenyl)-1-(methoxy-methyl-carbamoyl)-ethylcarbaminsäure-tert-butylester mit 2,5 g 2-Chlorethylmethylether in 10 ml Toluol und 10 ml 50% NaOH in Gegenwart von 50 mg Tetrabutylammoniumhydrogensulfat ist nach 1 h beendet. Die wässerige Phase wird mit Ether extrahiert, die vereinigten organischen Phasen mit gesättigter NaCl-Lösung gewaschen, getrocknet und eingeengt. Chromatographie des Rückstands an Kieselgel ergibt 1,11 g (S)-2-[4-(2-Methoxy-ethoxy)-phenyl]-1-(methoxy-methyl-carbamoyl)-ethylcarbaminsäure-tert-butylester, $[\alpha]_D^{20}$ = + 17,6° (c = 1, Chloroform).
b) Durch Umsetzung von 1 g der Vorstufe gemäss 3a erhält man nach Chromatographie an Kieselgel (Hexan/Essigester 9:1) 326 mg (S)-1-[4-(tert-Butyl-dimethyl-silanyloxy)-benzoyl]-2-[4-(2-methoxy-ethoxy)-phenyl]-ethylcarbaminsäure-tert-butylester, MS (ISP): 530 $(M+H)^+$.

c) Durch Umsetzung von 310 mg des Produkts von b) wie in Beispiel 3b und Alkylierung des Produkts analog Beispiel 1c ergibt nach Chromatographie an Kieselgel (Hexan/Essigester 2:1) 273 mg (S)-4-[2-tert-Butoxycarbonylamino-3-[4-(2-methoxy-ethoxy)-phenyl]-propionyl]-phenoxyessigsäureethylester, MS (ISP): 502 $(M+H)^+$.

d) Die Reaktion von 250 mg der Vorstufe gemäss Beispiel 12e führt nach Chromatographie an Kieselgel (Hexan/Essigester 1:2) zu 267 mg (S)-4-[2-[4-(tert-Butoxycarbonylamino-imino-methyl)-benzoylamino]-3-[4-(2-methoxy-ethoxy)-phenyl]-propionyl]-phenoxyessigsäureethylester, MS (ISP): 648 $(M+H)^+$.

Referenzbeispiel 14

Eine Lösung von 400 mg (S)-4-[2-[4-(Amino-imino-methyl)-benzoylamino]-3-methyl-butyryl]-phenoxyessigsäureethylester trifluoracetat (Beispiel 12) in 65 ml Ethanol und 17 ml Wasser wird mit 200 mg NaOH versetzt, 1 h bei Raumtemperatur gerührt, mit 7 ml 1N HCl angesäuert und eingeengt. Der Rückstand ergibt nach Chromatographie an silyliertem Kieselgel RP18 (THF/Wasser-Gradient) 287 mg (S)-4-[2-[4-(Amino-imino-methyl)-benzoylamino]-3-methyl-butyryl]-phenoxyessigsäure hydrochlorid (1:0,4), MS (ISP): 398 $(M+H)^+$.

Beispiel 15

Zu einer Suspension von 250 mg (S)-4-[2-[4-(Amino-imino-methyl)-benzoylamino]-3-hydroxy-propionyl]-phenoxyessigsäureethylester-trifluoracetat in 4 ml Dichlormethan und 4 ml gesättigter $Na_2CO_3$-Lösung gibt man 66 mg 2-Methoxy-ethyl-chloroformiat und rührt anschliessend 5 min. bei Raumtemperatur. Die wässerige Phase wird mit Dichlormethan extrahiert, die Dichlormethanphasen mit Wasser gewaschen, getrocknet und eingeengt. Nach Chromatographie des Rückstands an Kieselgel (Essigester) erhält man 55 mg (S)-4-[3-Hydroxy-2-[4-[imino-(2-methoxy-ethoxy-carbonylamino)-methyl]-benzoylamino]-propionyl]-phenoxyessigsäureethylester, MS (ISP): 516 $(M+H)^+$.

Beispiel 16

Eine Lösung von 200 mg (S)-4-[3-tert-Butoxy-2-[4-tert-butoxycarbonylamino-imino-methyl)-benzoylamino]-propionyl]-phenoxyessigsäure-tert-butylester in 2 ml Dichlormethan und 1 ml Trifluoressigsäure wird 2 h bei Raumtemperatur gerührt und eingeengt. Der Rückstand wird in Methanol suspendiert, abzentrifugiert und mit Ether gewaschen. Man erhält 41 mg (S)-4-[2-[4-(Amino-imino-methyl)-benzoylamino]-3-hydroxy-propionyl]-phenoxyessigsäure-trifluoracetat, MS (ISN): 384 $(M-H)^-$.

Das Ausgangsmaterial kann wie folgt hergestellt werden:

a) Die Reaktion von 1,35 g (S)-2-tert-Butoxy-1-(4-hydroxy-benzoyl)-ethylcarbaminsäure-tert-butylester mit tert-Butylbromoacetat analog Beispiel 1c ergibt 417 mg 1,19 g (S)-4-(2-tert-Butoxycarbonylamino-3-tert-butoxypropionyl)-phenoxyessigsäure-tert-butylester, (ISN): 450 $(M-H)^-$.

b) Die Umsetzung von 400 mg der Vorstufe mit N-tert-Butoxycarbonyl-4-amidinobenzoesäure wie in Beispiel 12a ergibt nach Chromatographie an Kieselgel (Hexan/Essigester 1:1) 230 mg (S)-4-[3-tert-Butoxy-2-[4-(tert-butoxycarbonylamino-imino-methyl)-benzoylamino]-propionyl]-phenoxyessigsäure-tert-butylester, MS (ISN): 596 $(M-H)^-$.

Beispiel 17

Die Umsetzung von 100 mg (S)-4-[2-[4-(Amino-imino-methyl)-benzoylamino]-3-hydroxy-propionyl]-phenoxyessigsäureethylester-trifluoracetat (Beispiel 9) mit 54 µl Isobutylchloroformiat wie in Beispiel 15 ergibt nach Chromatographie (Essigester) 65 mg (S)-4-[3-Hydroxy-2-[4-(imino-isobutoxycarbonylamino-methyl)-benzoylamino]-propionyl]-phenoxyessigsäureethylester, $[\alpha]_D^{20}$ = + 47,0° (c = 0,7, DMSO).

Beispiel 18

Durch Umsetzung von 170 mg (S)-4-[2-[4-(tert-Butoxycarbonylamino-imino-methyl)-benzoylamino]-3-methoxy-propionyl]-phenoxyessigsäureethylester mit Trifluoressigsäure wie in Beispiel 12 und Kristallisation des Rückstands mit Ether erhält man 124 mg (S)-4-[2-[4-(Amino-imino-methyl)-benzoylamino]-3-methoxy-propionyl]-phenoxyessigsäureethylester-trifluoracetat, MS (ISP): 428 $(M+H)^+$.

Das Ausgangsmaterial kann wie folgt hergestellt werden:

a) Die Reaktion von 1,18 g (S)-2-tert-Butoxycarbonylamino-N,3-dimethoxy-N-methyl-propionamid mit p-Brom-tert-butyldimethylsilylphenol analog Beispiel 3a ergibt nach Chromatographie an Kieselgel (Hexan/Essigester 8:1) 872

mg (S)-1-[4-(tert-Butyl-dimethyl-silanyloxy)-benzoyl]-2-methoxy-ethylcarbaminsäure-tert-butylester, MS (ISP): 410 $(M+H)^+$.

b) Die Abspaltung der Silylschutzgruppe von 850 mg der Vorstufe nach Beispiel 3b und Alkylierung des Produkts nach Beispiel 1c führt zu 546 mg (S)-4-(2-tert-Butoxycarbonylamino-3-methoxy-propionyl)-phenoxyessigsäure-ethylester, MS (EI): 308 (M-73).

c) Die Reaktion von 530 mg der Vorstufe mit N-tert-Butoxycarbonyl-4-amidinobenzoesäure nach Vorschrift von Beispiel 12e und Chromatographie an Kieselgel (Hexan/Essigester 1:2) ergibt 170 mg (S)-4-[2-[4-(tert-Butoxycarbonylamino-imino-methyl)-benzoylamino]-3-methoxy-propionyl]- phenoxyessigsäure-ethylester, MS (ISP): 528 $(M+H)^+$.

Beispiel 19

Aus 510 mg (S)-4-[3-tert-Butoxy-2-[4-(tert-butoxycarbonylamino-imino-methyl)-benzoylamino]-propionyl]-phenoxyessigsäure-(S)-sec-butylester erhält man nach Beispiel 12 und Chromatographie des Rohprodukts an silyliertem Kieselgel RP18 (THF/Wasser-Gradient) 280 mg (S)-4-[2-[4-(Amino-imino-methyl)-benzoylamino]-3-hydroxy-propionyl]-phenoxyessigsäure-(S)-sec-butylester-trifluoracetat, Smp. 85-87 °C, $[\alpha]_D^{20}$ = + 55,7° (c = 0,3, Dimethylsulfoxid).

Das Ausgangsmaterial kann wie folgt hergestellt werden:

a) Aus 790 mg (S)-2-tert-Butoxy-1-(4-hydroxy-benzoyl)-ethylcarbaminsäure-tert-butylester erhält man mit Bromessigsäure-(S)-sec-butylester nach Beispiel 1c und Chromatographie an Kieselgel (Hexan/Essigester 9:1) 780 mg (S)-4-(3-tert-Butoxy-2-tert-butoxycarbonylamino-propionyl)-phenoxyessigsäure-(S)-sec-butylester, $[\alpha]_D^{20}$ = + 35,3° (c = 0,45, Chloroform).

b) Die Reaktion von 720 mg der Vorstufe mit 510 mg N-tert-Butoxycarbonyl-4-amidinobenzoesäure analog Beispiel 12e ergibt nach Chromatographie an Kieselgel (Hexan/Essigester (3:1, 2:1) 570 mg (S)-4-[3-tert-Butoxy-2-[4-(tert-butoxycarbonylamino-imino-methyl)-benzoylamino]-propionyl]-phenoxyessigsäure-(S)-sec-butylester, $[\alpha]_D^{20}$ = + 51,3° (c = 1, Chloroform).

Beispiel 20

Aus 650 mg (2S,3R)-4-[3-tert-Butoxy-2-[4-(tert-butoxycarbonylamino-imino-methyl)-benzoylamino]-butyryl]-phenoxyessigsäureethylester erhält man nach Beispiel 12 und Chromatographie an silyliertem Kieselgel RP18 (THF/Wasser-Gradient) 350 mg (2S,3R)-4-[2-[4-(Amino-imino-methyl)-benzoylamino]-3-hydroxy-butyryl]-phenoxyessigsäureethylester trifluoracetat, Smp. 95-97 °C,$[\alpha]_D^{20}$ = + 98,8° (c = 0,4, DMSO).

Das Ausgangsmaterial kann wie folgt hergestellt werden:

a) Aus 15 g (1S,2R)-2-tert-Butoxy-1-carboxy-propylcarbaminsäure-tert-butylester ehält man in Analogie zu 7a nach Chromatographie an Kieselgel (Hexan/Essigester 5:1, 3:1) 11,19 g (1S,2R)-2-tert-Butoxy-1-(methoxy-methyl-carbamoyl)-propylcarbaminsäure-tert-butylester, $[\alpha]_D^{20}$ = + 30,0° (c = 0,7, Chloroform).

b) Die Reaktion von 17 g der Vorstufe mit p-Brom-tert butyldimethylsilylphenol analog Beispiel 3a ergibt nach Chromatographie an Kieselgel (Hexan/Essigester 9:1) 4,76 g (1S,2R)-2-tert-Butoxy-1-[4-(tert-butyl-dimethyl-silanyloxy)-benzoyl]-propylcarbaminsäure-tert-butylester, $[\alpha]_D^{20}$ = + 61,0° (c = 0,3, Chloroform).

c) Die Abspaltung der Silylschutzgruppe von 4,74 g der Vorstufe nach Beispiel 3b und Alkylierung des Produkts nach Beispiel 1c führt nach Chromatographie an Kieselgel (Hexan/Essigester 5:1) zu 3,34 g (2S,3R)-4-(3-tert-Butoxy-2-tert-butoxycarbonylamino-butyryl)-phenoxyessigsäureethylester, $[\alpha]_D^{20}$ = + 71,0° (c = 0,3, Chloroform).

d) Die Reaktion von 1,09 g der Vorstufe mit N-tert-Butoxycarbonyl-4-amidinobenzoesäure nach Beispiel 12e und Chromatographie an Kieselgel (Hexan/Essigester 2:1, 1:1) ergibt 660 mg (2S,3R)-4-[3-tert-Butoxy-2-[4-(tert-butoxycarbonylamino-imino-methyl)-benzoylamino]-butyryl]-phenoxyessigsäureethylester, $[\alpha]_D^{20}$ = + 126,0° (c = 0,3, Chloroform).

Beispiel 21

Aus 2 g (S)-4-[2-[4-(tert-Butoxycarbonylamino-imino-methyl)-benzoylamino]-3-tert-butoxy-propionyl]-phenoxyessigsäure-2-methoxy-ethylester erhält man nach Entschützen analog Beispiel 12 und Chromatographie an silyliertem Kieselgel RP18 (THF/Wasser-Gradient) 600 mg (S)-4-[2-[4-(Amino-imino-methyl)-benzoylamino]-3-hydroxy-propionyl]-phenoxyessigsäure-2-methoxy-ethylester trifluoracetat, Smp. 174 °C, $[\alpha]_D^{20}$ = + 51,6° (c = 0,5, DMSO).

Das Ausgangsmaterial kann wie folgt hergestellt werden:

a) Die Alkylierung von 1,64 g (S)-2-tert-Butoxy-1-(4-hydroxy-benzoyl)-ethylcarbaminsäure-tert-butylester mit 2-Methoxy-ethylbromoacetat nach Beispiel 1c führt nach Chromatographie an Kieselgel zu 1,58 g (S)-4-[3-tert-Butoxy-2-tert-butoxycarbonylamino-propionyl]-phenoxyessigsäure-2-methoxy-ethylester, MS (ISP): 454 $(M+H)^+$.

b) Die Reaktion von 1,55 g der Vorstufe mit N-tert-Butoxycarbonyl-4-amidinobenzoesäure nach Beispiel 12e und Chromatographie an Kieselgel (Hexan/Essigester 2:1, 1:1) ergibt 2,0 g (S)-4-[2-[4-(tert-Butoxycarbonylamino-imino-methyl)-benzoylamino]-3-tert-butoxy-propionyl]-phenoxyessigsäure-2-methoxy-ethylester, MS (ISP): 600 $(M+H)^+$.

Beispiel 22

Aus 4,7 g (S)-4-[2-[4-(tert-Butoxycarbonylamino-imino-methyl)-benzoylamino]-propionyl]-phenoxyessigsäure-2-methoxy-ethylester erhält man nach Beispiel 12 und Chromatographie an silyliertem Kieselgel RP18 (THF/Wasser-Gradient) 1,67 g (S)-4-[2-[4-(Amino-imino-methyl)-benzoylamino]-propionyl]-phenoxyessigsäure-2-methoxy-ethylester, $[\alpha]_D^{20}$ = + 60,0° (c = 0,4, DMSO).

Das Ausgangsmaterial kann wie folgt hergestellt werden:

a) Die Umsetzung von 3,98 g (S)-1-(4-Hydroxy-benzoyl)-ethylcarbaminsäure-tert-butylester mit 3,25 g 2-Methoxy-ethylbromoacetat analog Beispiel 1c ergibt nach Chromatographie an Kieselgel (Hexan Essigester 2:1) 3,31 g (S)-4-(2-tert-Butoxycarbonylamino-propionyl)-phenoxyessigsäure-2-methoxy-ethylester, Rf = 0,29 (Hexan Essigester 1:1).

b) Die Reaktion von 3,3 g der Vorstufe gemäss Beispiel 12e führt nach Chromatographie an Kieselgel (Hexan/Essigester 1:2) zu 4,76 g (S)-4-[2-[4-(tert-Butoxycarbonylamino-imino-methyl)-benzoylamino]-propionyl]-phenoxyessigsäure-2-methoxy-ethylester, MS (ISP): 528 $(M+H)^+$.

Beispiel 23

Die Reaktion von 199 mg (S)-4-[2-[4-(Amino-imino-methyl)-benzoylamino]-propionyl]-phenoxyessigsäure-ethylester trifluoracetat mit Ethylchloroformiat gemäss Beispiel 15 führt nach Chromatographie an Kieselgel (Hexan/Essigester 1:2) zu 137 mg (S)-4-[2-[4-(Ethoxycarbonylamino-imino-methyl)-benzoylamino]-propionyl]-phenoxy-essigsäureethylester, MS (ISP): 470 $(M+H)^+$.

Das Ausgangsmaterial kann wie folgt hergestellt werden:

a) Die Umsetzung von 530 mg (S)-1-(4-Hydroxy-benzoyl)-ethylcarbaminsäure-tert-butylester mit Ethylbromoacetat analog Beispiel 1c ergibt nach Chromatographie an Kieselgel (Hexan Essigester 5:1) 582 mg (S)-4-(2-tert-Butoxy-carbonylamino-propionyl)-phenoxyessigsäureethylester, $[\alpha]^{20}_D$ = - 1,8° (c = 0,5, Ethanol).

b) Die Reaktion von 702 mg des Produkts von a) mit N-tert-Butoxycarbonyl-4-amidinobenzoesäure und die Abspaltung der Schutzgruppe gemäss Beispiel 12 ergibt nach Chromatographie an silyliertem Kieselgel RP18 (THF/Wasser-Gradient) 487 mg (S)-4-[2-[4-(Amino-imino-methyl)-benzoylamino]-propionyl]-phenoxyessigsäureethylester-trifluoracetat, Smp. 180 °C, $[\alpha]_D^{20}$ = + 54,2° (c = 0,6, DMSO).

Beispiel 24

Die Abspaltung der Schutzgruppen an 1 g (E,Z)-(S)-4-[3-Acetoxy-2-[4-[(di-tert-butoxycarbonylamino)-tert-butoxycarbonylimino-methyl]-benzoylamino]-propionyl]-phenoxyessigsäureethylester analog Beispiel 12 ergibt nach Kristallisation mit Ether 760 mg (S)-4-[2-[4-(Amino-imino-methyl)-benzoylamino]-3-acetoxy-propionyl]-phenoxyessigsäureethylester trifluoracetat, MS (ISP): 456 $(M+H)^+$.

Das Ausgangsmaterial kann wie folgt hergestellt werden:

a) Eine Lösung von 440 mg 4-[(S)-3-tert-Butoxy-2-tert-butoxycarbonyl-amino-propionyl]-phenoxyessigsäureethylester in 10 ml Dichlormethan wird mit 0,28 ml Trimethylsilyljodid 3 h bei Raumtemperatur gerührt, mit 1 ml HCl in Dioxan (ca. 4 M) versetzt und eingeengt. Der Rückstand wird in 10 ml THF gelöst, mit 464 mg (E,Z)-4-(tri-tert-Butoxycarbonyl-amidino)-benzoesäure, 253 mg N-Methylmorpholin, 379 mg HBTU versetzt, und 16 h bei Raumtemperatur gerührt. Die entstandene Suspension wird eingeengt und der Rückstand an Kieselgel (Hexan/Essigester 1:1) chromatographiert. Man erhält 320 mg (E,Z)-(S)-4-[2-[4-[(di-tert-Butoxycarbonylamino)-tert-butoxycarbonylimino-methyl]-benzoylamino]-3-hydroxy-propionyl]-phenoxyessigsäureethylester, MS (ISP): 714 $(M+H)^+$.

b) Die Acetylierung von 1,42 g der Vorstufe mit 0,156 ml Acetylchlorid in Gegenwart von 223 mg Triethylamin in 30 ml Ether während 1 h bei Raumtemperatur ergibt eine Suspension. Der Niederschlag wird abgenutscht, die Mutterlauge eingeengt und der Rückstand an Kieselgel chromatographiert. Man erhält 1,13 g (E,Z)-(S)-4-[3-Acetoxy-2-[4-[(di-tert-butoxycarbonylamino)-tert-butoxycarbonylimino-methyl]-benzoylamino]-propionyl]-phenoxyessigsäureethylester, MS (ISP): 756 $(M+H)^+$.

Beispiel 25

Die Umsetzung von 1 g 4-[4-(Amino-imino-methyl)-benzoylaminoacetyl]-phenoxyessigsäuremethylester (Beispiel 25 in EP 0 381 033) mit Ethylchloroformiat analog Beispiel 15 ergibt nach Kristallisation in Methanol/THF 810 mg 4-[4-(Ethoxycarbonylamino-imino-methyl)-benzoylaminoacetyl]-phenoxyessigsäuremethylester, MS (ISP): 442 $(M+H)^+$.

Beispiel 26

Aus 400 mg (E,Z)-(R,S)-4-[2-[4-[(di-tert-Butoxycarbonylamino)-tert-butoxycarbonylimino-methyl]-benzoylamino]-3-methylsulfanyl-propionyl]-phenoxyessigsäureethylester erhält man nach Abspalten der Schutzgruppen nach Beispiel 12 und Kristallisation mit Ether 190 mg (R,S)-4-[2-[4-(Amino-imino-methyl)-benzoylamino]-3-methylsulfanyl-propionyl]-phenoxyessigsäureethylester trifluoracetat, MS (ISP): 444 $(M+H)^+$.

Das Ausgangsmaterial kann wie folgt hergestellt werden:

Eine Suspension von 713 mg (E,Z)-(S)-4-[3-Acetoxy-2-[4-[(di-tert-butoxycarbonylamino)-tert-butoxycarbonylimino-methyl]-benzoylamino]-propionyl]-phenoxyessigsäureethylester (Beispiel 24b) und 140 mg Natriummethanthiolat in 10 ml Acetonitril wird 45 min. bei Raumtemperatur gerührt, Unlösliches abgenutscht, die Mutterlauge eingedampft und der Rückstand an Kieselgel (Hexan/Essigester 2:1) chromatographiert. Man erhält 407 mg (E,Z)-(R,S)-4-[2-[4-[(di-tert-Butoxycarbonylamino)-tert-butoxycarbonylimino-methyl]-benzoylamino]-3-methylsulfanyl-propionyl]-phenoxyessigsäureethylester, MS (EI): 744 $(M+H)^+$.

Beispiel 27

Aus 119 mg (R,S)-4-[2-[4-(Amino-imino-methyl)-benzoylamino]-3-methylsulfanyl-propionyl]-phenoxyessigsäureethylester trifluoracetat erhält man in Analogie zu Beispiel 15 mit 28 mg Ethylchloroformiat und Chromatographie an Kieselgel (Hexan/Essigester 2:3) 78 mg (R,S)-4-[2-[4-(Ethoxycarbonylamino-imino-methyl)-benzoylamino]-3-methylsulfanyl-propionyl]-phenoxyessigsäureethylester, MS (EI): 516 $(M+H)^+$.

Beispiel 28

Die Umsetzung von 511 mg (S)-4-[2-[4-(Amino-imino-methyl)-benzoylamino]-propionyl]-phenoxyessigsäureethylester trifluoracetat mit 2-Methoxy-ethylchloroformiat analog zu Beispiel 15 und Chromatographie des Rückstands an Kieselgel (Hexan/Essigester 1:2) ergibt 280 mg (S)-4-[2-[4-[Imino-2-(methoxy-ethoxycarbonylamino)-methyl]-benzoylamino]-propionyl]-phenoxyessigsäureethylester, MS (ISP): 500 $(M+H)^+$.

Beispiel 29

Die Abspaltung der Schutzgruppen an 850 mg (S)-4-[2-(4-Ethoxycarbonylmethoxy-phenyl)-1-methyl-2-oxo-ethylcarbamoylmethoxy]-piperidin-1-carbonsäure-tert-butylester analog Beispiel 12 ergibt nach Chromatographie an silyliertem Kieselgel RP18 (THF/Wasser-Gradient) 437 mg (S)-4-(2-Piperidin-4-yloxyacetylamino-propionyl)-phenoxyessigsäureethylester trifluoacetat, MS (ISP): 393 $(M+H)^+$.

Das Ausgangsmaterial kann wie folgt hergestellt werden:

Eine Lösung von 702 mg (S)-4-(2-tert-Butoxycarbonylamino-propionyl)-phenoxyessigsäureethylester in 10 ml Dichlormethan wird mit 0,27 ml Trimethylsilyljodid 15 min. bei 0 °C gerührt, mit 1 ml HCl in Dioxan (4 M) versetzt und eingeengt. Der Rückstand wird in 10 ml Dichlormethan gelöst. Eine Lösung von 518 mg 1-tert-Butoxycarbonyl-piperidin-4-yl-oxyessigsäure, 594 mg TPTU und 0,55 ml N-Methylmorpholin in 10 ml Dichlormethan wird 30 min. bei 0 °C gerührt und anschliessend mit dem oben beschriebenen Rückstand versetzt. Nach 2 Stunden Rühren bei Raumtemperatur wird die Reaktionslösung eingeengt und der Rückstand an Kieselgel chromatographiert (Hexan/Essigester 1:1, 1:2). Man erhält 873 mg (S)-4-[2-(4-Ethoxycarbonylmethoxy-phenyl)-1-methyl-2-oxo-ethylcarbamoylmethoxy]-piperidin-1-carbonsäure-tert-butylester, MS (ISP): 493 $(M+H)^+$.

Beispiel 30

Die Umsetzung von 511 mg (S)-4-[2-[4-(Amino-imino-methyl)-benzoylamino]-propionyl]-phenoxyessigsäureethylester-trifluoracetat mit Diethylchlorphosphat analog zu 15 und Chromatographie des Rückstands an Kieselgel

(Dichlormethan/Ethanol 9:1) ergibt 340 mg (S)-4-[2-[4-(Diethoxyphosphorylamino-imino-methyl)-benzoylamino]-propionyl]-phenoxyessigsäureethylester, MS (ISP): 534 $(M+H)^+$.

Beispiel 31

Eine Lösung von 1 g (S)-4-[2-(4-Cyano-benzoylamino)-propionyl]-phenoxyessigsäureethylester in 10 ml Pyridin und 1ml Triethylamin wird mit Schwefelwasserstoff gesättigt und 16 h bei Raumtemperatur gerührt. Die Reaktionslösung wird eingeengt, der Rückstand in Essigester gelöst, mit Natriumhydrogencarbonatlösung, Kaliumhydrogensulfatlösung und ges. Natriumchloridlösung gewaschen, getrocknet und eingeengt. Der Rückstand ergibt mit Ether 580 mg (S)-4-[2-[4-(Thiocarbamoyl)-benzoylamino]-propionyl]-phenoxyessigsäureethylester. Dieser wird abgenutscht, in 65 ml Aceton und 3,3 ml Methyljodid gelöst und 3 h bei Siedetemperatur gerührt. Einengen der Lösung und Kristallisation des Rückstands in Ether ergibt 609 mg (S)-4-[2-[4-(1-Methyl-thioformimidoyl)-benzoylamino]-propionyl]-phenoxyessigsäureethylester hydrojodid. 314 mg dieses Esters werden in 10 ml THF gelöst, mit 110 mg 2-Methoxy-ethylamin versetzt und 64 h bei Raumtemperatur gerührt. Einengen der Lösung und Chromatographie des Rückstands an silyliertem Kieselgel RP18 (THF/Wasser-Gradient) ergibt 80 mg (R,S)-4-[2-[4-[Imino-(2-methoxy-ethyl)-amino-methyl]-benzoylamino]-propionyl]-phenoxyessigsäureethylester hydrojodid. MS (ISP): 456 $(M+H)^+$.

Das Ausgangsmaterial kann wie folgt hergestellt werden:

Die Kupplung von 5,62 g (S)-4-(2-tert-Butoxycarbonylamino-propionyl)-phenoxyessigsäureethylester mit 2,82 g 4-Cyanobenzoesäure in Analogie zu Beispiel 12e ergibt nach Chromatographie an Kieselgel (Hexan/Essigester 3:2) 3,67 g (S)-4-[2-(4-Cyano-benzoylamino)-propionyl]-phenoxyessigsäureethylester, MS (EI): 381 $(M+H)^+$.

Beispiel 32

Die Abspaltung der Schutzgruppe an 160 mg (R,S)-4-[2-[4-[(tert-Butyl-dimethyl-silanyloxyamino)-imino-methyl]-benzoylamino]-propionyl]-phenoxyessigsäureethylester mit Tetrabutylammoniumfluorid analog Beispiel 3b ergibt nach Kristallisation des Rückstands in Essigester/Hexan 70 mg (Z)-(R,S)-4-[2-[4-[Amino-hydroxyimino-methyl]-benzoylamino]-propionyl]-phenoxyessigsäureethylester, MS (ISP): 414 $(M+H)^+$.

Das Ausgangsmaterial kann wie folgt hergestellt werden:

Eine Lösung von 300 mg (S)-4-[2-[4-(1-Methyl-thioformimidoyl)-benzoylamino]-propionyl]-phenoxyessigsäureethylester (Beispiel 31) und 175 mg O-tert-Butyl-dimethylsilyl-hydroxylamin in 10 ml THF wird 16 h bei Raumtemperatur gerührt, eingeengt und der Rückstand an Kieselgel (Hexan/Essigester 3:2) chromatographiert. Man erhält 95 mg (R,S)-4-[2-[4-[(tert-Butyl-dimethyl-silanyloxyamino)-imino-methyl]-benzoylamino]-propionyl]-phenoxyessigsäureethylester, MS (EI): 528 $(M+H)^+$.

Beispiel 33

Aus 350 mg (S)-4-[2-[4-(Amino-imino-methyl)-benzoylamino]-propionyl)-phenoxyessigsäure-tetrahydropyran-4-yl-ester hydrochlorid (1:0,8) hydrojodid (1:0,4) und 80 mg Ethylchloroformiat erhält man nach Beispiel 15 und Chromatographie des Rückstands an Kieselgel (Hexan/Essigester 1:2) 204 mg (S)-4-[2-[4-(Ethoxycarbonylamino-imino-methyl)-benzoylamino]-propionyl]-phenoxyessigsäure-tetrahydropyran-4-yl-ester, MS (ISP): 526 $(M+H)^+$.

Beispiel 34

Aus 2,2 g (S)-4-[2-(6-tert-Butoxycarbonylaminomethyl-pyridin-3-ylcarbonylamino)-propionyl]-phenoxyessigsäureethylester erhält man gemäss Beispiel 12 und Chromatographie des Rückstands an silyliertem Kieselgel RP18 (THF/Wasser-Gradient) 863 mg (S)-4-[2-(6-Aminomethyl-pyridin-3-ylcarbonylamino)-propionyl]-phenoxyessigsäureethylester, MS (ISP): 386 $(M+H)^+$.

Das Ausgangsmaterial kann wie folgt hergestellt werden:

Die Kupplung von 1,97 g entschütztem (S)-4-(2-tert-Butoxycarbonylamino-propionyl)-phenoxyessigsäureethylester mit 1,7 g 6-tert-Butoxycarbonylamino-methyl-pyridin-3-carbonsäure in Gegewart von 2 g TPTU und 1,25 g N-Methylmorpholin ergibt nach Beispiel 12e und Chromatographie an Kieselgel (Hexan/Essigester 1:2) 2,28 g (S)-4-[2-(6-tert-Butoxycarbonylaminomethyl-pyridin-3-ylcarbonylamino)-propionyl]-phenoxyessigsäureethylester, MS (ISP): 486 $(M+H)^+$.

Beispiel 35

Aus 90 mg (Z)-(R,S)-4-[2-[4-(Amino-hydroxyimino-methyl)-benzoylamino]-propionyl]-phenoxyessigsäureethylester und 25 mg Ethylchloroformiat erhält man gemäss Beispiel 15 und Kristallisation des Rückstands in Essigester/Hexan 57 mg (Z)-(R,S)-4-[2-[4-[Amino-ethoxycarbonyloximino-methyl]-benzoylamino]-propionyl]-phenoxyessigsäureethylester, MS (ISP): 486 $(M+H)^+$.

Beispiel 36

Die Reaktion von 500 mg (S)-4-[2-[4-(Amino-imino-methyl)-benzoylamino]-propionyl]-phenoxyessigsäure-ethylester-trifluoracetat mit Phenylchloroformiat gemäss Beispiel 15 führt nach Chromatographie an Kieselgel (Hexan/Essigester 1:2) zu 211 mg (S)-4-[2-[4-(Imino-phenoxycarbonylamino-methyl)-benzoylamino]-propionyl]-phenoxyessigsäureethylester, MS (ISP): 518 $(M+H)^+$.

Beispiel 37

Die Reaktion von 511 mg (S)-4-[2-[4-(Amino-imino-methyl)-benzoylamino]-propionyl]-phenoxyessigsäure-ethylester-trifluoracetat mit 4-Methoxyphenylchloroformiat nach Beispiel 15 führt nach Chromatographie an Kieselgel (Hexan/Essigester 1:2) zu 145 mg (S)-4-[2-[4-[Imino-(4-methoxy-phenoxycarbonylamino)-methyl]-benzoylamino]-propionyl]-phenoxyessigsäureethylester, MS (ISP): 548 $(M+H)^+$.

Beispiel 38

Zu einer Lösung von 102 mg Tetrahydro-2H-pyran-4-ol und 101 mg 4-Methoxymorpholin in 10 ml Dichlormethan werden bei 0 °C 98 mg Triphosgen in 5 ml Dichlormethan zugegeben und anschliessend 2 h bei Raumtemperatur gerührt. Diese Lösung wird zu einer Suspension von 511 mg (S)-4-[2-[4-(Amino-imino-methyl)-benzoylamino]-propionyl]-phenoxyessigsäureethylester trifluoracetat in 10 ml Dichlormethan und 10 ml gesättigter Natriumcarbonatlösung zugegeben, 5 min. bei Raumtemperatur gerührt und das Reaktionsgemisch wie in Beispiel 15 aufgearbeitet. Chromatographie des Rückstands an Kieselgel (Essigester) ergibt 350 mg (S)-4-[2-[4-[Imino-(tetrahydro-pyran-4-yloxycarbonylamino)-methyl]-benzoylamino]-propionyl]-phenoxyessigsäureethylester, MS (ISP): 526 $(M+H)^+$.

Beispiel 39

In Analogie zu Beispiel 38 werden auch 540 mg (S)-4-[2-[4-(Amino-imino-methyl)-benzoylamino]-propionyl)-phenoxyessigsäure-tetrahydropyran-4-yl-ester trifluoracetat (Beispiel 5) umgesetzt. Nach Chromatographie des Rückstands an Kieselgel (Essigester) erhält man 378 mg (S)-4-[2-[4-[Imino-(tetrahydro-pyran-4-yloxycarbonylamino)-methyl]-benzoylamino]- propionyl]-phenoxyessigsäure-tetrahydropyran-4-yl-ester, MS (ISP): 582 $(M+H)^+$.

Beispiel 40

Die Reaktion von 920 mg (S)-4-[2-[4-(Amino-imino-methyl)-benzoylamino]-propionyl]-phenoxyessigsäure-2-methoxy-ethylester trifluoracetat mit 2-Methoxy-ethylchloroformiat gemäss Beispiel 15 führt nach Chromatographie an Kieselgel (Hexan/Essigester 1:3) zu 430 mg (S)-4-[2-[4-[Imino-(2-methoxy-ethoxy-carbonylamino)-methyl]-benzoylamino]-propionyl]-phenoxyessigsäure-2-methoxy-ethylester, MS (ISP): 530 $(M+H)^+$.

Beispiel 41

Die Abspaltung der Schutzgruppe von 160 mg (S)-4-[2-[4-[(tert-Butyl-dimethyl-silanyloxyamino)-imino-methyl]-benzoylamino]-propionyl]-phenoxyessigsäureethylester mit Tetrabutylammoniumfluorid analog Beispiel 3b ergibt nach Kristallisation des Rückstands in Essigester/Hexan 187 mg (Z)-(S)-4-[2-[4-(Amino-hydroxyimino-methyl)-benzoylamino]-propionyl]-phenoxyessigsäureethylester, MS (ISP): 414 $(M+H)^+$, $[\alpha]_D^{20}$ = + 68,8° (c = 0,5, DMSO).

Das Ausgangsmaterial kann wie folgt hergestellt werden:

Aus 2 g 4-Cyanobenzoesäure erhält man durch analoge Reaktionen wie in Beispiel 32a N-tert-Butyl-dimethylsilyloxy-amidinobenzoesäure. Diese wird direkt als Rohmaterial für die Kupplung an (S)-4-(2-tert-Butoxycarbonylamino-propionyl)-phenoxyessigsäureethylester in Gegenwart von TPTU nach Beispiel 12e eingesetzt. Nach Chromatographie des Rückstands an Kieselgel (Hexan/Essigester 1:1) erhält man 561 mg (S)-4-[2-[4-[(tert-Butyl-dimethyl-silanyloxyamino)-imino-methyl]-benzoylamino]-propionyl]-phenoxyessigsäureethylester, $[\alpha]_D^{20}$ = + 62,0° (c = 0,5, Chloroform).

Beispiel 42

Die Umsetzung von 500 mg (S)-4-[2-[4-(Amino-imino-methyl)-benzoylamino]-propionyl]-phenoxyessigsäureethylester-trifluoracetat mit 4-Fluor-phenylchloroformiat analog zu Beispiel 15 und Chromatographie des Rückstands an Kieselgel (Hexan/Essigester 1:2) ergibt 100 mg (S)-4-[2-[4-[(4-Fluor-phenoxycarbonylamino)-imino-methyl]-benzoylamino]-propionyl]-phenoxyessigsäureethylester, MS (ISP): 536 $(M+H)^+$.

Beispiel 43

Ausgehend von 1 g (S)-4-[2-[4-(Amino-imino-methyl)-benzoylamino]-propionyl]-phenoxyessigsäureethylester-trifluoracetat wird in Analogie zu Beispiel 38 mit 2-tert-Butyl-dimethyl-silanyloxyethanol 200 mg (S)-4-[2-[4-[Amino-2-tert-butyl-dimethyl-silanyloxy-ethoxycarbonylimino-methyl]-benzoylamino]-propionyl]-phenoxyessigsäureethylester hergestellt und wie in Beispiel 3a entschützt und an Kieselgel (Essigester/Ethanol 95:5) chromatographiert. Man erhält 56 mg (S)-4-[2-[4-[Amino-(2-hydroxy-ethoxycarbonylimino)-methyl]-benzoylamino]-propionyl]-phenoxyessigsäureethylester, MS (ISP): 486 $(M+H)^+$.

Beispiel 44

Ausgehend von 527 mg (S)-4-(2-tert-Butoxycarbonylamino-propionyl)-phenoxyessigsäureethylester und 462 mg 4-(Amino-methoxyimino-methyl)-benzoesäure trifluoracetat erhält man wie in Beispiel 12e nach Chromatographie an Kieselgel (Hexan/Essigester 2:3) 500 mg (E/Z)-(S)-4-[2-[4-(Amino-methoxyimino-methyl)-benzoylamino]-propionyl]-phenoxyessigsäureethylester-trifluoracetat, MS (ISP): 428 $(M+H)^+$.

Das Ausgangsmaterial kann wie folgt hergestellt werden:

Eine Lösung von 1,47 g 4-Cyanobenzoesäure-tert-butylester wird analog Beispiel 31 mit Schwefelwasserstoff, Methyljodid und O-Methyl-hydroxylamin-hydrochlorid umgesetzt und das Rohprodukt an Kieselgel (Hexan/Essigester 9:1) chromatographiert. Man erhält 1,0 g 4-(Amino-methoxyimino-methyl)-benzoesäure-tert-butylester. Die Abspaltung der Estergruppe gemäss Beispiel 12 mit Trifluoressigsäure ergibt nach Kristalliation in Ether 1,13 g 4-(Amino-methoxyimino-methyl)-benzoesäure-trifluoracetat.

Beispiel 45

Wie in Beispiel 3b wird von 290 mg (S)-4-[2-[4-[(tert-Butyl-dimethyl-silanyloxyamino)-imino-methyl]-benzoylamino]-3-hydroxy-propionyl]-phenoxyessigsäureethylester mit Tetrabutylammoniumfluorid die Silylschutzgruppe abgespalten. Nach Chromatographie an Kieselgel (Essigester erhält man 145 mg (Z)-(S)-4-[2-[4-[Amino-hydroxyimino-methyl]-benzoylamino]-3-hydroxy-propionyl]-phenoxyessigsäureethylester.

Das Ausgangsmaterial kann wie folgt hergestellt werden:

Aus einer Lösung von 1,27 g 4-[(S)-3-tert-Butoxy-2-tert-butoxycarbonylamino-propionyl]-phenoxyessigsäureethylester in 25 ml Dichlormethan wird gemäss Beispiel 24a das freie Amin erhalten, das anschliessend analog 41a mit N-tert-Butyl-dimethylsilyloxy-amidinobenzoesäure umgesetzt wird. Nach Chromatographie des Rückstands an Kieselgel (Hexan/Essigester 1:2) erhält man 290 mg (S)-4-[2-[4-[(tert-Butyl-dimethyl-silanyloxyamino)-imino-methyl]-benzoylamino]-3-hydroxy-propionyl]-phenoxyessigsäureethylester, $R_f$-Wert: 0,68 (Choroform, Methanol, Essigsäure, 88:10:2).

Beispiel 46

Eine Lösung von 0.8 g [[4-(p-Amidino-N-methylbenzamido)acetyl-o-phenylen]dioxy]diessigsäure (J. Med. Chem. 1992, *35*, 4393-4407) in 2-Propanol/konz. Schwefelsäure (20:1) wird über Nacht stehengelassen. Nach Entfernen der Lösungsmittel wird der Rückstand in 50 ml Wasser aufgenommen, durch Zugabe von Natriumhydrogencarbonat neutral gestellt und mit 80 ml Dichlormethan unterschichtet. Nach Zugabe von 0.3 g Chlorameisensäureethylester gefolgt von 20 ml 0.5 N Natriumhydroxidlösung wird die organische Phase abgetrennt, mit Wasser gewaschen, über Natriumsulfat getrocknet und eingeengt. Nach Chromatographie an Kieselgel (Essigsäureethylester) und Kristallisation (Diethylether) erhält man 0.51 g (E und/oder Z)-[[4-((p-Amino-ethoxycarbonylimino-methyl)-N-methylbenzamido)acetyl-o-phenylen]dioxy]diessigsäure-diisopropylester. Smp. 56-58°C. MS (EI): 512 $(M+H)^+$.

Beispiel 47

Aus [[1-[N-(p-Amidinobenzoyl)-L-tyrosyl]-4-piperidinyl]oxy]essigsäure (J. Med. Chem. 1992, *35,* 4393-4407) wurde auf analoge Weise, wie in Beispiel 46 beschrieben, (E/Z)-(S)-1-[2-[4-(Amino-ethoxycarbonylimino-methyl)-benzoylamino]-3-(4-hydroxy-phenyl)-propionyl]-piperidin-4-yloxyessigsäureethylester erhalten. $[\alpha]_D^{20}$ = + 18.1° (c=0.8, EtOH). Smp. 84 °C. MS (ISP): 569 $(M+H)^+$.

Beispiel 48

Aus [[1-[N-(p-Amidinobenzoyl)-L-tyrosyl]-4-piperidinyl]oxy]essigsäure (J. Med. Chem. 1992, *35,* 4393-4407) wurde auf analoge Weise, wie in Beispiel 46 beschrieben, (E/Z)-(S)-1-[2-[4-(Amino-ethoxycarbonylimino-methyl)-benzoylamino]-3-(4-hydroxy-phenyl)-propionyl]-piperidin-4-yloxyessigsäure-isopropylester erhalten. Smp. 88-90°C. MS (ISP): 583 $(M+H)^+$.

Beispiel 49

Als Nebenprodukt der in Beispiel 48 beschriebenen Umsetzung konnte (E/Z)-(S)-1-[2-[4-(Amino-ethoxycarbonylimino-methyl)-benzoylamino]-3-(4-ethoxycarbonyloxy-phenyl)-propionyl]-piperidin-4-yloxyessigsäure-isopropylester isoliert werden. Smp. 71-73°C. MS (ISP): 655 $(M+H)^+$.

Beispiel 50

Aus der Umsetzung von [[1-[N-(p-Amidinobenzoyl)-L-tyrosyl]-4-piperidinyl]oxy]essigsäure mit Isatosäureanhydrid in Gegenwart von Kaliumcarbonat erhält man nach üblicher Aufarbeitung und chromatographischer Reinigung des Rohproduktes 2-Amino-benzoesäure-(E/Z)-(S)-4-[2-[4-(amino-ethoxycarbonylimino-methyl)-benzoylamino]-3-(4-isopropoxycarbonylmethoxy-piperidin-1-yl)-3-oxo-propyl]-phenylester in Form eines farblosen Schaumes. MS (ISP): 702 $(M+H)^+$.

Beispiel 51

Aus der Umsetzung von [[1-[N-(p-Amidinobenzoyl)-L-tyrosyl]-4-piperidinyl]oxy]essigsäure mit Furan-2-carbonsäurechlorid in Gegenwart von Kaliumcarbonat erhält man nach üblicher Aufarbeitung und chromatographischer Reinigung des Rohproduktes Furan-2-carbonsäure-(E/Z)-(S)-4-[2-[4-(amino-ethoxycarbonylimino-methyl)-benzoylamino]-3-(4-isopropoxycarbonylmethoxy-piperidin-1-yl)-3-oxo-propyl]-phenylester in Form eines schwach gelben Schaumes. MS (ISP): 677 $(M+H)^+$.

Beispiel 52

Aus der Umsetzung von [[1-[N-(p-Amidinobenzoyl)-L-tyrosyl]-4-piperidinyl]oxy]essigsäure mit Acetanhydrid in Gegenwart von Kaliumcarbonat erhält man nach üblicher Aufarbeitung und chromatographischer Reinigung des Rohproduktes (E/Z)-(S)-1-[3-[4-Acetoxy-phenyl)-2-[4-(amino-ethoxycarbonylimino-methyl)-benzoylamino]-propionyl]-piperidin-4-yloxy essigsäure-isopropylester in Form eines farblosen Schaumes. MS (ISP): 625 $(M+H)^+$.

Beispiel 53

Aus der Umsetzung von[[1-[N-(p-Amidinobenzoyl)-L-tyrosyl]-4-piperidinyl]oxy]essigsäure mit Acetylsalicylsäurechlorid in Gegenwart von Triethylamin erhält man nach üblicher Aufarbeitung und chromatographischer Reinigung des Rohproduktes 2-Acetoxy-benzoesäure-(E/Z)-(S)-4-[2-[4-(amino-ethoxycarbonylimino-methyl)-benzoylamino]-3-(4-isopropoxycarbonylmethoxy-piperidin-1-yl)-3-oxo-propyl]-phenylester. Smp. 77-80 °C. MS (EI): 745 $(M+H)^+$.

Beispiel 54

Aus der Umsetzung von [[1-[N-(p-Amidinobenzoyl)-L-tyrosyl]-4-piperidinyl]oxy]essigsäure-tert-butyl ester (EP 505868) mit Chlorameisensäureethylester, wie in Beispiel 46 beschrieben, gefolgt von der Behandlung mit konz. Ameisensäure isoliert man nach üblicher Aufarbeitung und Chromatographie (E/Z)-(S)-1-[2-[4-(Amino-ethoxycarbonylimino-methyl)-benzoylamino]-3-(4-hydroxy-phenyl)-propionyl]-piperidin-4-yloxyessigsäure. Smp. 134 °C. MS (ISN): 539 $(M-H)^+$.

Beispiel 55

Aus der Umsetzung von (S)-1-[2-(5-Amidinopyridin-2-ylcarbonylamino)-3-(4-methoxy-phenyl)-propionyl]-piperidin-4-yloxyessigsäure (EP 505868) in analoger Weise, wie in Beispiel 46 beschrieben, erhält man (E/Z)-(S)-1-[2-(5-Amino-ethoxycarbonylimino-methylpyridin-2-ylcarbonyl-amino)-3-(4-methoxy-phenyl)-propionyl]-piperidin-4-yloxy-essigsäure-ethylester. $[\alpha]_D^{20}$ = + 22.6° (c=1.0, EtOH). Smp. 52-54 °C. MS (ISP): 584 $(M+H)^+$.

Beispiel 56

Die Umsetzung von (S)-1-[3-(4-Hydroxy-phenyl)-2-[4-(imino-propylamino-methyl)-benzoylamino]-propionyl]-piperidin-4-yloxyessigsäure-tert-butylester mit konz. Ameisensäure ergibt nach chromatographischer Reinigung des Rohproduktes an Kieselgel RP 18 mit einem Wasser/Methanol-Gradienten (S)-1-[3-(4-Hydroxy-phenyl)-2-[4-(imino-propylamino-methyl)-benzoylamino]-propionyl]-piperidin-4-yloxyessigsäure. Smp. 160 $^oC$. MS (ISP): 511 $(M+H)^+$.

Das Ausgangsmaterial kann wie folgt hergestellt werden:

a) Die Kupplung von Z-Tyr-OH mit 4-Piperidinyloxyessigsäure-tert-butyl ester (J. Med. Chem. 1992, *35,* 4393-4407) gefolgt von hydrogenolytischer Entfernung der Z-Schutzgruppe ergibt 1-[[L-Tyrosyl]-4-piperidinyloxy]-essigsäure-tert-butyl ester.

b) Durch Umsetzung des Produktes der Vorstufe mit 4-Cyano-benzoylchlorid in Gegenwart von Natriumhydrogencarbonat erhält man (S)-1-[3-(4-Hydroxy-phenyl)-2-[4-cyano-benzoylamino]-propionyl]-piperidin-4-yloxyessigsäure-tert-butylester.

c) Durch aufeinander folgende Behandlung des Produktes der Vorstufe mit Schwefelwasserstoff in Pyridin, Methyliodid in Aceton und n-Propylamin in einem Gemisch von Methanol und Essigsäure erhält man (S)-1-[3-(4-Hydroxy-phenyl)-2-[4-(imino-propylamino-methyl)-benzoylamino]-propionyl]-piperidin-4-yloxyessigsäure-tert-butylester. MS (ISP): 567 $(M+H)^+$.

Beispiel 57

Die Umsetzung von (S)-4-[[[4-[1-(4-tert-Butoxycarbonylmethoxy-piperidin-1-carbonyl)-2-(4-hydroxy-phenyl)-ethylcarbamoyl]-phenyl]-imino-methyl]-amino]-buttersäure mit konz. Ameisensäure ergibt nach chromatographischer Reinigung des Rohproduktes die (S)-4-[[[4-[1-(4-carboxy-methoxy-piperidine-1-carbonyl)-2-(4-hydroxy-phenyl)-ethylcarbamoyl]-phenyl]-imino-methyl]-amino]-buttersäure. Smp. 156-160 $^oC$. MS (ISP): 555 $(M+H)^+$.

Das Ausgangsmaterial kann wie folgt hergestellt werden:

Durch aufeinander folgende Behandlung von (S)-1-[3-(4-Hydroxy-phenyl)-2-[4-cyano-benzoylamino]-propionyl]-piperidin-4-yloxyessigsäure-tert-butylester (Beispiel 56b)) mit Schwefelwasserstoff in Pyridin, Methyljodid in Aceton und 4-Aminobuttersäure in einem Gemisch von Methanol und Essigsäure erhält man (S)-4-[[[4-[1-(4-tert-Butoxycarbonylmethoxy-piperidin-1-carbonyl)-2-(4-hydroxy-phenyl)-ethylcarbamoyl]-phenyl]-imino-methyl]-amino]-buttersäure in Form eines farblosen Schaumes. MS (ISP): 611 $(M+H)^+$.

Beispiel 58

Aus der Umsetzung von [[1-[N-(p-Amidinobenzoyl)-L-alanyl]-4-piperidinyl]oxy]essigsäure-tert-butyl ester (J. Med. Chem. 1992, *35*, 4393-4407) mit Chlorameisensäureethylester, wie in Beispiel 46 beschrieben, gefolgt von der Behandlung mit konz. Ameisensäure isoliert man nach üblicher Aufarbeitung und Chromatographie (E/Z)-(S)-[1-[2-[4-(Amino-ethoxycarbonylimino-methyl)-benzoylamino]-propionyl]-piperidin-4-yloxy]-essigsäure in Form eines farblosen Schaumes. MS (ISP): 449 $(M+H)^+$.

Beispiel 59

Aus [[1-[N-(p-Amidinobenzoyl)-L-alanyl]-4-piperidinyl]oxy]essigsäure (J. Med. Chem. 1992, *35,* 4393-4407) wurde auf analoge Weise, wie in Beispiel 46 beschrieben, (E/Z)-(S)-1-[2-[4-(Amino-ethoxycarbonylimino-methyl)-benzoylamino]-propionyl]-piperidin-4-yloxyessigsäure-ethylester in Form eines farblosen Schaumes erhalten. $[\alpha]_D^{20}$ = + 41.7$^o$ (c=1.0, EtOH). MS (ISP): 477 $(M+H)^+$.

Beispiel 60

Aus [[1-[N-(p-Amidinobenzoyl)-L-alanyl]-4-piperidinyl]oxy]essigsäure wurde auf analoge Weise, wie in Beispiel 46 beschrieben, (E/Z)-(S)-1-[2-[4-(Amino-ethoxycarbonylimino-methyl)-benzoylamino]-propionyl]-piperidin-4-yloxyessigsäure-isopropylester in Form eines farblosen Schaumes erhalten. MS (ISP): 491 $(M+H)^+$.

Beispiel 61

Aus [[1-[N-(p-Amidinobenzoyl)-L-alanyl]-4-piperidinyl]oxy]essigsäure wurde auf analoge Weise, wie in Beispiel 46 beschrieben, (E/Z)-(S)-1-[2-[4-(Amino-n-butoxycarbonylimino-methyl)-benzoylamino]-propionyl]-piperidin-4-yloxyessigsäure-ethylester in Form eines farblosen Schaumes erhalten. $[\alpha]_D^{20}$ = + 37.4° (c=0.8, EtOH). MS (ISP): 505 $(M+H)^+$.

Beispiel 62

Aus [[1-[N-(p-Amidinobenzoyl)-L-alanyl]-4-piperidinyl]oxy]essigsäure wurde auf analoge Weise, wie in Beispiel 46 beschrieben, (E/Z)-(S)-1-[2-[4-(Amino-methoxyethoxycarbonylimino-methyl)-benzoylamino]-propionyl]-piperidin-4-yloxyessigsäure-ethylester in Form eines farblosen Schaumes erhalten. $[\alpha]_D^{20}$ = + 40.0° (c=0.9, EtOH). MS (ISP): 507 $(M+H)^+$.

Beispiel 63

Durch Veresterung von (S)-1-[2-(5-Amino-imino-methyl-pyridin-2-ylcarbonylamino)-propionyl]-piperidin-4-yloxy-essigsäure (J. Med. Chem. 1992, *35,* 4393-4407) mit Tetrahydro-2H-pyran-4-ol in Gegenwart von p-Toluolsulfonsäure erhält man nach chromatographischer Reinigung des Rohproduktes an Silicagel RP 18 mit einem Wasser/Acetonitril-Gradienten das p-Toluolsulfonatsalz von (S)-1-[2-(5-Amino-imino-methyl-pyridin-2-ylcarbonylamino)-propionyl]-piperidin-4-yloxy-essigsäure-tetrahydropyran-4-ylester. $[\alpha]_D^{20}$ = + 28.8° (c=0.5, MeOH). MS (ISP): 462 $(M+H)^+$.

Beispiel 64

Aus (S)-1-[2-(5-Amino-imino-methyl-pyridin-2-ylcarbonylamino)-propionyl]-piperidin-4-yloxy-essigsäure wurde in analoger Weise, wie in Beispiel 46 beschrieben, (E/Z)-(S)-1-[2-(5-Amino-ethoxycarbonylimino-methyl-pyridin-2-ylcarbonylamino)-propionyl]-piperidin-4-yloxy-essigsäure-isopropylester, $[\alpha]_D^{20}$ = + 42.6° (c=1.0, EtOH). Smp. 62-64 °C. MS (EI): 492 $(M+H)^+$, erhalten.

Beispiel 65

Aus [[1-[(p-Amidino-N-methylbenzamido)acetyl]-4-piperidinyl]oxy]essigsäure (EP 505868) wurde in analoger Weise, wie in Beispiel 46 beschrieben, (E/Z)-[1-[2-[4-(Amino-ethoxycarbonylimino-methyl)-benzoyl]-methyl-amino]-acetyl]-piperidin-4-yloxy-essigsäure-ethylester, MS (ISP): 477 $(M+H)^+$, erhalten.

Beispiel 66

Aus (E/Z)-(S)-1-[2-[4-(Amino-methoxyimino-methyl)-benzoylamino]-propionyl]-piperidin-4-yloxyessigsäure-tert-butylester erhält man durch Behandlung mit Trifluoressigsäure in Dichlormethan (E/Z)-(S)-1-[2-[4-(Amino-methoxyimino-methyl)-benzoylamino]-propionyl]-piperidin-4-yloxyessigsäure. Smp. 193-195 °C. MS (ISP): 407 $(M+H)^+$.

Das Ausgangsmaterial kann wie folgt hergestellt werden:

a) Die Kupplung von Z-Ala-OH mit 4-Piperidinyloxyessigsäure-tert-butyl ester (J. Med. Chem. 1992, *35,* 4393-4407) gefolgt von hydrogenolytischer Entfernung der Z-Schutzgruppe ergibt 1-[[L-Alanyl]-4-piperidinyloxy]essigsäure-tert-butyl ester.

b) Durch Umsetzung des Produktes der Vorstufe mit 4-Cyano-benzoylchlorid in Gegenwart von Natriumhydrogencarbonat erhält man (S)-1-[2-[4-cyano-benzoylamino]-propionyl]-piperidin-4-yloxyessigsäure-tert-butylester.

c) Durch aufeinander folgende Behandlung des Produktes der Vorstufe mit Schwefelwasserstoff in Pyridin, Methyliodid in Aceton und O-Methyl-hydroxylaminhydrochlorid in DMF in Gegenwart von Triethylamin erhält man (E/Z)-(S)-1-[2-[4-(Amino-methoxyimino-methyl)-benzoylamino]-propionyl]-piperidin-4-yloxyessigsäure-tert-butylester in Form eines zähen, farblosen Öles. MS (ISP): 463 $(M+H)^+$.

Beispiel 67

Durch Veresterung von (E/Z)-(S)-1-[2-[4-(Amino-methoxyimino-methyl)-benzoylamino]-propionyl]-piperidin-4-yloxyessigsäure in Ethanol in Gegenwart von konz. Schwefelsäure erhält man (E/Z)-(S)-1-[2-[4-(Amino-methoxyimino-methyl)-benzoylamino]-propionyl]-piperidin-4-yloxyessigsäure-ethylester als farbloses Harz. MS (ISP): 435 $(M+H)^+$.

Beispiel 68

Aus (S)-1-[2-[4-tert-Butoxycarbonylaminomethyl-benzoylamino)-3-(4-hydroxy-phenyl)-propionyl]-piperidin-4-yloxyessigsäure-tert-butylester erhält man durch Behandlung mit Ameisensäure (S)-1-[2-(4-Aminomethyl-benzoylamino)-3-(4-hydroxy-phenyl)-propionyl]-piperidin-4-yloxyessigsäure. $[\alpha]_D^{20}$ = + 19.4° (c=0.5, $H_2O$). Smp. 166-168 °C. MS (ISN): 454 $(M-H)^+$.

Das Ausgangsmaterial kann durch Kupplung von 1-[[L-Tyrosyl]-4-piperidinyloxy]essigsäure-tert-butyl ester (Beispiel 56a)) mit 4-tert-Butoxycarbonylaminomethyl-benzoesäure in Gegenwart von 1-Ethoxycarbonyl-2-ethoxy-1,2-dihydrochinolin (EEDQ) erhalten werden.

Beispiel 69

Aus (S)-1-[2-[4-tert-Butoxycarbonylaminomethyl-benzoylamino)-3-(4-hydroxy-phenyl)-propionyl]-piperidin-4-yloxyessigsäure-tert-butylester (Beispiel 68) erhält man durch Behandlung mit Methyliodid/Kaliumcarbonat gefolgt von Ameisensäure (S)-1-[2-(4-Aminomethyl-benzoylamino)-3-(4-methoxy-phenyl)-propionyl]-piperidin-4-yloxyessigsäure. $[\alpha]_D^{20}$ = + 11.7° (c=0.5, $H_2O$). Smp. 130 °C. MS (CI): 470 $(M+H)^+$.

Beispiel 70

Durch Veresterung von (S)-1-[2-(4-Aminomethyl-benzoylamino)-3-(4-methoxy-phenyl)-propionyl]-piperidin-4-yloxyessigsäure mit Isopropanol in Gegenwart von Schwefelsäure erhält man (S)-1-[2-(4-Aminomethylbenzoylamino)-3-(4-methoxy-phenyl)-propionyl]-piperidin-4-yloxyessigsäure-isopropylester als Hemisulfatsalz. $[\alpha]_D^{20}$ = + 5.1° (c=0.8, $H_2O$). Smp. 138-140 °C. MS (EI): 512 $(M+H)^+$.

Beispiel 71

Aus (S)-1-[2-(5-Aminomethyl-pyridin-2-ylcarbonylamino)-3-(4-methoxy-phenyl)-propionyl]-piperidin-4-yloxyessigsäure-tert-butylester erhält man durch Behandlung mit Ameisensäure (S)-1-[2-(5-Aminomethyl-pyridin-2-ylcarbonylamino)-3-(4-methoxy-phenyl)-propionyl]-piperidin-4-yloxyessigsäure. $[\alpha]_D^{20}$ = + 20.0 ° (c=0.6, $H_2O$). Smp. 174 °C (dec.). MS (ISP): 471 $(M+H)^+$.

Das Ausgangsmaterial kann durch katalytische Hydrierung von (S)-1-[2-(5-Cyan-pyridin-2-ylcarbonylamino)-3-(4-methoxy-phenyl)-propionyl]-piperidin-4-yloxyessigsäure-tert-butylester (EP 505 868) über Palladiumkohle in einem Gemisch von Methanol/Wasser/Essigsäure hergestellt werden.

Beispiel 72

Aus (S)-1-[2-(5-Aminomethyl-pyridin-2-ylcarbonylamino)-3-(4-methoxyphenyl)-propionyl]-piperidin-4-yloxyessigsäure erhält man durch Veresterung mit Isopropanol, wie in Beispiel 70 beschrieben, nach Ansäuern des Rohproduktes mit Salzsäure das Hydrochlorid von (S)-1-[2-(5-Aminomethyl-pyridin-2-ylcarbonylamino)-3-(4-methoxy-phenyl)-propionyl]-piperidin-4-yloxyessigsäure-isopropylester. Smp. 82-84 °C (aus Diethylether). MS (ISP): 513 $(M+H)^+$.

Referenzbeispiel 73

Aus (S)-4-[1-(4-tert-Butoxycarbonylmethoxy-piperidin-1ylcarbonyl)-2-(4-hydroxy-phenyl)-ethylcarbamoylmethoxy]-piperidin-1-carbonsäure-tert-butylester erhält man durch Behandlung mit Ameisensäure (S)-1-[3-(4-Hydroxy-phenyl)-2-piperidin-4-yloxyacetylamino-propionyl]-piperidin-4-yloxyessigsäure. $[\alpha]_D^{20}$ = + 6.7 ° (c=0.7, $H_2O$). Smp. 156 °C (dec.). MS (ISP): 464 $(M+H)^+$.

Das Ausgangsmaterial kann wie folgt hergestellt werden:

a) Durch Behandlung von 4-Piperidinyloxyessigsäure-tert-butylester (J. Med. Chem. 1992, *35,* 4393-4407) mit Ameisensäure erhält man 4-Piperidinyloxyessigsäure, welche mit Di-tert-butyl-dicarbonat in Dioxan in Gegenwart von Natronlauge zu 1-tert-Butoxycarbonyl-piperidin-4-yloxyessigsäure umgesetzt werden kann.

b) Das Produkt der Vorstufe kann mit 1-[[L-Tyrosyl]-4-piperidinyloxy]essigsäure-tert-butyl ester (Beispiel 56a)) in Gegenwart von 1-Ethyloxycarbonyl-2-isobutyloxy-1,2-dihydrochinolin in Dichlormethan zu (S)-4-[1-(4-tert-Butoxycarbonylmethoxy-piperidin-1-ylcarbonyl)-2-(4-hydroxy-phenyl)-ethylcarbamoylmethoxy]-piperidin-1-carbonsäure-tert-butylester gekoppelt werden. MS (ISP): 620 $(M+H)^+$.

Referenzbeispiel 74

Durch Umsetzung von 2-Acetoxy-benzoesäure-(S)-4-[2-[1-tert-butoxycarbonyl-piperidin-4-yloxyacetylamino]-3-(4-ethoxycarbonylmethoxy-piperidin-1-yl)-3-oxo-propyl]-phenylester mit Ameisensäure erhält man nach Zugabe von Salzsäure zum Rohprodukt das Hydrochloridsalz von 2-Acetoxy-benzoesäure-(S)-4-[2-[piperidin-4-yloxyacetylamino]-3-(4-ethoxycarbonylmethoxy-piperidin-1-yl)-3-oxo-propyl]-phenylester. Smp. 74-76 $^{o}C$ (dec.). MS (ISP): 654 $(M+H)^+$.

Das Ausgangsmaterial kann wie folgt erhalten werden:

a) Die Kupplung von Z-Tyr-OH mit 4-Piperidinyloxyessigsäure-ethylester (erhalten durch saure Umesterung von 4-Piperidinyloxyessigsäure-tert-butylester, in Gegenwart von 1-Ethyloxycarbonyl-2-isobutyloxy-1,2-dihydrochinolin) gefolgt von der Umsetzung mit Acetylsalicylsäurechlorid in Gegenwart von Kaliumcarbonat und anschliessender hydrogenolytischer Entfernung der Z-Schutzgruppe ergibt 2-Acetoxy-benzoesäure-(S)-4-[2-amino-3-(4-ethoxycarbonylmethoxy-piperidin-1-yl)-3-oxo-propyl]-phenylester.
b) Durch Kupplung des Produktes der Vorstufe mit 1-tert-Butoxycarbonyl-piperidin-4-yloxyessigsäure (Beispiel 73b)) in Gegenwart von 1-Ethyloxycarbonyl-2-isobutyloxy-1,2-dihydrochinolin in Dichlormethan erhält man 2-Acetoxy-benzoesäure-(S)-4-[2-[1-tert-butoxycarbonyl-piperidin-4-yloxyacetylamino]-3-(4-ethoxycarbonylmethoxy-piperidin-1-yl)-3-oxo-propyl]-phenylester. MS (ISP): 754 $(M+H)^+$.

Beispiel 75

Durch Veresterung von (E/Z)-(S)-1-[2-[4-(Amino-hydroxyimino-methyl)-benzoylamino]-propionyl]-piperidin-4-yloxyessigsäure in Ethanol in Gegenwart von konz. Schwefelsäure erhält man (E/Z)-(S)-1-[2-[4-(Amino-hydroxyimino-methyl)-benzoylamino]-propionyl]-piperidin-4-yloxyessigsäure-ethylester in Form von watteartigen Kristallen. Smp. 205-207 $^{o}C$. MS (ISP): 421 $(M+H)^+$.

Das Ausgangsmaterial kann wie folgt hergestellt werden:

a) Durch Umsetzung von (S)-1-[2-[4-cyano-benzoylamino]-propionyl]-piperidin-4-yloxyessigsäure-tert-butylester (Beispiel 66) mit Hydroxylamin-hydrochlorid in Methanol in Gegenwart von Natriummethanolat erhält man nach Rühren über Nacht und üblicher Aufarbeitung (E/Z)-(S)-1-[2-[4-(Amino-hydroxyimino-methyl)-benzoylamino]-propionyl]-piperidin-4-yloxyessigsäure-tert-butylester. Smp. 193-194 $^{o}C$. MS (ISP): 449 $(M+H)^+$.
b) Durch Behandlung des Produktes der Vorstufe mit Ameisensäure bei 50 $^{o}C$ erhält man nach Kristallisation aus Essigester (E/Z)-(S)-1-[2-[4-(Amino-hydroxyimino-methyl)-benzoylamino]-propionyl]-piperidin-4-yloxyessigsäure. Smp. 176-178 $^{o}C$. MS (ISN): 391 $(M+H)^+$.

Beispiel 76

Durch Veresterung von (E/Z)-(S)-1-[2-[4-(Amino-hydroxyimino-methyl)-benzoylamino]-propionyl]-piperidin-4-yloxyessigsäure (Beispiel 75b) in 2-Propanol in Gegenwart von konz. Schwefelsäure erhält man (E/Z)-(S)-1-[2-[4-(Amino-hydroxyimino-methyl)-benzoylamino]-propionyl]-piperidin-4-yloxyessigsäure-isopropylester in Form von watteartigen Kristallen. Smp. 205-207 $^{o}C$. MS (ISP): 435 $(M+H)^+$.

Beispiel 77

220 mg [1-[4-[4-(tert-Butoxycarbonylamino-imino-methyl)-phenyl]-4-oxo-butyryl]-piperidin-4-yloxy]-essigsäure-tert-butylester und 3.4 ml Ameisensäure werden 24 h bei 20°C gerührt. Das Reaktionsgemisch wird im Vakuum eingedampft, der Rückstand in Wasser gelöst und wieder eingedampft. Aus Methanol kristallisieren 120 mg [1-[4-[4-(Amino-imino-methyl)-phenyl]-4-oxo-butyryl]-piperidin-4-yloxy]-essigsäure, Smp. > 250°C, MS: 362 (100, M+H).

Das Ausgangsmaterial kann wie folgt erhalten werden.

a) 4-(4-Cyano-phenyl)-4-oxo-buttersäure wird in THF mit 2-Chloro-4,6-dimethoxy-1,3,5-triazin und N-Methylmorpholin aktiviert und dann mit Piperidin-4-yloxy-essigsäure-tert-butylester umgesetzt zu [1-[4-(4-Cyano-phenyl)-4-oxo-butyryl]-piperidin-4-yloxy]-essigsäure-tert-butylester.
b) Dieser wird in Pyridin und Triethylamin mit Schwefelwasserstoff zum [1-[4-Oxo-4-(4-thiocarbamoyl-phenyl)-butyryl]-piperidin-4-yloxy]-essigsäure-tert-butylester, Smp. 140°C, umgesetzt.
c) Letzterer wird zunächst mit Methyljodid in Aceton, dann mit Ammoniumacetat und Essigsäure in Methanol und schliesslich mit Di-tert-butyldicarbonat in DMF-Triethylamin zum [1-[4-[4-(tert-Butoxycarbonylamino-imino-methyl)-phenyl]-4-oxo-butyryl]-piperidin-4-yloxy]-essigsäure-tert-butylester umgesetzt. MS: 518 (100, M+H).

Beispiel 78

76 mg [1-[4-[4-(tert-Butoxycarbonylamino-imino-methyl)-phenyl]-4-oxo-butyryl]-piperidin-4-yloxy]-essigsäureethylester werden in 0.5 ml Methylenchlorid und 0.5 ml Trifluoressigsäure 2 h bei 20°C gerührt. Das Reaktionsgemisch wird im Vakuum eingedampft, der Rückstand in Alkohol gelöst und wieder eingedampft. Das kristalline Produkt wird mit Ether verrührt, abgenutscht und getrocknet. Man erhält 74 mg [1-[4-[4-(Amino-imino-methyl)-phenyl]-4-oxo-butyryl]-piperidin-4-yloxy]-essigsäureethylester-trifluoracetat (1:1), Smp. 188-190°C.

Das Ausgangsmaterial kann wie folgt erhalten werden:

a) [1-[4-[4-(Amino-imino-methyl)-phenyl]-4-oxo-butyryl]-piperidin-4-yloxy]-essigsäure wird in 1N HCl in Ethanol in dessen Ethylester übergeführt.
b) Dieser wird mit Di-tert-butyldicarbonat in DMF-Triethylamin zum Ausgangsmaterial umgesetzt, Smp. 100°C.

Beispiel 79

Ein Gemisch von 160 mg [1-[[[4-(Amino-imino-methyl)-benzoyl]-(2-methoxyethyl)-amino]-acetyl]-piperidin-4-yloxy]-essigsäure-isopropylester-hydrochlorid (1:1), 3.2 ml Methylenchlorid, 2.6 ml Wasser und 0.6 ml gesättigter Natriumcarbonatlösung wird mit 0.036 ml Chlorameisensäureethylester versetzt und 2 h bei 20°C gut gerührt. Das Reaktionsgemisch wird mit Methylenchlorid verdünnt, mit Wasser und Kochsalzlösung gewaschen, getrocknet und im Vakuum eingedampft. Chromatographie des Rückstandes an Kieselgel mit Methylenchlorid-Isopropanol gibt 109 mg [1-[[[4-(Ethoxycarbonylamino-imino-methyl)-benzoyl)-(2-methoxy-ethyl)-amino]-acetyl]-piperidin-4-yloxy]-essigsäure-isopropylester als farblosen Schaum. MS: 535 (100, M+H).

Das Ausgangsmaterial erhält man aus [1-[[[4-(Amino-imino-methyl)-benzoyl]-(2-methoxy-ethyl)-amino]-acetyl]-piperidin-4-yloxy]-essigsäure (EP 505 868) in 1N HCl in Isopropanol bei 20°C.

Beispiel 80

In Analogie zu Beispiel 79 erhält man aus dem [1-[4-[4-(Amino-imino-methyl)-phenyl]-4-oxo-butyryl]-piperidin-4-yloxy]-essigsäureisopropylesterjodid-acetat den [1-[4-[4-(Ethoxycarbonylamino-imino-methyl)-phenyl]-4-oxo-butyryl]-piperidin-4-yloxy]-essigsäureisopropylester, Smp. 86-89°C.

Das Ausgangsmaterial kann wie folgt hergestellt werden:

a) [1-[4-Oxo-4-(4-thiocarbamoyl-phenyl)-butyryl]-piperidin-4-yloxy]-essigsäure-tert-butylester wird in Methylenchlorid und Trifluoressigsäure zur [1-[4-Oxo-4-(4-thiocarbamoyl-phenyl)-butyryl]-piperidin-4-yloxy]-essigsäure, Smp. 203-207°C, gespalten.
b) Daraus erhält man mit Schwefelsäure in Isopropanol den [1-[4-Oxo-4-(4-thiocarbamoyl-phenyl)-butyryl]-piperidin-4-yloxy]-essigsäureisopropylester, Smp. 123-128°C.
c) Dieser wird mit Methyljodid in Aceton und anschliessend mit Ammoniumacetat und Essigsäure in Methanol zum Ausgangsmaterial umgesetzt.

Beispiel 81

In Analogie zu Beispiel 79 erhält man aus dem [1-[4-[4-(Amino-imino-methyl)-phenyl]-4-oxo-butyryl]-piperidin-4-yloxy]-essigsäure-isopropylesterjodid-acetat und Chlorameisensäureisobutylester den [1-[4-[4-(Isobutoxycarbonylamino-imino-methyl)-phenyl]-4-oxo-butyryl]-piperidin-4-yloxy]-essigsäureisopropylester, Smp. 94°C.

Beispiel 82

1.7 g (RS)-[1-[4-[4-(tert-Butoxycarbonylamino-imino-methyl)-phenyl]-2-methyl-4-oxo-butyryl]-piperidin-4-yloxy]-essigsäure-tert-butylester werden in 8.5 ml Methylenchlorid und 8.5 ml Trifluoressigsäure 3 h bei 20°C gerührt. Nach dem Eindampfen des Lösungsmittels im Vakuum wird der Rückstand in Wasser gelöst und die Lösung wieder eingedampft. Nach dem Trocknen des Rückstandes wird in Alkohol verrührt, abgenutscht und getrocknet. Man erhält 1.13 g (RS)-[1-[4-[4-(Amino-imino-methyl)-phenyl]-2-methyl-4-oxo-butyryl]-piperidin-4-yloxy-essigsäure-trifluoracetat (1:1), Smp. 217°C.

Das Ausgangsmaterial kann wie folgt erhalten werden:

a) 4-(4-Amino-phenyl)-2-methyl-4-oxo-buttersäure wird über die entsprechende Diazoniumverbindung (Sandmeyer-Reaktion) zur 4-(4-Cyano-phenyl)-2-methyl-4-oxo-buttersäure, Smp. 137°C, umgesetzt.

b) Wie im Beispiel 77a) beschrieben wird diese mit Piperidin-4-yloxy-essigsäure-tert-butylester gekoppelt zum (RS)-[1-[4-(4-Cyano-phenyl)-2-methyl-4-oxo-butyryl]-piperidin]-4-yloxy-essigsäure-tert-butylester, Smp. 114-116°C.

c) Mit Schwefelwasserstoff in Pyridin/Triethylamin erhält man daraus den (RS)-[1-[2-Methyl-4-oxo-4-(4-thiocarbamoyl-phenyl)-butyryl]-piperidin-4-yloxy]-essigsäure-tert-butylester, Smp. 152-155°C.

d) Dieser wird zunächst mit Methyljodid in Aceton, dann mit Ammoniumacetat und Essigsaure in Methanol und anschliessend mit Di-tert-butyl-dicarbonat in Methylenchlorid und wässriger Natriumcarbonatlösung zum Ausgangsmaterial umgesetzt, MS: 532 (100, M+H).

Beispiel 83

734 mg (RS)-[1-[4-[4-(Amino-imino-methyl)-phenyl]-2-methyl-4-oxo-butyryl]-piperidin-4-yloxy]-essigsäure-trifluoracetat werden in 15 ml 1N HCl in Isopropanol 19 h bei ca. 20°C gerührt. Nach Abdampfen des Lösungsmittels und Trocknen im Hochvakuum erhält man 650 mg (RS)-[1-[4-[4-(Amino-imino-methyl)-phenyl]-2-methyl-4-oxo-butyryl]-piperidin-4-yloxy]-essigsäure-isopropylester-hydrochlorid (1:1) als hygroskopisches amorphes Pulver, MS: 376 (100, M+H).

Beispiel 84

In Analogie zu Beispiel 79 erhält man aus dem (RS)-[1-[4-[4-(Amino-imino-methyl)-phenyl]-2-methyl-4-oxo-butyryl]-piperidin-4-yloxy]-essigsäure-isopropylester-hydrochlorid den (RS)-[1-[4-[4-(Ethoxycarbonylamino-imino-methyl)-phenyl-2-methyl-4-oxo-butyryl]-piperidin-4-yloxy]-essigsäure-isopropylester als harzigen Schaum, MS: 490 (100, M+H).

Beispiel 85

Ebenso erhält man aus dem gleichen Ausgangsmaterial mit Chlorameisensäure-isobutylester den (RS)-[1-[4-[4-(Isobutoxycarbonylamino-imino-methyl)-phenyl]-2-methyl-4-oxo-butyryl]-piperidin-4-yloxy]-essigsäure-isopropylester als Schaum, MS: 518 (100, M+H).

Beispiel 86

209 mg (RS)-2-[4-(tert-Butoxycarbonylamino-imino-methyl)-benzoyl]-4-(4-ethoxycarbonylmethoxy-phenyl)-4-oxo-buttersäureethylester und 4.2 ml Ameisensäure werden 6,5 h bei 20°C stehen gelassen. Das Reaktionsgemisch wird eingedampft, der Rückstand in Wasser gelöst und wieder eingedampft. Der getrocknete Rückstand wird in Aether verrührt und abgenutscht. Es werden 158 mg (RS)-2-[4-(Amino-imino-methyl)-benzoyl]-4-(4-ethoxycarbonylmethoxy-phenyl)-4-oxo-buttersäureethylester-formiat (1:1), Smp. 162°C, erhalten.

Das Ausgangsmaterial kann wie folgt erhalten werden:

a) Aus 4-Cyanobenzoylessigsäureethylester und [4-(Bromoacetyl)phenoxy]-essigsäureethylester erhält man in Aceton in Gegenwart von Kaliumcarbonat den (RS)-2-(4-Cyano-benzoyl)-4-[(4-ethoxycarbonylmethoxy)-phenyl]-4-oxo-buttersäure-ethylester; MS: 437 (2, M).

b) Dieser wird in Pyridin und Triethylamin mit Schwefelwasserstoff zum (RS)-4-(4-Ethoxycarbonylmethoxy-phenyl)-4-oxo-2-(4-thiocarbamoyl-benzoyl)-buttersäure-ethylester umgesetzt; MS: 472 (100, M+H).

c) Letzterer wird zunächst mit Methyljodid in Aceton, dann mit Ammoniumacetat und Essigsäure in Methanol und schliesslich mit Di-tert-butyl-dicarbonat in Methylenchlorid und wässriger Natriumcarbonatlösung zum (RS)-2-[4-(tert-Butoxycarbonylamino-imino-methyl)-benzoyl]-4-(4-ethoxycarbonylmethoxy-phenyl)-4-oxo-buttersäureethylester umgesetzt; MS: 555 (100, M+H).

Beispiel 87

In Analogie zu Beispiel 79 erhält man aus dem (RS)-2-[4-(Amino-imino-methyl)-benzoyl]-4-(4-ethoxycarbonylmethoxy-phenyl)-4-oxo-buttersäureethylester-formiat und Chlorameisensäureethylester den (RS)-2-[4-(Ethoxycarbonylamino-imino-methyl)-benzoyl]-4-(4-ethoxycarbonylmethoxy-phenyl)-4-oxo-buttersäureethylester; MS: 527 (100, M+H).

Beispiel 88

3.8 g 4-[3-(4-Thiocarbamoyl-benzoyl)-propionyl]-phenoxyessigsäureethylester, 76 ml Aceton und 5.9 ml Methyljodid werden 3 h bei 45°C gerührt. Das Reaktionsgemisch wird eingedampft, der Rückstand in 117 ml Methanol gelöst, mit 2.2 g Ammoniumacetat und 0.54 ml Essigsäure versetzt und 3.5 h bei 60°C gerührt. Das Reaktionsgemisch wird bis zum Beginn der Kristallisation im Vakuum eingeengt und kalt gestellt. Die Fällung wird abgenutscht und zur Reinigung in Acetonitril verrührt. Man erhält 2 g [4-[4-[4-(Amino-imino-methyl)-phenyl]-4-oxo-butyryl]-phenoxy]-essigsäureethylester-acetat (1:1), Smp. 212°C.

Das Ausgangsmaterial kann wie folgt erhalten werden:

a) Zu einer Suspension von 14.4 g wasserfreiem Magnesiumchlorid in 151 ml trockenem Methylenchlorid und 25.2 ml Acetessigsäure-tert-butylester werden bei 5°C 24.4 ml Pyridin zugetropft. Nach 15 Minuten gibt man 25 g 4-Cyanobenzoylchlorid dazu und rührt anschliessend 2 h bei 20°C. Zur Aufarbeitung verdünnt man mit Essigester, wäscht mit eiskalter verdünnter Salzsäure und Wasser, trocknet und dampft im Vakuum ein. Der Rückstand wird in 600 ml tert-Butyl-methylether gelöst und unter kräftigem Rühren mit 160 ml 10-proz. Ammoniaklösung versetzt. Nach 2 h trennt man die Phasen, wäscht mit Wasser, trocknet und dampft im Vakuum ein. Filtration an Kieselgel und Kristallisation aus Pentan ergibt 13.1 g reinen 3-(4-Cyanophenyl)-3-oxo-propionsäure-tert-butylester, Smp. 82-83°C.

b) Dieser wird in Aceton in Gegenwart von Kaliumcarbonat mit [4-(Bromacetyl)-phenoxy]-essigsäureethylester zum (RS)-[4-[3-tert-Butoxycarbonyl-3-(4-cyano-benzoyl)-propionyl]-phenoxy]-essigsäureethylester umgesetzt; MS: 465 (1, M).

c) Letzterer wird 4 h in Ameisensäure bei 20°C gerührt, eingedampft, mit Toluol abgedampft und 1,5 h in Toluol auf 70°C erwärmt. Dabei erhält man [4-[3-(4-Cyano-benzoyl)-propionyl]-phenoxy]-essigsäureethylester, Smp. 115-116°C.

d) Dieser wird in Pyridin und Triethylamin mit Schwefelwasserstoff zum 4-[3-(4-Thiocarbamoyl-benzoyl)-propionyl]-phenoxy-essigsäureethylester, Smp. 179-180°C, umgesetzt.

Beispiel 89

In Analogie zu Beispiel 79 erhält man aus dem [4-[4-[4-(Amino-imino-methyl)-phenyl]-4-oxo-butyryl]-phenoxy]-essigsäureethylester-acetat den [4-[4-[4-(Ethoxycarbonylamino-imino-methyl)-phenyl]-4-oxo-butyryl]-phenoxy]-essigsäure-ethylester, Smp. 167-168°C.

Beispiel 90

Durch Umsetzung von [[4-(p-Cyan-N-methylbenzamido)acetyl-o-phenylen]dioxy]diessigsäure-dimethylester (J. Med. Chem. **1992**, *35*, 4393-4407) mit Hydroxylaminhydrochlorid in Methanol in Gegenwart von Natriummethanolat erhält man nach Rühren über Nacht und Aufarbeitung [[[4-(p-Amino-hydroxyimino-methyl)-N-methylbenzamido]acetyl-o-phenylen]dioxy]diessigsäure-dimethylester in Form eines farblosen Festkörpers. Smp. 58-60°C. MS (ISP): 488 $(M+H)^+$.

Beispiel 91

Durch Umsetzung von (S)-[2-[1-[2-[4-Cyano-benzoylamino]-propionyl]-piperidin-4-yloxy]-acetylamino]-essigsäure-methylester mit Hydroxylaminhydrochlorid in Methanol in Gegenwart von Natriummethanolat erhält man nach Rühren über Nacht, Aufarbeitung und chromatographischer Reinigung an Kieselgel (Dichlormethan/Methanol 6:1) (Z)-(S)-[2-[1-[2-[4-(Amino-hydroxyimino-methyl)-benzoylamino]-propionyl]-piperidin-4-yloxy]-acetylamino]-essigsäure-methylester. Smp. 168-172 °C. MS (ISP): 464 $(M+H)^+$.

Das Ausgangsmaterial kann wie folgt hergestellt werden:

a) Behandlung von 1-Benzyloxycarbonyl-piperidin-4-yloxy-essigsäure-tert-butylester (J. Med. Chem. **1992**, *35*, 4393-4407) mit Ameisensäure gefolgt von der Umsetzung des entstandenen Produktes mit Glycin-ethylester-hydrochlorid in Gegenwart von *O*-Benzotriazol-1-yl-*N, N, N', N'*-tetramethyluronium hexafluorophosphat (HBTU) und *N*-Methylmorpholin ergibt [[1-Benzyloxycarbonyl]-piperidin-4-yloxy-acetylamino]-essigsäure-ethylester als farbloses Öl. MS (ISP): 379 $(M+H)^+$.

b) Durch katalytische Hydrierung des Produktes der Vorstufe gefolgt von Kupplung mit Z-Ala-OH in Gegenwart von HBTU und *N*-Methylmorpholin erhält man (S)-Benzyl-[2-[4-[[[[Ethoxycarbonyl]methylamino]carbonyl]-methoxy]piperidinyl]-1-oxopropyl]carbamat in Form eines schwach gelben Öls. MS (ISP): 450 $(M+H)^+$.

c) Durch katalytische Hydrierung des Produktes der Vorstufe gefolgt von Kupplung mit 4-Cyanbenzoesäurechlorid in Gegenwart von Natriumhydrogencarbonat erhält man (S)-[2-[1-[2-[4-Cyano-benzoylamino]-propionyl]-piperidin-4-yloxy]-acetylamino]-essigsäure-ethylester in Form eines farblosen Harzes. MS (ISP): 445 $(M+H)^+$.

Beispiel 92

Durch Veresterung von (Z)-(S)-1-[2-[4-(Amino-hydroxyimino-methyl)-benzoylamino]-propionyl]-piperidin-4-yloxyessigsäure (Beispiel 75) in 1-Hexanol in Gegenwart von konz. Schwefelsäure erhält man (Z)-(S)-1-[2-[4-(Amino-hydroxyimino-methyl)-benzoylamino]-propionyl]-piperidin-4-yloxyessigsäure-n-hexylester in Form von watteartigen Kristallen. Smp. 169-171 $^oC$. MS (ISP): 477 $(M+H)^+$.

Beispiel 93

Durch Umsetzung von (Z)-(S)-1-[2-[4-(Amino-hydroxyimino-methyl)-benzoylamino]-propionyl]-piperidin-4-yloxyessigsäure (Beispiel 75) mit (E/Z)-2-Brommethyl-pent-2-ensäure-isobutylester (J. Antibiotics **1992**, *45,* 1358-1364) in Gegenwart von Triethylamin erhält man (E/Z)-(S)-2-[[1-[2-[4-[(Z)-Amino-hydroxyimino-methyl)-benzoylamino]-propionyl]-piperidin-4-yloxy]-acetoxymethyl]pent-2-ensäure-isobutylester in Form farbloser Kristalle. Smp. 94-96 $^oC$. MS (ISP): 561 $(M+H)^+$.

Beispiel 94

Durch Umsetzung von (Z)-(S)-1-[2-[4-(Amino-hydroxyimino-methyl)-benzoylamino]-propionyl]-piperidin-4-yloxyessigsäure (Beispiel 75) mit Pivalinsäure-chlormethylester in Gegenwart von Triethylamin erhält man (Z)-(S)-[1-[2-[4-(Amino-hydroxyimino-methyl)-benzoylamino]-propionyl]-piperidin-4-yloxy]essigsäure-tert-butylcarbonyloxy-methylester in Form farbloser Kristalle. Smp. 126 $^oC$. MS (ISP): 507 $(M+H)^+$.

Beispiel 95

Durch Umsetzung von (Z)-(S)-1-[2-[4-(Amino-hydroxyimino-methyl)-benzoylamino]-propionyl]-piperidin-4-yloxyessigsäure (Beispiel 75) mit 1-Iodethyl-isopropyl-carbonat (J. Antibiotics **1987**, *40,*370-384) in Gegenwart von Dicyclohexylamin in Dimethylacetamid erhält man (Z)-(S)-[1-[2-[4-(Amino-hydroxyimino-methyl)-benzoylamino]-propionyl]-piperidin-4-yloxy]essigsäure-(R/S)-1-isopropoxycarbonyloxy-ethylester als farbloses Pulver. Smp. 113 $^oC$ (Zers). MS (ISP): 523 $(M+H)^+$.

Beispiel 96

Durch Umsetzung von (Z)-(S)-1-[2-[4-(Amino-hydroxyimino-methyl)-benzoylamino]-propionyl]-piperidin-4-yloxyessigsäure (Beispiel 75) mit Methanol, wie in Beispiel 75 beschrieben erhält man (Z)-(S)-[1-[2-[4-(Amino-hydroxyimino-methyl)-benzoylamino]-propionyl]-piperidin-4-yloxy]-essigsäure-methylester als farbloses Pulver. Smp. 177-178 $^oC$ (Zers). MS (ISP): 407 $(M+H)^+$.

Beispiel 97

Ausgehend von (S)-1-[2-(5-Cyan-pyridin-2-ylcarbonylamino)-propionyl]-piperidin-4-yloxy-essigsäure-tert-butylester (J. Med. Chem. **1992**, *35,* 4393-4407) erhält man auf die in Beispiel 75 beschriebene Weise (Z)-(S)-[1-[2-[5-(Amino-hydroxyimino-methyl)-pyridin-2-ylcarbonylamino]-propionyl]-piperidin-4-yloxy]essigsäure-ethylester in Form farbloser Kristalle. Smp. 148-150 $^oC$. MS (ISP): 422 $(M+H)^+$.

Beispiel 98

Ausgehend von (S)-1-[3-(4-Hydroxy-phenyl)-2-[4-cyan-benzoylamino]-propionyl]-piperidin-4-yloxy-essigsäure-tert-butylester (Beispiel 56 b) erhält man auf die in Beispiel 75 beschriebene Weise (Z)-(S)-[1-[2-[4-(Amino-hydroxyimino-methyl)-benzoylamino]-3-(4-hydroxyphenyl)-propionyl]-piperidin-4-yloxy]essigsäure-ethylester in Form farbloser Kristalle. Smp. 107-110 $^oC$. MS (ISP): 513 $(M+H)^+$.

Beispiel 99

Durch Veresterung von [[1-[$N^2$-(p-Amidinobenzoyl)-L-ornithyl]-piperidin-4-yl]oxy]essigsäure (J. Med. Chem.

**1992**, *35,* 4393-4407) mit Ethanol in Gegenwart von Salzsäure, gefolgt von der Umsetzung des entstandenen Produktes mit Chlorameisensäureethylester, wie in Beispiel 46 beschrieben, erhält man (E/Z)-(S)-[1-[2-[4-(Amino-ethoxycarbonylimino-methyl)-benzoylamino]-5-ethoxycarbonylamino-pentanoyl]-piperidin-4-yloxy]essigsäure-ethylester als farblosen Schaum. MS (ISP): 592 $(M+H)^+$.

Beispiel 100

Durch Umsetzung von (S)-[1-[2-[4-Cyan-benzoylamino]-5-benzoylamino-pentanoyl]-piperidin-4-yloxy]essigsäure-ethylester mit Hydroxylamin-hydrochlorid, wie in Beispiel 75 beschrieben, erhält man (Z)-(S)-[1-[2-[4-(Amino-hydroxyimino-methyl)-benzoylamino]-5-benzoylamino-pentanoyl]-piperidin-4-yloxy]essigsäure-ethylester als farbloses Harz. MS (ISP): 568 $(M+H)^+$.

Das Ausgangsmaterial kann wie folgt hergestellt werden:

a) Durch Umsetzung von [[1-[$N^5$-(tert-Butoxycarbonyl)-L-ornithyl]-piperidin-4-yl]oxy]essigsäure tert-butylester (J. Med. Chem. **1992**, *35,* 4393-4407) mit 4-Cyanbenzoesäurechlorid in einem Zweiphasengemisch von Dichlormethan und gesättigter Natriumhydrogencarbonatlösung erhält man nach Aufarbeitung (S)-[1-[2-[4-Cyanbenzoylamino]-5-tert-butoxycarbonylamino-pentanoyl]-piperidin-4-yloxy]essigsäure-tert-butylester. MS (ISP): 559 $(M+H)^+$.
b) Die Umsetzung des Produktes der Vorstufe mit Trifluoressigsäure in Dichlormethan ergibt (S)-[1-[2-[4-Cyanbenzoylamino]-5-amino-pentanoyl]-piperidin-4-yloxy]essigsäure als farbloses Harz. MS (ISP): 403 $(M+H)^+$.
c) Durch Veresterung des Produktes der Vorstufe mit Ethanol in Gegenwart von konz. Schwefelsäure, gefolgt von der Umsetzung mit Benzoesäurechlorid in einem Zweiphasengemisch von Dichlormethan und gesättigter Natriumhydrogencarbonatlösung erhält man (S)-[1-[2-[4-Cyanbenzoylamino]-5-benzoylamino-pentanoyl]-piperidin-4-yloxy]essigsäure-ethylester. MS (EI): 348 $(M-C_9H_{16}NO_3)^+$, 188 $(C_9H_{18}NO_3)$.

Beispiel 101

Durch Umsetzung von (S)-[1-[2-[4-Cyan-benzoylamino]-5-ethoxycarbonylamino-pentanoyl]-piperidin-4-yloxy]essigsäure-ethylester mit Hydroxylaminhydrochlorid in Gegenwart von Natriumethanolat, wie in Beispiel 75 beschrieben, erhält man (Z)-(S)-[1-[2-[4-(Amino-hydroxyimino-methyl)-benzoylamino]-5-ethoxycarbonylamino-pentanoyl]-piperidin-4-yloxy]essigsäure-ethylester als farblosen Schaum. MS (ISP): 536 $(M+H)^+$.

Das Ausgangsmaterial kann wie folgt hergestellt werden:

Durch Veresterung von (S)-[1-[2-[4-Cyanbenzoylamino]-5-amino-pentanoyl]-piperidin-4-yloxy]essigsäure (Beispiel 100) mit Ethanol in Gegenwart von konz. Schwefelsäure, gefolgt von der Umsetzung mit Chlorameisensäure-ethylester in einem Zweiphasengemisch von Dichlormethan und 1N Natriumhydroxidlösung erhält man (S)-[1-[2-[4-Cyan-benzoylamino]-5-ethoxycarbonylamino-pentanoyl]-piperidin-4-yloxy]essigsäure-ethylester. MS (ISP): 503 $(M+H)^+$.

Beispiel 102

Durch Umsetzung von (S)-[1-[2-[4-Cyan-benzoylamino]-5-(3-butyl-ureido)-pentanoyl]-piperidin-4-yloxy]essigsäure-ethylester mit Hydroxylaminhydrochlorid in Gegenwart von Natriumethanolat, wie in Beispiel 75 beschrieben, erhält man (Z)-(S)-[1-[2-[4-(Amino-hydroxyimino-methyl)-benzoylamino]-5-(3-butyl-ureido)-pentanoyl]-piperidin-4-yloxy]essigsäure-ethylester als farblosen Schaum. MS (ISP): 563 $(M+H)^+$.

Das Ausgangsmaterial kann wie folgt hergestellt werden:

Durch Veresterung von (S)-[1-[2-[4-Cyanbenzoylamino]-5-amino-pentanoyl]-piperidin-4-yloxy]essigsäure (Beispiel 100) mit Ethanol in Gegenwart von konz. Schwefelsäure, gefolgt von der Umsetzung mit n-Butylisocyanat in einem Zweiphasengemisch von Dichlormethan und gesättigter Natriumhydrogencarbonatlösung erhält man (S)-[1-[2-[4-Cyanbenzoylamino]-5-(3-butyl-ureido)-pentanoyl]-piperidin-4-yloxy]essigsäure-ethylester. MS (ISP): 530 $(M+H)^+$.

Referenzbeispiel 103

Ausgehend von 4-Piperidinyloxy-essigsäure-tert-butylester und (S)-2-Benzyloxycarbonyl-amino-buttersäure erhält man auf die in J. Med. Chem. **1992**, *35,* 4393-4407 beschriebene Weise das Ameisensäuresalz von (S)-[1-[2-[4-(Amino-imino-methyl)-benzoylamino]-butyryl]-piperidin-4-yloxy]essigsäure in Form farbloser Kristalle. Smp. 240 $^oC$ (Zers.). MS (ISP): 391 $(M+H)^+$.

Referenzbeispiel 104

Durch Veresterung von (S)-[1-[2-[4-(Amino-imino-methyl)-benzoylamino]-butyryl]-piperidin-4-yloxy]essigsäure ( Beispiel 103) in Ethanol in Gegenwart von konz. Schwefelsäure erhält man das Hemisulfat von (S)-[1-[2-[4-(Amino-imino-methyl)-benzoylamino]-butyryl]-piperidin-4-yloxy]essigsäure-ethylester in Form farbloser Kristalle. Smp. 256-260 $^{o}$C (Zers.). MS (ISP): 419 $(M+H)^+$.

Beispiel 105

Durch Umsetzung von (S)-[1-[2-[4-(Amino-imino-methyl)-benzoylamino]-butyryl]-piperidin-4-yloxy]essigsäure-ethylester ( Beispiel 104) mit Chlorameisensäure-n-butylester in einem Zweiphasengemisch von Dichlormethan und gesättigter Natriumhydrogencarbonatlösung erhält man (E/Z)-(S)-[1-[2-[4-(Amino-n-butoxycarbonylimino-methyl)-benzoylamino]-butyryl]-piperidin-4-yloxy]essigsäure-ethylester in Form eines farblosen Schaumes. MS (ISP): 519 $(M+H)^+$.

Beispiel 106

Durch Veresterung von (Z)-(S)-[1-[2-[4-(Amino-hydroxyimino-methyl)-benzoylamino]-butyryl]-piperidin-4-yloxy]essigsäure in Ethanol in Gegenwart von konz. Schwefelsäure erhält man (Z)-(S)-[1-[2-[4-(Amino-hydroxyimino-methyl)-benzoylamino]-butyryl]-piperidin-4-yloxy]essigsäure-ethylester in Form eines farblosen Schaumes. MS (ISP): 435 $(M+H)^+$.

Das Ausgangsmaterial kann wie folgt hergestellt werden:

a) Die Umsetzung von (S)-[1-[2-Amino-butanoyl]-piperidin-4-yloxy]essigsäure-t-butylester (analog hergestellt wie in J. Med. Chem. **1992**, *35*, 4393-4407 beschrieben) mit 4-Cyanbenzoesäurechlorid ergibt (S)-[1-[2-[4-Cyanbenzoylamino]-butyryl]-piperidin-4-yloxy]essigsäure-tert-butylylester. MS (EI): 429 $(M^+)$.
b) Die Umsetzung des Produktes der Vorstufe mit Hydroxylaminhydrochlorid ergibt (Z)-(S)-[1-[2-[4-(Amino-hydroxyimino-methyl)-benzoylamino]-butyryl]-piperidin-4-yloxy]essigsäure-tert-butylylester. MS (ISP): 463 $(M+H)^+$.
c) Die Behandlung des Produktes der Vorstufe mit Ameisensäure ergibt (Z)-(S)-[1-[2-[4-(Amino-hydroxyimino-methyl)-benzoylamino]-butyryl]-piperidin-4-yloxy]essigsäure in Form eines farblosen Schaumes. MS (ISP): 407 $(M+H)^+$.

Referenzbeispiel 107

Ausgehend von 4-Piperidinyloxy-essigsäure-tert-butylester und Z-Me-Ala-OH erhält man auf die in J. Med. Chem. **1992**, *35,* 4393-4407 beschriebene Weise (S)-[1-[2-[[4-(Amino-imino-methyl)-benzoyl]-methylamino]-propionyl]-piperidin-4-yloxy]essigsäure als farbloses Harz. MS (ISP): 391 $(M+H)^+$.

Referenzbeispiel 108

Aus 1-tert-Butoxycarbonyl-piperidin-4-yloxy-essigsäure (Beispiel 73) und 1-[[L-Alanyl]-4-piperidinyloxy]essigsäure-tert-butylester (Beispiel 66) erhält man auf die in Beispiel 73 beschriebene Weise (S)-[1-[2-(Piperidin-4-yloxy-acetamido)-propionyl]-piperidin-4-yloxy]essigsäure in Form eines farblosen Schaumes. MS (ISN): 370 $(M-H)^+$.

Beispiel 109

Durch Umsetzung von (S)-3-[1-[2-[4-Cyan-benzoyl-amino]-propionyl]-piperidin-4-yl]propionsäure-ethylester mit Hydroxylaminhydrochlorid, wie in Beispiel 75 beschrieben, erhält man (Z)-(S)-3-[1-[2-[4-(Amino-hydroxy-imino-methyl)-benzoyl-amino]-propionyl]-piperidin-4-yl]propionsäure-ethylester in Form farbloser Kristalle. Smp. 194-195 $^{o}$C. $[\alpha]_D^{20}$ = + 75.6$^{o}$ (c=1, Essigsäure). MS (ISP): 419 $(M+H)^+$.

Das Ausgangsmaterial kann wie folgt hergestellt werden:

a) Die Veresterung von 3-[Piperidin-4-yl]-propionsäure (J. Org. Chem. **1945**, *10*, 562) mit Ethanol in Gegenwart von konz. Schwefelsäure gefolgt von der Kupplung mit Z-Ala-OH in Gegenwart von 1-Ethyloxycarbonyl-2-isobutyloxy-1,2-dihydrochinolin (EEDQ) in Dichlormethan ergibt 3-[1-(2-Benzyloxycarbonylamino-propionyl)-piperidin-4-yl]-propionsäure-ethylester als farbloses Öl. MS (ISP): 391 $(M+H)^+$.
b) Durch katalytische Hydrierung des Produktes der Vorstufe in Ethanol in Gegenwart von 10% Pd/C gefolgt von der Umsetzung mit 4-Cyanbenzoylchlorid erhält man (S)-3-[1-[2-[4-Cyan-benzoyl-amino]-propionyl]-piperidin-4-yl]propionsäure-ethylester in Form farbloser Kristalle. Smp. 108-109 $^{o}$C. MS (EI): 385 $(M)^{+}$.

Beispiel 110

Durch katalytische Hydrierung von (Z)-(S)-[[Acetyl-1-[2-[4-(amino-hydroxyimino-methyl)-benzoyl-amino]-propionyl]-piperidin-4-yl]-amino]essigsäure in Wasser/Essigsäure 9:1 in Gegenwart von 10% Pd/C erhält man (S)-[[Acetyl-1-[2-[4-(amino-imino-methyl)-benzoyl-amino]-propionyl]-piperidin-4-yl]-amino]essigsäure als farbloses Pulver. Smp. über 230 $^{o}$C (Zers.). MS (ISP): 418 $(M+H)^{+}$.

Das Ausgangsmaterial kann wie folgt hergestellt werden:

a) Die Umsetzung von N-Benzyloxycarbonyl-4-piperidon mit dem Hydrochlorid von Glycin-ethylester in Gegenwart von Natriumborhydrid gefolgt von Acylierung des Produktes mit Essigsäureanhydrid ergibt Acetyl-[1-[benzyloxycarbonyl]-piperidin-4-yl]-amino-essigsäure-ethylester als farbloses Öl.

b) Durch katalytische Hydrierung des Produktes der Vorstufe in Gegenwart von 10% Pd/C in Ethanol gefolgt von der Kupplung mit Z-Ala-OH in Gegenwart von 1-Ethoxycarbonyl-2-ethoxy-1,2-dihydrochinolin (EEDQ) erhält man (S)-[[[Acetyl-1-[2-benzyloxycarbonyl-amino]-propionyl]-piperidin-4-yl]-amino]essigsäure-ethylester als farbloses Öl. MS (ISP): 456 $(M+Na)^{+}$.

c) Durch katalytische Hydrierung des Produktes der Vorstufe über 10% Pd/C in Ethanol in Gegenwart von Essigsäure gefolgt von der Umsetzung mit 4-Cyanbenzoylchlorid in einem Zweiphasengemisch von Dichlormethan und gesättigter Natriumhydrogencarbonatlösung erhält man (S)-[[Acetyl-1-[2-[4-cyan-benzoyl-amino]-propionyl]-piperidin-4-yl]-amino]essigsäure-ethylester als farblosen Schaum. MS (ISP): 428 $(M+H)^{+}$.

d) Durch Umsetzung des Produktes der Vorstufe mit Hydroxylaminhydrochlorid gefolgt von der Verseifung mit Natronlauge erhält man (Z)-(S)-[[Acetyl-1-[2-[4-(amino-hydroxyimino-methyl)-benzoyl-amino]-propionyl]-piperidin-4-yl]-amino]essigsäure in Form farbloser Kristalle. Smp. über 270 $^{o}$C (Zers.). MS (ISP): 434 $(M+H)^{+}$.

Beispiel 111

Auf analoge Weise, wie in Beispiel 110 beschrieben, erhält man unter Verwendung von Di-tert-butyl-dicarbonat anstelle von Essigsäureanhydrid (Z)-(R/S)-[[tert-Butoxycarbonyl-1-[2-[4-(amino-hydroxyimino-methyl)-benzoyl-amino]-propionyl]-piperidin-4-yl]-amino]essigsäure-ethylester als farblosen Schaum. MS (ISP): 520 $(M+H)^{+}$.

Beispiel 112

Durch Veresterung von (Z)-[1-[2-[4-[Amino-hydroxyimino-methyl]-phenylcarbamoyl]-acetyl]-piperidin-4-yloxy]-essigsäure mit Ethanol erhält man (Z)-[1-[2-[4-[Amino-hydroxyimino-methyl]-phenylcarbamoyl]-acetyl]-piperidin-4-yloxy]-essigsäure-ethylester in Form farbloser Kristalle. Smp. 180 $^{o}$C (Zers.). MS (ISP): 407 $(M+H)^{+}$.

Das Ausgangsmaterial kann wie folgt hergestellt werden:

a) Die Umsetzung von 4-Amino-benzonitril mit Meldrumsäure ergibt 2-[4-Cyan-phenylcarbamoyl]-essigsäure.

b) Durch Kupplung des Produktes der Vorstufe mit 4-Piperidinyloxy-essigsäure-tert-butylester (J. Med. Chem. **1992**, *35,* 4393-4407) in Gegenwart von *O*-Benzotriazol-1-yl-*N, N, N', N"*-tetramethyluronium-hexafluorophosphat (HBTU) und *N*-Methylmorpholin erhält man [1-[2-[4-Cyan-phenylcarbamoyl]-acetyl]-piperidin-4-yloxy]-essigsäure-tert-butylester. MS (EI): 401 $(M)^{+}$.

c) Die Umsetzung des Produktes der Vorstufe mit Hydroxylaminhydrochlorid, wie in Beispiel 75 beschrieben, ergibt (Z)-[1-[2-[4-[Amino-hydroxyimino-methyl]-phenylcarbamoyl]-acetyl]-piperidin-4-yloxy]-essigsäure-tert-butylester. Smp. 161-163 $^{o}$C. MS (ISP): 435 $(M+H)^{+}$.

d) Durch Behandlung des Produktes der Vorstufe mit Ameisensäure erhält man (Z)-[1-[2-[4-[Amino-hydroxyimino-methyl]-phenylcarbamoyl]-acetyl]-piperidin-4-yloxy]-essigsäure in Form farbloser Kristalle. Smp. 133-135 $^{o}$C. MS (ISN): 377 $(M-H)^{+}$.

Beispiel 113

Durch katalytische Hydrierung von (Z)-[1-[2-[4-[Amino-hydroxyimino-methyl]-phenylcarbamoyl]-acetyl]-piperidin-4-yloxy]-essigsäure-ethylester (Beispiel 112) in Gegenwart von 10% Pd/C in Ethanol/Essigsäure 15:1 erhält man das Acetatsalz von [1-[2-[4-[Amino-imino-methyl]-phenylcarbamoyl]-acetyl]-piperidin-4-yloxy]-essigsäure-ethylester in Form farbloser Kristalle. Smp. 191-192 $^{o}$C. MS (ISP): 391 $(M+H)^{+}$.

Beispiel 114

Durch Umsetzung von [1-[2-[4-[Amino-imino-methyl]-phenylcarbamoyl]-acetyl]-piperidin-4-yloxy]-essigsäure-ethylester (Beispiel 113) mit Chlorameisensäureethylester in Gegenwart von 0.2 N Natronlauge erhält man (E/Z)-[1-[2-[4-[Amino-ethoxycarbonylimino-methyl]-phenylcarbamoyl]-acetyl]-piperidin-4-yloxy]-essigsäure-ethylester als farbloses Pulver. Smp. 96-102 $^oC$. MS (ISP): 463 $(M+H)^+$.

Beispiel 115

Durch Veresterung von (Z)-(S)-[1-[2-[4-[Amino-hydroxyimino-methyl]-benzyloxy]-propionyl]-piperidin-4-yloxy]-essigsäure mit Ethanol erhält man (Z)-(S)-[1-[2-[4-[Amino-hydroxyimino-methyl]-benzyloxy]-propionyl]-piperidin-4-yloxy]-essigsäure-ethylester in Form farbloser Kristalle. $[\alpha]_D^{20}$ = -27.3$^o$ (c=1, Ethanol). Smp. 137-138 $^oC$. MS (ISP): 408 $(M+H)^+$.

Das Ausgangsmaterial kann wie folgt hergestellt werden:

a) Die Umsetzung von L-(-)-Milchsäure-ethylester mit 4-Brommethyl-benzonitril in DMF in Gegenwart von Silberoxid gefolgt von der Verseifung des Produktes mit 1 N Natronlauge ergibt (S)-2-(4-Cyan-benzyloxy)-propionsäure als schwach gelbes Öl. MS (EI): 160 $(M-COOH)^+$.

b) Durch analoge Umsetzung des Produktes der Vorstufe, wie in Beispiel 112b), c) und d) beschrieben, erhält man (Z)-(S)-[1-[2-[4-[Amino-hydroxyimino-methyl]-benzyloxy]-propionyl]-piperidin-4-yloxy]-essigsäure. MS (ISP): 380 $(M+H)^+$.

Beispiel 116

170 mg Hydroxylaminhydrochlorid, 10.8 ml DMSO und 0.71 ml Triethylamin werden 10 Min. bei 20 °C gerührt, mit 578 mg [1-[4-(4-Cyano-phenyl)-4-oxo-butyryl]-piperidin-4-yloxy]-essigsäure-ethylester versetzt und 20 Std. bei 20 °C gerührt. Das Reaktionsgemisch wird mit Essigester verdünnt und die Lösung mit Wasser und gesättigter Kochsalzlösung gewaschen, getrocknet und im Vakuum eingedampft. Chromatographie des Rückstandes an Kieselgel mit Methylenchlorid-Alkohol 96:4 gibt 343 mg (E)/(Z)-[1-[4-[4-(Amino-hydroxyimino-methyl)-phenyl]-4-oxo-butyryl]-piperidin-4-yloxy]-essigsäure-ethylester, Smp. 166-167 °C.

Das Ausgangsmaterial kann wie folgt erhalten werden:

a. [1-[4-(4-Cyano-phenyl)-4-oxo-butyryl]-piperidin-4-yloxy]-essigsäure-tert.-butylester (Beispiel 77a) wird in Ameisensäure bei 50 °C zur [1-[4-(4-Cyano-phenyl)-4-oxo-butyryl]-piperidin-4-yloxy]-essigsäure, Smp. 160-165 °C, gespalten.

b. In 5N ethanolischer Salzsäure erhält man daraus den [1-[4-(4-Cyano-phenyl)-4-oxo-butyryl]-piperidin-4-yloxy]-essigsäure-ethylester, Smp. 79-83°C.

Beispiel 117

In Analogie zu Beispiel 79 erhält man aus dem (RS)-[1-[4-[4-(Amino-imino-methyl)-phenyl]-2-methyl-4-oxo-butyryl]-piperidin-4-yloxy]-essigsäure-isopropylester-hydrochlorid und Di-tert.-butyl-dicarbonat den (RS)-[1-[4-[4-(tert.-Butoxycarbonylamino-imino-methyl)-phenyl]-2-methyl-4-oxo-butyryl]-piperidin-4-yloxy]-essigsäure-isopropylester als harzigen Schaum, MS: 518 (100, M+H).

Beispiel 118

279 mg (E)/(Z)-[1-[4-[4-(Amino-hydroxyimino-methyl)-phenyl]-4-oxo-butyryl]-piperidin-4-yloxy]-essigsäure-ethylester, 2.8 ml DMSO, 140 mg Hydroxylamin-hydrochlorid und 0.28 ml Triethylamin werden zwei Tage bei 20 °C gerührt. Die Lösung wird mit Essigester verdünnt, mit Wasser und Kochsalzlösung gewaschen, getrocknet und im Vakuum eingedampft. Chromatographie des Rückstands an Kieselgel mit Hexan-Aceton 1:2 gibt 207 mg [1-[4-[4-(E)/(Z)-(Amino-hydroxyimino-methyl)-phenyl]-4-(E)/(Z)-hydroxyimino-butyryl]-piperidin-4-yloxy]-essigsäure-ethylester als harzigen Schaum, MS: 421 (100, M+H).

Beispiel 119

190 mg (E)/(Z)-[1-[4-[4-(Amino-hydroxyimino-methyl)-phenyl]-4-oxo-butyryl]-piperidin-4-yloxy]-essigsäure-ethylester, 3.8 ml Methylenchlorid und 0.05 ml Acetanhydrid werden vier Stunden bei 20 °C gerührt. Nach Zugabe von

0.25 ml Ethanol wird die Lösung 20 Min. weitergerührt und dann eingedampft. Aus Ethanol werden 170 mg (E)/(Z)-[1-[4-[4-(Acetoxyimino-amino-methyl)-phenyl]-4-oxo-butyryl]-piperidin-4-yloxy]-essigsäure-ethylester vom Smp. 134-135 °C erhalten.

Beispiel 120

180 mg (E)/(Z)-[1-[4-[4-(Amino-hydroxyimino-methyl)-phenyl]-4-oxo-butyryl]-piperidin-4-yloxy]-essigsäure-ethylester, 3.52 ml Aceton und 0.88 ml Ameisensäure werden 24 Stunden bei 60 °C gerührt. Das Reaktionsgemisch wird zur Trockene eingedampft und der Rückstand mit Methylenchlorid-Ethanol 97:3 an Kieselgel chromatographiert. Es werden 152 mg [1-[4-[4-(5,5-Dimethyl-4,5-dihydro-1,2,4-oxadiazol-3-yl)-phenyl]-4-oxo-butyryl]-piperidin-4-yloxy]-essigsäure-ethylester als Schaum erhalten. MS: 445 (50, M+H).

Beispiel 121

In Analogie zu Beispiel 116 erhält man aus dem (R)-[1-[4-(4-Cyano-phenyl)-2-methyl-4-oxo-butyryl]-piperidin-4-yloxy]-essigsäure-ethylester den (R)-(E)/(Z)-[1-[4-[4-(Amino-hydroxyimino-methyl)-phenyl]-2-methyl-4-oxo-butyryl]-piperidin-4-yloxy]-essigsäure-ethylester, Smp. 172-173 °C, $[\alpha]_D^{20}$ = +83.2° (MeOH, c = 0.5 %).

Das Ausgangsmaterial kann wie folgt erhalten werden:

a. Durch Spaltung von 4-Piperidinyloxy-essigsäure-tert.-butylester in Ameisensäure erhält man 4-Piperidinyloxy-essigsäure, Smp. > 270 °C.

b. Diese wird in 5N ethanolischer HCl ins Hydrochlorid des 4-Piperidinyloxy-essigsäure-ethylesters, Smp. 104-106°C, übergeführt.

c. Aus 3-(4-Cyano-phenyl)-3-oxo-propionsäure-tert.-butylester und (R)-2-Trifluoromethylsulfonyloxy-propionsäure-isobutylester erhält man in Tetrahydrofuran in Gegenwart von Natrium-bis-(trimethylsilyl)-amid den 2-(R)-3(R,S)-3-tert.-Butoxycarbonyl-4-(4-cyano-phenyl)-2-methyl-4-oxo-buttersäure-isobutylester.

d. Durch Erwärmen auf 45 °C in Ameisensäure erhält man daraus den (R)-4-(4-Cyano-phenyl)-2-methyl-4-oxo-buttersäure-isobutylester, $[\alpha]_D^{20}$ = +21° (Methanol, c = 0.5 %).

e. Dieser wird in wässerigem THF mit Lithiumhydroxyd zur (R)-4-(4-Cyano-phenyl)-2-methyl-4-oxo-buttersäure verseift. Smp. 125-126 °C, $[\alpha]_D^{20}$ = +37.4° (Methanol, c = 0.5 %).

f. Durch Kopplung der (R)-4-(4-Cyano-phenyl)-2-methyl-4-oxo-buttersäure mit dem Hydrochlorid des 4-Piperidinyloxy-essigsäure-ethylesters erhält man den (R)-[1-[4-(4-Cyano-phenyl)-2-methyl-4-oxo-butyryl]-piperidin-4-yloxy]-essigsäure-ethylester, Smp. 97-98 °C, $[\alpha]_D^{20}$ = + 91.8 °, (Methanol, c = 0.5 %).

Beispiel 122

In Analogie zu Beispiel 116 erhält man aus dem [1-[4-(4-Cyano-phenyl)-4-oxo-butyryl]-piperidin-4-yloxy]-essigsäure-tert.-butyl-ester den (E)/(Z)-[1-[4-[4-(Amino-hydroxyimino-methyl)-phenyl]-4-oxo-butyryl]-piperidin-4-yloxy]-essigsäure-tert.-butyl-ester, Smp. 188 °C.

Beispiel 123

434 mg (R)-[1-[4-[4-(tert.-Butoxycarbonylamino-imino-methyl)-phenyl]-2-methyl-4-oxo-butyryl]-piperidin-4-yloxy]-essigsäure-tert.-butylester werden in 4.4 ml Methylenchlorid und 4.4 ml Trifluoressigsäure eine Stunde bei 20°C gerührt. Das Lösungmittel wird eingedampft, der Rückstand in Wasser gelöst und die Lösung wieder eingedampft. Aus Acetonitril kristallisieren 332 mg (R)-[1-[4-[4-(Amino-imino-methyl)-phenyl]-2-methyl-4-oxo-butyryl]-piperidin-4-yloxy]-essigsäure-trifluoracetat (1:1), Smp. 212 °C, $[\alpha]_D^{20}$ = + 69.0 °, (Methanol, c = 0.5 %).

Das Ausgangsmaterial kann wie folgt hergestellt werden:

a. Durch Kopplung der (R)-4-(4-cyano-phenyl)-2-methyl-4-oxo-buttersäure mit Piperidin-4-yloxy-essigsäure-tert.-butyl-ester erhält man den (R)-[1-[4-(4-Cyano-phenyl)-2-methyl-4-oxo-butyryl]-piperidin-4-yloxy]-essigsäure-tert.-butylester, Smp. 142 °C, $[\alpha]_D^{20}$ = + 83.2 °, (Methanol, c = 0.5 %).

b. Mit Schwefelwasserstoff in Pyridin/Triethylamin erhält man daraus den (R)-[1-[2-Methyl-4-oxo-4-(4-thiocarbamoyl-phenyl)-butyryl]-piperidin-4-yloxy]-essigsäure-tert.-butylester als gelbes Harz, MS: 449 (63, M+H), $[\alpha]_D^{20}$ = + 76.4 °, (Methanol, c = 0.5 %).

c. Dieser wird zunächst mit Methyljodid in Aceton, dann mit Ammoniumacetat und Essigsäure in Methanol und schliesslich mit Di-tert.-butyl-dicarbonat in Methylenchlorid und wässeriger Natriumcarbonatlösung zum Ausgangsmaterial umgesetzt; MS: 532 (100, M+H), $[\alpha]_D^{20}$ = + 64.4 °, (Methanol, c = 0.5 %).

Beispiel 124

In Analogie zu Beispiel 116 erhält man aus dem (RS) [1-[4-(4-Cyano-phenyl)-3-methyl-4-oxo-butyryl]-piperidin-4-yloxy]-essigsäure-ethylester den (E)/(Z)-(RS)-[1-[4-[4-(Amino-hydroxyimino-methyl)-phenyl]-3-methyl-4-oxo-butyryl]-piperidin-4-yloxy]-essigsäure-ethylester als Schaum, MS: 420 (100, M+H).

Das Ausgansmaterial kann wie folgt erhalten werden:

a. (RS)-4-(4-Amino-phenyl)-3-methyl-4-oxo-buttersäure wird nach Sandmeyer zur (RS)-4-(4-Cyano-phenyl)-3-methyl-4-oxo-buttersäure umgesetzt, Smp 110-113 °C.

b. Diese wird mit dem Hydrochlorid des 4-Piperidinyloxy-essigsäure-ethyl-esters zum (RS) [1-[4-(4-Cyano-phenyl)-3-methyl-4-oxo-butyryl]-piperidin-4-yloxy]-essigsäure-ethylester gekoppelt; MS: 387 (100, M+H).

Beispiel 125

In Analogie zu Beispiel 116 erhält man aus dem (R)-[1-[4-(4-Cyano-phenyl)-2-methyl-4-oxo-butyryl]-piperidin-4-yloxy]-essigsäure-pyridin-3-ylmethyl-ester den (R)-(E)/(Z)-[1-[4-[4-(Amino-hydroxyimino-methyl)-phenyl]-2-methyl-4-oxo-butyryl]-piperidin-4-yloxy]-essigsäure-pyridin-3-ylmethyl-ester als Harz, MS: 483 (100, M+H), $[\alpha]_D^{20}$ = + 69.8 °, (Methanol, c = 0.5%).

Das Ausgangsmaterial kann auf folgende Weise erhalten werden:

a. (R)-[1-[4-(4-Cyano-phenyl)-2-methyl-4-oxo-butyryl]-piperidin-4-yloxy]-essigsäure-tert.-butylester wird in Ameisensäure zu der (R)-[1-[4-(4-Cyano-phenyl)-2-methyl-4-oxo-butyryl]-piperidin-4-yloxy]-essigsäure gespalten, Smp. 185-186 °C, $[\alpha]_D^{20}$ = + 91.6 °, (Methanol, c = 0.5 %).

b. Diese wird in Pyridin in Gegenwart von DCC und pTSOH mit 3-Pyridyl-methanol zum (R)-[1-[4-(4-Cyano-phenyl)-2-methyl-4-oxo-butyryl]-piperidin-4-yloxy]-essigsäure-pyridin-3-ylmethyl-ester verestert, Smp. 123-124 °C, $[\alpha]_D^{20}$ = + 77.6 °, (Methanol, c = 0.5 %).

Beispiel 126

In Analogie zu Beispiel 78 erhält man aus dem (RS)-[1-[4-[4-(tert.-Butoxycarbonylamino-imino-methyl)-phenyl]-3-methyl-4-oxo-butyryl]-piperidin-4-yloxy]-essigsäure-ethylester den (RS)-[1-[4-[4-(Amino-imino-methyl)-phenyl]-3-methyl-4-oxo-butyryl]-piperidin-4-yloxy]-essigsäure-ethylester als Trifluoracetat (1:1.9), MS: 404 (100, M+H).

Das Ausgangsmaterial kann wie folgt erhalten werden:

a. (RS) [1-[4-(4-Cyano-phenyl)-3-methyl-4-oxo-butyryl]-piperidin-4-yloxy]-essigsäure-ethylester wird in Pyridin/Triethylamin mit Schwefelwasserstoff in den (RS)-[1-[3-Methyl-4-oxo-4-(4-thiocarbamoyl-phenyl)-butyryl]-piperidin-4-yloxy]-essigsäure-ethylester übergeführt. MS: 386 (0.5, M-$H_2S$).

b. Dieser wird zunächst mit Methyliodid in Aceton, dann mit Ammoniumacetat/Essigsäure in Methanol und schliesslich mit Di-tert.-butyl-dicarbonat in Methylenchlorid/Wasser/Natriumcarbonat zum (RS)-[1-[4-[4-(tert.-Butoxycarbonylamino-imino-methyl)-phenyl]-3-methyl-4-oxo-butyryl]-piperidin-4-yloxy]-essigsäure-ethylester umgesetzt, MS: 504 (100, M+H).

Beispiel 127

200 mg (RS)-[1-[4-[4-(Amino-imino-methyl)-phenyl]-3-methyl-4-oxo-butyryl]-piperidin-4-yloxy]-essigsäure-ethylester-trifluoracetat werden 4 Stunden in 20 ml 25-proz. Salzsäure gerührt. Das Reaktionsgemisch wird im Vakuum zur Trockene eingedampft, der Rückstand in Wasser aufgenommen und wieder zur Trockene eingedampft. Man erhält 144 mg (RS)-[1-[4-[4-(Amino-imino-methyl)-phenyl]-3-methyl-4-oxo-butyryl]-piperidin-4-yloxy]-essigsäure-hydrat (2:3)-hydrochlorid (8:9), Smp. 173-175°C.

Beispiel 128

In Analogie zu Beispiel 79 erhält man aus dem (RS)-[1-[4-[4-(Amino-imino-methyl)-phenyl]-3-methyl-4-oxo-butyryl]-piperidin-4-yloxy]-essigsäure-ethylester-trifluoracetat mit Chlorameisensäure-isobutylester den (RS)-[1-[4-[4-(Imino-isobutoxycarbonylamino-methyl)-phenyl]-3-methyl-4-oxo-butyryl]-piperidin-4-yloxy]-essigsäure-ethylester als Schaum, MS: 504 (100, M+H).

Beispiel 129

In Analogie zu Beispiel 79 erhält man aus dem (R)-[1-[4-[4-(Amino-imino-methyl)-phenyl]-2-methyl-4-oxo-butyryl]-piperidin-4-yloxy]-essigsäure-ethylester-acetat/jodid und Chlorameisensäureethylester den (R)-[1-[4-[4-(Ethoxycarbonylamino-imino-methyl)-phenyl]-2-methyl-4-oxo-butyryl]-piperidin-4-yloxy]-essigsäure-ethylester als Harz; MS: 476 (36, M+H), $[\alpha]_D^{20}$ = + 72.6°, (Methanol, c = 0.5 %).

Das Ausgangsmaterial kann wie folgt erhalten werden:

a. (R)-[1-[4-(4-Cyano-phenyl)-2-methyl-4-oxo-butyryl]-piperidin-4-yloxy]-essigsäure-ethylester wird mit Schwefelwasserstoff in Pyridin und Triethylamin zum (R)-[1-[2-Methyl-4-oxo-4-(4-thiocarbamoyl-phenyl)-butyryl]-piperidin-4-yloxy]-essigsäure-ethylester, Smp. 171-172 °C, $[\alpha]_D^{20}$ = + 88.4° (Methanol, c = 0.5 %) umgesetzt.
b. Durch Umsetzen mit Methyliodid in Aceton und anschliessende Reaktion mit Ammoniumacetat und Essigsäure in Methanol erhält man daraus das (R)-[1-[4-[4-(Amino-imino-methyl)-phenyl]-2-methyl-4-oxo-butyryl]-piperidin-4-yloxy]-essigsäure-ethylester-acetat/iodid.

Beispiel 130

Ebenso wie im Beispiel 129 wird der (R)-[1-[4-[4-(Imino-isobutoxycarbonylamino-methyl)-phenyl]-2-methyl-4-oxo-butyryl]-piperidin-4-yloxy]-essigsäure-ethylester hergestellt. MS: 504 (100, M+H), $[\alpha]_D^{20}$ = + 70.25° (Methanol, c = 0.4 %).

Beispiel 131

In Analogie zu Beispiel 116 erhält man aus dem (R)-[1-[4-(4-Cyano-phenyl)-2-methyl-4-oxo-butyryl]-piperidin-4-yloxy]-essigsäure-pyridin-4-ylmethyl-ester den (R)-(E)/(Z)-[1-[4-[4-(Amino-hydroxyimino-methyl)-phenyl]-2-methyl-4-oxo-butyryl]-piperidin-4-yloxy]-essigsäure-pyridin-4-ylmethyl-ester als Harz, MS: 483 (100, M+H), $[\alpha]_D^{20}$ = + 71.8°, (Methanol, c = 0.5%).

Das Ausgangsmaterial erhält man aus der (R)-[1-[4-(4-Cyano-phenyl)-2-methyl-4-oxo-butyryl]-piperidin-4-yloxy]-essigsäure und 4-Pyridylmethanol in Pyridin in Gegenwart von DCC und pTSOH. Smp. 99 -104 °C, $[\alpha]_D^{20}$ = +69.8°, (Methanol, c = 0.5 %).

Beispiel 132

In Analogie zu Beispiel 116 erhält man aus dem (RS)-3-[1-[(R)-4-(4-Cyano-phenyl)-2-methyl-4-oxo-butyryl]-piperidin-4-yloxyacetoxymethyl]-piperidin-1-carbonsäure-tert-butylester den (E)- oder (Z)-(RS)-3-[1-[(R)-4-[4-(Amino-hydroxyimino-methyl)-phenyl]-2-methyl-4-oxo-butyryl]-piperidin-4-yloxyacetoxymethyl]-piperidin-1-carbonsäure-tert-butylester; MS: 589 (100, M+H), $[\alpha]_D^{20}$ = + 60°, (Methanol, c = 0.5 %).

Das Ausgangsmaterial erhält man aus der (R)-[1-[4-(4-Cyano-phenyl)-2-methyl-4-oxo-butyryl]-piperidin-4-yloxy]-essigsäure und (RS)-3-(Hydroxy-methyl)-piperidin-1-carbonsäure-tert-butylester in Pyridin in Gegenwart von DCC und pTSOH. MS: 556 (100, M+H), $[\alpha]_D^{20}$ = + 56°, (Methanol, c = 0.5%).

Beispiel 133

Ebenso wie im Beispiel 129 beschrieben wird der (R)-[1-[4-[4-(Imino-isopropoxycarbonylamino-methyl)-phenyl]-2-methyl-4-oxo-butyryl]-piperidin-4-yloxy]-essigsäure-ethylester hergestellt. MS: 490 (100, M+H), $[\alpha]_D^{20}$ = +72° (Methanol, c = 0.4 %).

Beispiel 134

In Analogie zu Beispiel 78 erhält man aus dem (R)-[1-[4-[4-(tert-Butoxycarbonylamino-imino-methyl)-phenyl]-2-methyl-4-oxo-butyryl]-piperidin-4-yloxy]-essigsäure-ethylester das (R)-[1-[4-[4-(Amino-imino-methyl)-phenyl]-2-methyl-4-oxo-butyryl]-piperidin-4-yloxy]-essigsäure-ethylester-trifluoracetat, Smp. 186 °C, $[\alpha]_D^{20}$ = + 67.4° (Methanol, c = 0.5%).

Das Ausgangsmaterial erhält man aus dem (R)-[1-[4-[4-(Amino-imino-methyl)-phenyl]-2-methyl-4-oxo-butyryl]-piperidin-4-yloxy]-essigsäure-ethylester-acetat/iodid und Di-tert-butyl-dicarbonat in Methylenchlorid/Wasser/$Na_2CO_3$; MS: 504 (91, M+H), $[\alpha]_D^{20}$ = + 66° (Methanol, c = 0.5 %).

Beispiel 135

259 mg (R)-[1-[4-[4-(Amino-imino-methyl)-phenyl]-2-methyl-4-oxo-butyryl]-piperidin-4-yloxy]-essigsäure-ethylester-trifluoracetat, 3.9 ml DMF, 0.14 ml Triaethylamin und 159 mg Benzoesäure-4-nitrophenoxycarbonyloxymethyl-ester werden drei Stunden bei Raumtemperatur gerührt. Das Reaktionsgemisch wird im Vakuum zur Trockene eingedampft. Chromatographie des Rückstandes an Kieselgel mit Methylenchlorid-Isopropanol 19:1 gibt 223 mg (R)-[1-[4-[4-(Benzoyloxymethoxycarbonylamino-imino-methyl)-phenyl]-2-methyl-4-oxo-butyryl]-piperidin-4-yloxy]-essigsäure-ethylester, MS: 582 (100, M+H), $[\alpha]_D^{20}$ = + 67.4° (Methanol, c = 0.5 %).

Der Benzoesäure-4-nitrophenoxycarbonyloxymethyl-ester kann aus Iodomethyl-4-nitrophenyl-carbonat und Silberbenzoat in siedendem Benzol erhalten werden.

Beispiel 136

Ebenso wie im Beispiel 135 beschrieben erhält man aus dem (R)-[1-[4-[4-(Amino-imino-methyl)-phenyl]-2-methyl-4-oxo-butyryl]-piperidin-4-yloxy]-essigsäure-ethylester-trifluoracetat mit Pivalinsäure-4-nitrophenoxycarbonyloxymethyl-ester den (R)-[1-[4-[4-(Imino-pivaloyloxymethoxycarbonylamino-methyl)-phenyl]-2-methyl-4-oxo-butyryl]-piperidin-4-yloxy]-essigsäure-ethylester als Schaum, MS: 562 (100, M+H), $[\alpha]_D^{20}$ = + 60.8° (Methanol, c = 0.5 %).

Der Pivalinsäure-4-nitrophenoxycarbonyloxymethyl-ester kann aus Iodomethyl-4-nitrophenyl-carbonat und Silberpivalat in siedendem Benzol erhalten werden.

Beispiel 137

In Analogie zu Beispiel 116 erhält man aus dem (R)-[1-[4-(4-Cyano-phenyl)-2-methyl-4-oxo-butyryl]-piperidin-4-yloxy]-essigsäure-2-pyridin-2-yl-ethyl-ester den (R)-(E)/(Z)-[1-[4-[4-(Amino-hydroxyimino-methyl)-phenyl]-2-methyl-4-oxo-butyryl]-piperidin-4-yloxy]-essigsäure-2-pyridin-2-yl-ethyl-ester, MS: 497 (100, M+H), $[\alpha]_D^{20}$ = + 67° (Methanol, c = 0.5 %).

Das Ausgangsmaterial erhält man aus der (R)-[1-[4-(4-Cyano-phenyl)-2-methyl-4-oxo-butyryl]-piperidin-4-yloxy]-essigsäure und 2-Pyridin-2-yl-ethanol in Pyridin in Gegenwart von DCC und pTSOH. MS: 464 (100, M+H), $[\alpha]_D^{20}$ = + 72.8° (Methanol, c = 0.5 %).

Beispiel 138

In Analogie zu Beispiel 116 erhält man aus dem (R)-[1-[4-(4-Cyano-phenyl)-2-methyl-4-oxo-butyryl]-piperidin-4-yloxy]-essigsäure-2-pyridin-3-yl-ethyl-ester den (R)-(E)/(Z)-[1-[4-[4-(Amino-hydroxyimino-methyl)-phenyl]-2-methyl-4-oxo-butyryl]-piperidin-4-yloxy]-essigsäure-2-pyridin-3-yl-ethyl-ester, MS: 497 (100, M+H), $[\alpha]_D^{20}$ = + 65.8° (Methanol, c = 0.5.%).

Das Ausgangsmaterial erhält man aus der (R)-[1-[4-(4-Cyano-phenyl)-2-methyl-4-oxo-butyryl]-piperidin-4-yloxy]-essigsäure und 2-Pyridin-3-yl-ethanol in Pyridin in Gegenwart von DCC und pTSOH. MS: 464 (100, M+H), $[\alpha]_D^{20}$ = + 70.8° (Methanol, c = 0.5 %).

Beispiel 139

In Analogie zu Beispiel 78 erhält man aus dem (E)- oder (Z)-(RS)-3-[1-[(R)-4-[4-(amino-hydroxyimino-methyl)-phenyl]-2-methyl-4-oxo-butyryl]-piperidin-4-yloxyacetoxymethyl]-piperidin-1-carbonsäure-tert-butylester den (E) oder (Z)-(R)-[1-[4-[4-(amino-hydroxyimino-methyl)-phenyl]-2-methyl-4-oxo-butyryl]-piperidin-4-yloxy-essigsäure-(RS)-piperidin-3-yl-methyl-ester als Trifluoracetat (1:2), MS: 489 (100, M+H), $[\alpha]_D^{20}$ = + 45.75° (Methanol, c = 0.4 %).

Beispiel 140

In Analogie zu Beispiel 116 erhält man aus dem (R)-4-[[1-[4-(4-Cyano-phenyl)-2-methyl-4-oxo-butyryl]-piperidin-4-yloxy]-acetoxymethyl]-piperidin-1-carbonsäure-tert-butylester den (E)- oder (Z)-(R)-4-[1-[4-[4-(Amino-hydroxyimino-methyl)-phenyl]-2-methyl-4-oxo-butyryl]-piperidin-4-yloxyacetoxymethyl]-piperidin-1-carbonsäure-tert-butylester; MS: 589 (100, M+H), $[\alpha]_D^{20}$ = + 58.6°, (Methanol, c = 0.5 %).

Das Ausgangsmaterial erhält man aus der (R)-[1-[4-(4-Cyano-phenyl)-2-methyl-4-oxo-butyryl]-piperidin-4-yloxy]-essigsäure und 4-(Hydroxy-methyl)-piperidin-1-carbonsäure-tert-butylester in Pyridin in Gegenwart von DCC und pTSOH. MS: 556 (100, M+H), $[\alpha]_D^{20}$ = + 63.3°, (Methanol, c = 0.3%).

Beispiel 141

In Analogie zu Beispiel 78 erhält man aus dem (E)- oder (Z)-(R)-4-[1-[4-[4-(Amino-hydroxyimino-methyl)-phenyl]-2-methyl-4-oxo-butyryl]-piperidin-4-yloxyacetoxymethyl]-piperidin-1-carbonsäure-tert-butylester den (E) oder (Z)-(R)-[1-[4-[4-(Amino-hydroxyimino-methyl)-phenyl]-2-methyl-4-oxo-butyryl]-piperidin-4-yloxy]-essigsäure-piperidin-4-yl-methyl-ester als Trifluoracetat (1:3), MS: 489 (100, M+H), $[\alpha]_D^{20}$ = + 41.5° (Methanol, c = 0.4 %).

Beispiel 142

In Analogie zu Beispiel 116 erhält man aus dem (R)-[1-[4-(4-Cyano-phenyl)-2-methyl-4-oxo-butyryl]-piperidin-4-yloxy]-essigsäure-2-pyridin-4-yl-ethyl-ester den (E)/(Z)-(R)-[1-[4-[4-(Amino-hydroxyimino-methyl)-phenyl]-2-methyl-4-oxo-butyryl]-piperidin-4-yloxy]-essigsäure-2-pyridin-4-yl-ethyl-ester, MS: 497 (100, M+H), $[\alpha]_D^{20}$ = + 67° (Methanol, c = 0.5 %).

Das Ausgangsmaterial erhält man aus der (R)-[1-[4-(4-Cyano-phenyl)-2-methyl-4-oxo-butyryl]-piperidin-4-yloxy]-essigsäure und 2-Pyridin-4-yl-ethanol in Pyridin in Gegenwart von DCC und pTSOH.

Beispiel 143

In Analogie zu Beispiel 116 erhält man aus dem (R)-[1-[4-(4-Cyano-phenyl)-2-methyl-4-oxo-butyryl]-piperidin-4-yloxy]-essigsäure-(R)-1-phenyl-ethyl-ester den (E)/(Z)-(R)-[1-[4-[4-(Amino-hydroxyimino-methyl)-phenyl]-2-methyl-4-oxo-butyryl]-piperidin-4-yloxy]-essigsäure-(R)-1-phenyl-ethyl-ester, Smp. 94 °C, $[\alpha]_D^{20}$ = + 111.6° (Methanol, c = 0.5 %).

Das Ausgangsmaterial erhält man aus der (R)-[1-[4-(4-Cyano-phenyl)-2-methyl-4-oxo-butyryl]-piperidin-4-yloxy]-essigsäure und (R)-1-Phenyl-ethanol in Pyridin in Gegenwart von DCC und pTSOH; $[\alpha]_D^{20}$ = + 96.6° (Methanol, c = 0.5 %).

Beispiel 144

In Analogie zu Beispiel 116 erhält man aus dem (R)-[1-[4-(4-Cyano-phenyl)-2-methyl-4-oxo-butyryl]-piperidin-4-yloxy]-essigsäure-(S)-1-phenyl-ethyl-ester den (E)/(Z)-(R)-[1-[4-[4-(Amino-hydroxyimino-methyl)-phenyl]-2-methyl-4-oxo-butyryl]-piperidin-4-yloxy]-essigsäure-(S)-1-phenyl-ethyl-ester, Smp. 127 °C, $[\alpha]_D^{20}$ = + 32.8° (Methanol, c = 0.5 %).

Das Ausgangsmaterial erhält man aus der (R)-[1-[4-(4-Cyano-phenyl)-2-methyl-4-oxo-butyryl]-piperidin-4-yloxy]-essigsäure und (S)-1-Phenyl-ethanol in Pyridin in Gegenwart von DCC und pTSOH; Smp. 98 °C, $[\alpha]_D^{20}$ = + 33.8° (Methanol, c = 0.5 %).

Beispiel 145

In Analogie zu Beispiel 116 erhält man aus dem (R)-[1-[4-(4-Cyano-phenyl)-2-methyl-4-oxo-butyryl]-piperidin-4-yloxy]-essigsäure-(S)-1-pyridin-4-yl-ethyl-ester den (E)/(Z)-(R)-[1-[4-[4-(Amino-hydroxyimino-methyl)-phenyl]-2-methyl-4-oxo-butyryl]-piperidin-4-yloxy]-essigsäure-(S)-1-pyridin-4-yl-ethyl-ester, $[\alpha]_D^{20}$ = + 41° (Methanol, c = 0.5 %).

Das Ausgangsmaterial erhält man aus der (R)-[1-[4-(4-Cyano-phenyl)-2-methyl-4-oxo-butyryl]-piperidin-4-yloxy]-essigsäure und (S)-1-(4-Pyridyl)-ethanol in Pyridin in Gegenwart von DCC und pTSOH; Smp. 115 °C, $[\alpha]_D^{20}$ = +45.8° (Methanol, c = 0.5 %).

Beispiel 146

In Analogie zu Beispiel 116 erhält man aus dem (R)-[1-[4-(4-Cyano-phenyl)-2-methyl-4-oxo-butyryl]-piperidin-4-yloxy]-essigsäure-(R)-1-pyridin-4-yl-ethyl-ester den (E)/(Z)-(R)-[1-[4-[4-(Amino-hydroxyimino-methyl)-phenyl]-2-methyl-4-oxo-butyryl]-piperidin-4-yloxy]-essigsäure-(R)-1-pyridin-4-yl-ethyl-ester, MS: 497 (100, M+H), $[\alpha]_D^{20}$ = + 96.8° (Methanol, c = 0.5%).

Das Ausgangsmaterial erhält man aus der (R)-[1-[4-(4-Cyano-phenyl)-2-methyl-4-oxo-butyryl]-piperidin-4-yloxy]-essigsäure und (R)-1-(4-Pyridyl)-ethanol in Pyridin in Gegenwart von DCC und pTSOH; Smp. 115 °C, $[\alpha]_D^{20}$ = +112.4° (Methanol, c = 0.5 %).

Beispiel 147

In Analogie zu Beispiel 116 erhält man aus dem (R)-[1-[4-(4-Cyano-phenyl)-2-methyl-4-oxo-butyryl]-piperidin-4-yloxy]-essigsäure-methyl-ester den (E)/(Z)-(R)-[1-[4-[4-(Amino-hydroxyimino-methyl)-phenyl]-2-methyl-4-oxo-butyryl]-piperidin-4-yloxy]-essigsäure-methyl-ester, Smp. 147-149 °C, $[\alpha]_D^{20}$ = + 86° (Methanol, c = 0.5 %).

Das Ausgangsmaterial erhält man aus der (R)-[1-[4-(4-Cyano-phenyl)-2-methyl-4-oxo-butyryl]-piperidin-4-yloxy]-essigsäure und Methanol in Pyridin in Gegenwart von DCC und pTSOH als (2:1)-Addukt mit Dicyclohexylharnstoff, Smp. 101 °C, $[\alpha]_D^{20}$ = + 76.2° (Methanol, c = 0.5 %).

Beispiel 148

In Analogie zu Beispiel 79 erhält man aus dem (R)-[1-[4-[4-(Amino-imino-methyl)-phenyl]-2-methyl-4-oxo-butyryl]-piperidin-4-yloxy]-essigsäure-methylester-acetat/iodid und Chlorameisensäuremethylester den (R)-[1-[4-[4-(Imino-methoxycarbonylamino-methyl)-phenyl]-2-methyl-4-oxo-butyryl]-piperidin-4-yloxy]-essigsäure-methylester, MS: 448 (100, M+H), $[\alpha]_D^{20}$ = +75.4° (Methanol, c = 0.5 %).

Das Ausgangsmaterial kann wie folgt hergestellt werden:

a. (R)-[1-[4-(4-Cyano-phenyl)-2-methyl-4-oxo-butyryl]-piperidin-4-yloxy]-essigsäure-methylester wird mit Schwefelwasserstoff in Pyridin und Triethylamin in den (R)-[1-[2-Methyl-4-oxo-4-(4-thiocarbamoyl-phenyl)-butyryl]-piperidin-4-yloxy]-essigsäure-methylester, Smp. 172-177 °C, übergeführt.

b. Durch Umsetzen mit Methyliodid in Aceton und anschliessende Reaktion mit Ammoniumacetat und Essigsäure in Methanol erhält man daraus das (R)-[1-[4-[4-(Amino-imino-methyl)-phenyl]-2-methyl-4-oxo-butyryl]-piperidin-4-yloxy]-essigsäure-methylester-acetat/iodid.

Beispiel 149

In Analogie zu Beispiel 79 erhält man aus dem (R)-[1-[4-[4-(Amino-imino-methyl)-phenyl]-2-methyl-4-oxo-butyryl]-piperidin-4-yloxy]-essigsäure-methylester-acetat/iodid und Chlorameisensäureethylester den (R)-[1-[4-[4-(Ethoxycarbonylamino-imino-methyl)-phenyl]-2-methyl-4-oxo-butyryl]-piperidin-4-yloxy]-essigsäure-methylester, MS: 462 (100, M+H), $[\alpha]_D^{20}$ = +66° (Methanol, c = 0.5 %).

Beispiel 150

214 mg eines Gemisches von (R)-[1-[4-[4-[tert-Butoxycarbonylimino-(isobutoxycarbonyl-tert-butoxycarbonyl-amino)-methyl]-phenyl]-2-methyl-4-oxo-butyryl]-piperidin-4-yloxy]-essigsäure-pyridin-3-ylmethyl-ester und (R)-[1-[4-[4-[(Di-tert-butoxycarbonyl-amino)-isobutoxycarbonylimino-methyl]-phenyl]-2-methyl-4-oxo-butyryl]-piperidin-4-yloxy]-essigsäure-pyridin-3-ylmethyl-ester werden in 1.2 ml Methylenchlorid und 1.2 ml Trifluoressigsäure 2 Std. bei 20 °C gerührt. Das Lösungsmittel wird im Vakuum eingedampft, der Rückstand in Essigester gelöst und nacheinander mit verdünnter $NaHCO_3$-Lösung, Wasser und gesättigter NaCl-Lösung gewaschen, getrocknet und im Vakuum eingedampft. Man erhält 151 mg (R)-[1-[4-[4-(Imino-isobutoxycarbonylamino-methyl)-phenyl]-2-methyl-4-oxo-butyryl]-piperidin-4-yloxy]-essigsäure-pyridin-3-ylmethyl-ester als Schaum; MS: 567 (100, M+H), $[\alpha]_D^{20}$ = + 61° (Methanol, c = 0.2 %).

Das Ausgangsmaterial kann wie folgt erhalten werden:

a. (R)-[1-[4-[4-(Imino-isobutoxycarbonylamino-methyl)-phenyl]-2-methyl-4-oxo-butyryl]-piperidin-4-yloxy]-essigsäure-ethylester wird in Acetonitril in Gegenwart von 4-Dimethylamino-pyridin mit Di-tert-butyl-dicarbonat zu einem Gemisch von (R)-[1-[4-[4-[tert-Butoxycarbonylimino-(isobutoxycarbonyl-tert-butoxycarbonyl-amino)-methyl]-phenyl]-2-methyl-4-oxo-butyryl]-piperidin-4-yloxy]-essigsäure-ethylester und (R)-[1-[4-[4-[(Di-tert-butoxycarbonyl-amino)-isobutoxycarbonylimino-methyl]-phenyl]-2-methyl-4-oxo-butyryl]-piperidin-4-yloxy]-essigsäure-ethylester umgesetzt.

b. Durch Verseifung mit LiOH in wässerigem THF erhält man daraus ein Gemisch von (R)-[1-[4-[4-[tert-Butoxycarbonylimino-(isobutoxycarbonyl-tert-butoxycarbonyl-amino)-methyl]-phenyl]-2-methyl-4-oxo-butyryl]-piperidin-4-yloxy]-essigsäure und (R)-[1-[4-[4-[(Di-tert-butoxycarbonylamino)-isobutoxycarbonylimino-methyl]-phenyl]-2-methyl-4-oxo-butyryl]-piperidin-4-yloxy]-essigsäure.

c. Dieses wird in Pyridin mit 3-Hydroxymethyl-pyridin, DCC und p-TsOH zu einem Gemisch von (R)-[1-[4-[4-[tert-Butoxycarbonylimino-(isobutoxycarbonyl-tert-butoxycarbonyl-amino)-methyl]-phenyl]-2-methyl-4-oxo-butyryl]-piperidin-4-yloxy]-essigsäure-pyridin-3-ylmethyl-ester und (R)-[1-[4-[4-[(Di-tert-butoxycarbonyl-amino)-isobutoxycarbonylimino-methyl]-phenyl]-2-methyl-4-oxo-butyryl]-piperidin-4-yloxy]-essigsäure-pyridin-3-ylmethyl-ester umgesetzt.

Beispiel 151

In Analogie zu Beispiel 79 erhält man aus dem (RS)-3-[[1-[(R)-4-[4-(Amino-imino-methyl)-phenyl]-2-methyl-4-oxo-butyryl]-piperidin-4-yloxy]-acetoxymethyl]-piperidin-1-carbonsäure-tert-butyl-ester-acetat/iodid und Chlorameisensäure-methylester den (RS)-3-[[1-[(R)-4-[4-(Imino-methoxycarbonylamino-methyl)-phenyl]-2-methyl-4-oxo-butyryl]-piperidin-4-yloxy]-acetoxymethyl]-piperidin-1-carbonsäure-tert-butylester, MS: 631 (100, M+H), $[\alpha]_D^{20}$ = + 57.2° (Methanol, c = 0.5 %).

Das Ausgangsmaterial kann wie folgt erhalten werden:

a. (RS)-3-[1-[(R)-4-(4-Cyano-phenyl)-2-methyl-4-oxo-butyryl]-piperidin-4-yloxy-acetoxymethyl]-piperidin-1-carbonsäure-tert-butylester wird mit $H_2S$ in Pyridin und Triethylamin zum (RS)-3-[[1-[(R)-2-Methyl-4-oxo-4-(4-thiocarbamoyl-phenyl)-butyryl]-piperidin-4-yloxy]-acetoxymethyl]-piperidin-1-carbonsäure-tert-butylester umgesetzt; MS: 590 (100, M+H), $[\alpha]_D^{20}$ = +58.8° (Methanol, c = 0.5 %).
b. Durch Umsetzen mit Methyliodid in Aceton und anschliessende Reaktion mit Ammoniumacetat und Essigsäure in Methanol erhält man daraus das (RS)-3-[[1-[(R)-4-[4-(Amino-imino-methyl)-phenyl]-2-methyl-4-oxo-butyryl]-piperidin-4-yloxy]acetoxymethyl]-piperidin-1-carbonsäure-tert-butylester-acetat/iodid.

Beispiel 152

In Analogie zu Beispiel 79 erhält man aus dem (RS)-3-[[1-[(R)-4-[4-(Amino-imino-methyl)-phenyl]-2-methyl-4-oxo-butyryl]-piperidin-4-yloxy]acetoxymethyl]-piperidin-1-carbonsäure-tert-butylester-acetat/iodid und Chlorameisensäure-ethylester den (RS)-3-[[1-[(R)-4-[4-(Ethoxycarbonylamino-imino-methyl)-phenyl]-2-methyl-4-oxo-butyryl]-piperidin-4-yloxy]-acetoxymethyl]-piperidin-1-carbonsäure-tert-butylester, MS: 645 (100, M+H), $[\alpha]_D^{20}$ = + 56.2° (Methanol, c = 0.5 %).

Beispiel 153

In Analogie zu Beispiel 79 erhält man aus dem (R)-[1-[4-[4-(Amino-imino-methyl)-phenyl]-2-methyl-4-oxo-butyryl]-piperidin-4-yloxy]-essigsäure-ethylester-acetat/iodid und Chlorameisensäuremethylester den (R)-[1-[4-[4-(Imino-methoxycarbonylamino-methyl)-phenyl]-2-methyl-4-oxo-butyryl]-piperidin-4-yloxy]-essigsäure-ethyl-ester.

Beispiel 154

In Analogie zu Beispiel 116 erhält man aus dem [4-[4-(4-Cyano-phenyl)-4-oxo-butyryl]-phenoxy]-essigsäure-ethylester den (E)/(Z)-[4-[4-[4-(Amino-hydroxyimino-methyl)-phenyl]-4-oxo-butyryl]-phenoxy]-essigsäure-ethylester, Smp. 174-175 °C

Beispiel 155

In Analogie zu Beispiel 116 erhält man aus dem (RS)-[4-[4-(4-Cyano-phenyl)-2-methyl-4-oxo-butyryl]-phenoxy]-essigsäure-ethylester den (E)/(Z)-(RS)-[4-[4-[4-(Amino-hydroxyimino-methyl)-phenyl]-2-methyl-4-oxo-butyryl]-phenoxy]-essigsäure-ethylester, Smp. 166-167 °C.

Das Ausgangsmaterial kann wie folgt hergestellt werden:

a. Aus [4-(1-Oxo-propyl)-phenoxy]-essigsäure-ethylester und Brom erhält man in Essigsäure den (RS)-[4-(2-Brom-1-oxo-propyl)-phenoxy]-essigsäure-ethylester, Smp. 72-76 °C.
b. Dieser wird in Aceton in Gegenwart von Natrium-bis-(trimethylsilyl)-amid mit 3-(4-Cyano-phenyl)-3-oxo-propionsäure-tert.-butylester zum [4-[3-tert.-Butoxycarbonyl-3-(4-cyanobenzoyl)-2-methyl-propionyl]-phenoxy]-essigsäure-ethylester (racemisches Diastereomerengemisch) umgesetzt; MS: 406 (1.7, M - $COOC_2H_5$).
c. In Ameisensäure erhält man daraus den (RS)-[4-[4-(4-Cyano-phenyl)-2-methyl-4-oxo-butyryl]-phenoxy]-essigsäure-ethylester, Smp. 117-123 °C.

Beispiel 156

365 mg [4-[4-(4-Cyano-phenyl)-4-oxo-butyryl]-phenoxy]-essigsäure-ethylester werden in 20 ml 0.1 N ethanolischer Hydroxylaminlösung 4 Tage bei 20 °C gerührt. Das Reaktionsgemisch wird im Vakuum eingeengt, mit Essigester verdünnt und mit Wasser und gesättigter Kochsalzlösung gewaschen. Die Essigesterlösung wird über $Na_2SO_4$ getrocknet und im Vakuum einge—dampft. Chromatographie an Kieselgel mit $CH_2Cl_2$-Ethanol 98:2 gibt 111 mg [4-[(E)/(Z)-4-[(E)/(Z)-4-(Amino-hydroxyimino-methyl)-phenyl]-1-hydroxyimino-4-oxo-butyl]-phenoxy]-essigsäure-ethylester, Smp. 180-181 °C.

Beispiel 157

84 mg [4-[4-[4-(tert-Butoxycarbonylamino-imino-methyl)-phenyl]-4-oxo-butyryl]-phenoxy]-essigsäure und 1.7 ml Ameisensäure werden sieben Stunden bei 20 °C gerührt. Das Reaktionsgemisch wird eingedampft, der Rückstand in Wasser aufgenommen und wieder eingedampft. Der feste Rückstand wird in Wasser suspendiert, mit Ammoniak auf pH 8 eingestellt, abgenutscht, mit Wasser gewaschen und getrocknet. Man erhält 50 mg [4-[4-[4-(Amino-imino-methyl)-phenyl]-4-oxo-butyryl]-phenoxy]-essigsäure, Smp. 284 °C.

Das Ausgangsmaterial kann wie folgt erhalten werden:

a. [4-[4-[4-(Amino-imino-methyl)-phenyl]-4-oxo-butyryl]-phenoxy]-essigsäure-ethylester acetat (1:1) werden in $CH_2Cl_2$ und Wasser in Gegenwart von $Na_2CO_3$ mit Di-tert-butyl-dicarbonat in den [4-[4-[4-(tert-Butoxycarbonylamino-imino-methyl)-phenyl]-4-oxo-butyryl]-phenoxy]-essigsäure-ethylester umgewandelt, Smp. 164 °C.
b. Durch Verseifung mit NaOH in EtOH erhält man daraus die [4-[4-[4-(tert-Butoxycarbonylamino-imino-methyl)-phenyl]-4-oxo-butyryl]-phenoxy]-essigsäure, Smp. 284 °C (Zers.).

Beispiel 158

278 mg Hydroxylamin-hydrochlorid, 4 ml DMSO und 0.56 ml Triethylamin werden 10 Min. bei 20 °C gerührt. Nach Zugabe von 146 mg [4-[4-(4-Cyano-phenyl)-4-oxo-butyryl]-phenoxy]-essigsäure-ethylester wird 3 Tage bei 20 °C gerührt. Das Reaktionsgemisch wird mit Essigester verdünnt, mit Wasser und Kochsalzlösung gewaschen, getrocknet und eingedampft. Chromatographie an Kieselgel mit $CH_2Cl_2$-EtOH 9:1 gibt 42 mg [4-[4-[4-(Amino-hydroxyimino-methyl)-phenyl]-1,4-bis-hydroxyimino-butyl]-phenoxy]-essigsäure-ethylester (alle Oxime : E oder Z), Smp. 210 °C (Zers.).

Beispiel 159

In Analogie zu Beispiel 116 erhält man aus dem (RS)-[4-[2-(2-Acetoxy-ethyl)-4-(4-cyano-phenyl)-4-oxo-butyryl]-phenoxy-essigsäure-ethyl-ester den (E/Z)-(RS)-[4-[2-(2-Acetoxy-ethyl)-4-[4-(amino-hydroxyimino-methyl)-phenyl]-4-oxo-butyryl]-phenoxy]-essigsäure-ethyl-ester als farbloses Harz, MS: 485 (100, M+H).

Das Ausgangsmaterial kann wie folgt hergestellt werden:

a. Aus 4-Hydroxy-1-(4-hydroxy-phenyl)-butan-1-on und Bromessigsäure-ethylester erhält man in Aceton in Gegenwart von $K_2CO_3$ den [4-(4-Hydroxy-butyryl)-phenoxy]-essigsäure-ethylester, Smp. 64-66 °C.
b. Mit Essigsäureanhydrid in Pyridin erhält man daraus den [4-(4-Acetoxy-butyryl)-phenoxy]-essigsäure-ethylester, Smp. 94-96 °C.
c. Dieser wird mit Brom in Essigsäure zum [4-(4-Acetoxy-2-bromo-butyryl)-phenoxy]-essigsäure-ethylester umgesetzt.
d. Letzterer wird in Aceton in Gegenwart von Natrium-bis-(trimethylsilyl)-amid mit 3-(4-Cyano-phenyl)-3-oxo-propionsäure-tert.-butylester zum 5-Acetoxy-2-(4-cyano-benzoyl)-3-(4-ethoxycarbonylmethoxy-benzoyl)-pentansäure-tert-butylester (racemisches Diastereomerengemisch) umgesetzt, MS: 478 (0.2, M-$CO_2$Et).
e. Durch Erwärmen in Ameisensäure auf 40 °C und anschliessende Chromatographie erhält man daraus den (RS)-[4-[2-(2-Acetoxy-ethyl)-4-(4-cyano-phenyl)-4-oxo-butyryl]-phenoxy-essigsäure-ethyl-ester, Smp. 102-104°C.

Beispiel 160

425 mg (RS)-[4-[4-[4-(tert-Butoxycarbonylamino-imino-methyl)-phenyl]-2-methyl-4-oxo-butyryl]-phenoxy]-essigsäure-ethylester werden 2.5 Stunden in 40 ml Essigsäure auf 40 °C erwärmt. Das Reaktionsgemisch wird im Vakuum zur Trockene eingedampft. Aus Essigester-Aether erhält man 239 mg (RS)-[4-[4-[4-(Amino-imino-methyl)-phenyl]-2-methyl-4-oxo-butyryl]-phenoxy]-essigsäure-ethylester als Acetat (1:1), Smp. 198 °C.

Das Ausgangsmaterial kann wie folgt erhalten werden:

a. (RS)-[4-[4-(4-Cyano-phenyl)-2-methyl-4-oxo-butyryl]-phenoxy]-essigsäure-ethylester wird mit $H_2S$ in Pyridin und Triethylamin in den (RS)-[4-[2-methyl-4-oxo-4-(4-thiocarbamoyl-phenyl)-butyryl]-phenoxy]-essigsäure-ethylester, Smp. 112-113 °C, umgewandelt.
b. Dieser wird zunächst mit Methyliodid in Aceton, anschliessend mit Ammoniumacetat und Essigsäure in Methanol und schliesslich mit Di-tert.-butyl-dicarbonat in Methylenchlorid/Wasser/$Na_2CO_3$ zum (RS)-[4-[4-[4-(tert-Butoxycarbonylamino-imino-methyl)-phenyl]-2-methyl-4-oxo-butyryl]-phenoxy]-essigsäure-ethylester umgesetzt; MS: 497 (100, M+H).

Beispiel 161

In Analogie zu Beispiel 79 erhält man aus dem (RS)-[4-[4-[4-(Amino-imino-methyl)-phenyl]-2-methyl-4-oxo-butyryl]-phenoxy]-essigsäure-ethylester und Chlorameisensäure-isobutylester den (RS)-[4-[4-[4-(Imino-isobutoxycarbonylamino-methyl)-phenyl]-2-methyl-4-oxo-butyryl]-phenoxy]-essigsäure-ethylester als Harz. MS: 497 (100, M+H).

Beispiel 162

In Analogie zu Beispiel 160 erhält man aus dem (RS)-[4-[2-(2-Acetoxy-ethyl)-4-[4-(tert.-butoxycarbonyl-amino-imino-methyl)-phenyl]-4-oxo-butyryl]-phenoxy]-essigsäure-ethyl-ester den (RS)-[4-[2-(2-Acetoxy-ethyl)-4-[4-(amino-imino-methyl)-phenyl]-4-oxo-butyryl]-phenoxy]-essigsäure-ethyl-ester als Acetat (1:1), Smp. 183 °C.

Das Ausgangsmaterial kann wie folgt erhalten werden:

a. Aus dem (RS)-[4-[2-(2-Acetoxy-ethyl)-4-(4-cyano-phenyl)-4-oxo-butyryl]-phenoxy-essigsäure-ethyl-ester erhält man mit $H_2S$ in Pyridin und Triethylamin den (RS)-[4-[2-(2-Acetoxy-ethyl)-4-oxo-4-(4-thiocarbamoyl-phenyl)-butyryl]-phenoxy]-essigsäure-ethyl-ester, MS: 508 (100, M+Na).

b. Dieser wird zunächst mit Methyliodid in Aceton, anschliessend mit Ammoniumacetat und Essigsäure in Methanol und schliesslich mit Di-tert.-butyl-dicarbonat in Methylenchlorid/Wasser/$Na_2CO_3$ zum (RS)-[4-[2-(2-Acetoxy-ethyl)-4-[4-(tert.-butoxycarbonylamino-imino-methyl)-phenyl]-4-oxo-butyryl]-phenoxy]-essigsäure-ethyl-ester umgesetzt, MS: 569 (100, M+H).

Beispiel 163

Eine Lösung von 413 mg (Z)-(S)-[4-[2-[4-(Amino-hydroxyimino-methyl)-benzoylamino]-propionyl]-phenoxy]-essigsäureethylester und 120 mg 4-Methylmorpholin in 10 ml Dichlormethan wird bei 0 °C mit 98 mg Triphosgen in 10 ml Dichlormethan versetzt und 2 h bei Raumtemperatur gerührt. Die Reaktionslösung wird mit Wasser gewaschen, getrocknet und eingeengt. Der Rückstand wird in Ether suspendiert und abgenutscht. Man erhält 50 mg (S)-[4-[2-[4-(5-Oxo-4,5-dihydro-1,2,4-oxadiazol-3-yl)-benzoylamino]-propionyl]-phenoxy]-essigsäureethylester, MS (ISP): 440 $(M+H)^+$.

Beispiel 164

Zu einer Suspension von 650 mg (S)-4-[2-[4-(Amino-imino-methyl)-benzoylamino]-propionyl]-phenoxyessigsäureethylester-trifluoracetat in 10 ml Dichlormethan und 10 ml gesättigter $NaHCO_3$-Lösung gibt man eine Lösung von 120 mg S-Ethyl-chlorothioformat in 2 ml Dichlormethan und rührt anschliessend 47 h bei Raumtemperatur. Die wässerige Phase wird mit Dichlormethan extrahiert, die Dichlormethanphasen mit Wasser gewaschen, getrocknet und eingeengt. Nach Chromatographie des Rückstands an Kieselgel (Hexan/Essigester 1:2) erhält man 230 mg (S)-[4-[2-[4-(Amino-ethylsulfanylcarbonylimino-methyl)-benzoylamino]-propionyl]-phenoxy]-essigsäureethylester, $[\alpha]^{20}_{D}$ = + 75,1° (c = 0,7, Chloroform).

Beispiel 165

Eine Lösung von 190 mg (S)-4-[2-[4-[(Amino-(2-tert-butyl-dimethylsilanyloxy-ethoxycarbonylimino)-methyl]-benzoylamino]-propionyl]-phenoxy-essigsäureethylester in 10 ml Tetrahydrofuran wird bei - 20 °C mit 0,3 ml Tetrabutylammoniumfluorid (1 M in Tetrahydrofuran) versetzt und 2 h bei Raumtemperatur gerührt. Das Reaktionsgemisch wird mit Essigester verdünnt, mit Wasser extrahiert, getrocknet und eingeengt. Chromatographie des Rückstands an Kieselgel (Essigester/5 % Ethanol) ergibt 56 mg (S)-[4-[2-[4-[Amino-(2-hydroxy-ethoxycarbonylimino)-methyl]-benzoylamino]-propionyl]-phenoxy]-essigsäureethylester, MS (ISP): 486 $(M+H)^+$.

Das Ausgangsmaterial kann wie folgt hergestellt werden:

Zu einer Lösung von 230 mg 2-(tert-Butyl-dimethyl-silanyloxy)-ethanol und 101 mg 4-Methylmorpholin in 8 ml Dichlormethan werden bei 0 °C 98 mg Triphosgen gelöst in 4 ml Dichlormethan zugegeben und 2 h bei Raumtemperatur gerührt. Die Reaktionslösung wird anschliessend zu einer Suspension von 510 mg (S)-4-[2-[4-(Amino-imino-methyl)-benzoylamino]-propionyl]-phenoxyessigsäureethylester-trifluoracetat in 10 ml Dichlormethan und 10 ml gesättigter $Na_2CO_3$-Lösung zugegeben. Nach 10 min. Rühren bei Raumtemperatur werden die Phasen getrennt, die organische Phase mit Wasser gewaschen, getrocknet und eingeengt. Chromatographie des Rückstands an Kieselgel (Hexan/Essigester 1:2) ergibt 200 mg (S)-[4-[2-[4-[(Amino-(2-tert-butyl-dimethylsilanyloxy-ethoxycarbonylimino)-methyl]-benzoylamino]-propionyl]-phenoxy]-essigsäureethylester, Rf = 0,73 (Essigester).

Beispiel 166

In Analogie zu Beispiel 165 a) erhält man aus 511 mg (S)-4-[2-[4-(Amino-imino-methyl)-benzoylamino]-propionyl]-phenoxyessigsäure-ethylester-trifluoracetat und 104 mg 2-Acetoxyethanol nach Chromatographie an Kieselgel (Hexan/Essigester 1:4) 75 mg (S)-[4-[2-[4-[(2-Acetoxyethoxycarbonylimino)-amino-methyl]-benzoylamino]-propionyl]-phenoxy]-essigsäureethylester, MS (ISP): 528 $(M+H)^+$.

Beispiel 167

In Analogie zu Beispiel 165 a) erhält man aus 511 mg (S)-4-[2-[4-(Amino-imino-methyl)-benzoylamino]-propionyl]-phenoxyessigsäure- ethylester-trifluoracetat und 118 mg Propionsäure-2-hydroxy-ethylester nach Chromatographie an Kieselgel (Hexan/Essigester 1:4) 175 mg Propionsäure-(S)-2-[[4-[2-(4-ethoxycarbonylmethoxy-phenyl)-1-methyl-2-oxo-ethylcarbamoyl]-phenyl]-imino-methylcarbamoyloxy]-ethylester, MS (ISP): 542 $(M+H)^+$.

Beispiel 168

In Analogie zu Beispiel 165 a) erhält man aus 511 mg (S)-4-[2-[4-(Amino-imino-methyl)-benzoylamino]-propionyl]-phenoxyessigsäure-ethylester-trifluoracetat und 132 mg Isobuttersäure-2-hydroxy-ethylester nach Chromatographie an Kieselgel (Hexan/Essigester 1:2) 42 mg 2-Methylpropionsäure-(S)-2-[[4-[2-(4-ethoxycarbonylmethoxy-phenyl)-1-methyl-2-oxo-ethylcarbamoyl]-phenyl]-imino-methylcarbamoyloxy]-ethylester, MS (ISP): 556 $(M+H)^+$.

Beispiel 169

In Analogie zu Beispiel 165 a) erhält man aus 511 mg (S)-4-[2-[4-(Amino-imino-methyl)-benzoylamino]-propionyl]-phenoxyessigsäure-ethylester-trifluoracetat und 130 mg Methacrylsäure-2-hydroxy-ethylester nach Chromatographie an Kieselgel (Hexan/Essigester 1:3) 160 mg 2-Methyl-acrylsäure-(S)-2-[[4-[2-(4-ethoxycarbonylmethoxy-phenyl)-1-methyl-2-oxo-ethylcarbamoyl]-phenyl]-imino-methylcarbamoyloxy]-ethylester, MS (ISP): 554 $(M+H)^+$.

Beispiel 170

In Analogie zu Beispiel 165 a) erhält man aus 511 mg (S)-4-[2-[4-(Amino-imino-methyl)-benzoylamino]-propionyl]-phenoxyessigsäure-ethylester-trifluoracetat und 166 mg Benzoesäure-2-hydroxy-ethylester nach Chromatographie an Kieselgel (Hexan/Essigester 1:2) 130 mg Benzoesäure-(S)-2-[[4-[2-(4-ethoxycarbonylmethoxy-phenyl)-1-methyl-2-oxo-ethylcarbamoyl]-phenyl]-imino-methylcarbamoyloxy]-ethylester, MS (ISP): 590 $(M+H)^+$.

Beispiel 171

In Analogie zu Beispiel 165 a) erhält man aus 511 mg (S)-4-[2-[4-(Amino-imino-methyl)-benzoylamino]-propionyl]-phenoxyessigsäure-ethylester-trifluoracetat und 224 mg 2-Acetoxybenzoesäure-2-hydroxy-ethylester nach Chromatographie an Kieselgel (Hexan/Essigester 1:4) 210 mg 2-Acetoxybenzoesäure-(S)-2-[[4-[2-(4-ethoxycarbonylmethoxy-phenyl)-1-methyl-2-oxo-ethylcarbamoyl]-phenyl]-imino-methylcarbamoyloxy]-ethylester, MS (ISP): 648 $(M+H)^+$.

Beispiel 172

In Analogie zu Beispiel 165 a) erhält man aus 511 mg (S)-4-[2-[4-(Amino-imino-methyl)-benzoylamino]-propionyl]-phenoxyessigsäure-ethylester-trifluoracetat und 103 mg Essigsäure-2-hydroxyethylamid nach Chromatographie an Kieselgel (Essigester) 54 mg (S)-[4-[2-[4-[(2-Acetyl-amino-ethoxycarbonylimino)-amino-methyl]-benzoylamino]-propionyl]-phenoxy]-essigsäureethylester , MS (ISP): 527 $(M+H)^+$.

Beispiel 173

Eine Suspension von 263 mg (S)-4-[2-[4-(Amino-imino-methyl)-benzoylamino]-propionyl]-phenoxyessigsäureethylester-trifluoracetat, 217 mg Essigsäure-4-nitro-phenoxycarbonyloxymethylester und 165 ml 4-Methylmorpholin

in 5 ml Dichlormethan und 5 ml Tetrahydrofuran wird 16 h bei Raumtemperatur gerührt. Einengen des Reaktionsgemisches und Chromatographie des Rückstands an Kieselgel (Hexan/Essigester 1:4) ergibt 193 mg (S)-[4-[2-[4-(Acetoxymethoxycarbonylimino-amino-methyl)-benzoylamino]-propionyl]-phenoxy]-essigsäureethylester, $[\alpha]_D^{20}$ = + 65,0° (c = 0,6, Chloroform).

Beispiel 174

In Analogie zu Beispiel 165 a) erhält man aus 250 mg (S)-4-[2-[4-(Amino-imino-methyl)-benzoylamino]-propionyl]-phenoxyessigsäureethylester-trifluoracetat und 104 mg (S)-Milchsäureethylester nach Chromatographie an Kieselgel (Hexan/Essigester 1:2) 160 mg (S)-2-[[4-[(S)-2-(4-Ethoxycarbonylmethoxy-phenyl)-1-methyl-2-oxo-ethylcarbamoyl]-phenyl]-imino-methylcarbamoyloxy]-propionsäuremethylester, MS (ISP): 528 $(M+H)^+$.

Beispiel 175

Analog zu Beispiel 31 erhält man ausgehend von 380 mg 1-(8-Cyano-5-oxo-2,3,4,5-tetrahydro-1,4-benzoxazepin-4-ylacetyl)-piperidin-4-yloxyessigsäuremethylester nach Reaktion mit Ammoniumacetat, Einengen der Reaktionslösung und Fällung des Produkts mit Ether 130 mg 1-[8-(Amino-imino-methyl)-5-oxo-2,3,4,5-tetrahydro-1,4-benzoxazepin-4-ylacetyl]- piperidin-4-yloxyessigsäuremethylester-acetat, MS (ISP): 419 $(M+H)^+$.

Das Ausgangsmaterial kann wie folgt hergestellt werden:

a) Eine Lösung von 1,63 g 4-Cyanosalicylsäure und 1,26 g N-Hydroxy-succinimid in 25 ml Dichlormethan und 25 ml Dimethylformamid wird bei 0°C mit 2,06 g Dicyclohexylcarbodiimid versetzt und 16 h bei 0 °C gerührt. Der Niederschlag wird abgenutscht. Zu der Mutterlauge werden 1,4 ml Triethylamin und 1,38 g N-(2-Hydroxyethyl)glycin-tert-butylester (EP 288256) zugegeben und 1 h bei Raumtemperatur gerührt. Einengen der Reaktionslösung und Chromatographie des Rückstands an Kieselgel (Hexan/Essigester 1:2) ergibt 475 mg [(4-Cyano-2-hydroxy-benzoyl)-(2-hydroxy-ethyl)-amino]-essigsäure-tert-butylester, MS (ISP): 321 $(M+H)^+$.

b) Zu einer Lösung von 464 mg [(4-Cyano-2-hydroxy-benzoyl)-(2-hydroxy-ethyl)-amino]-essigsäure-tert-butylester und 570 mg Triphenylphosphin in 10 ml Tetrahydrofuran werden bei 0 °C 378 mg Diethylazodicarboxylat in 5 ml Tetrahydrofuran zugetropft und 1 h bei Raumtemperatur gerührt. Einengen der Lösung und Chromatographie des Rückstands an Kieselgel (Hexan/Essigester 2:1) ergibt 300 mg 8-Cyano-5-oxo-2,3,4,5-tetrahydro-1,4-benzoxazepin-4-yl-essigsäure-tert-butylester, MS (EI): 246 (M-56).

c) Die Abspaltung der Estergruppe an 300 mg der Vorstufe in 4 ml Dichlormethan und 2 ml Trifluoressigsäure erfolgt bei Raumtemperatur. Die Lösung wird eingeengt, der Rückstand mit Ether abgenutscht, in 50 ml Dimethylformamid gelöst und mit 292 mg 4-Methylmorpholin 597 mg O-Benzotriazol-1-yl-N,N,N',N'-tetramethyluronium-hexafluorophosphat und 330 mg 4-Piperidinoxyessigsäuremethylester hydrochlorid versetzt. Nach 16 h Rühren bei Raumtemperatur wird die Lösung eingeengt, der Rückstand in Essigester aufgenommen, die organische Phase mit 1M $KHSO_4$-Lösung und ges. NaCl-Lösung gewaschen, getrocknet und eingeengt. Chromatographie des Rückstands an Kieselgel (Essigester) ergibt 400 mg 1-(8-Cyano-5-oxo-2,3,4,5-tetrahydro-1,4-benzoxazepin-4-ylacetyl)-piperidin-4-yloxyessigsäuremethylester, MS (ISP): 402 $(M+H)^+$.

Beispiel 176

A) Durch Behandlung von 350 mg (S)-[1-[2-(8-Cyano-5-oxo-2,3,4,5-tetrahydro-1,4-benzoxazepin-4-yl)-propionyl]-piperidin-4-yloxy]-essigsäure-ethylester mit Schwefelwasserstoff, Methyljodid und Ammoniumacetat erhält man nach Chromatographie an silyliertem Kieselgel RP18 163 mg (S)-[1-[2-[8-(Amino-imino-methyl)-5-oxo-2,3,4,5-tetrahydro-1,4-benzoxazepin-4-yl]-propionyl]-piperidin-4-yloxy]-essigsäureethylester-trifluoracetat, MS (ISP): 447 $(M+H)^+$.

B) Zu einer Suspension von 135 mg (S)-[1-[2-[8-(Amino-imino-methyl)-5-oxo-2,3,4,5-tetrahydro-1,4-benzoxazepin-4-yl]-propionyl]-piperidin-4-yloxy]-essigsäureethylester-trifluoracetat in 3 ml Dichlormethan und 3 ml gesättigter $NaHCO_3$-Lösung gibt man 29 mg Chlorameisensäureethylester und rührt anschliessend 5 min. bei Raumtemperatur. Die wässerige Phase wird mit Dichlormethan extrahiert, die Dichlormethanphasen mit Wasser gewaschen, getrocknet und eingeengt. Nach Chromatographie des Rückstands an Kieselgel (Essigester) erhält man 86 mg (S)-[1-[2-[8-(Amino-ethoxycarbonylimino-methyl)-5-oxo-2,3,4,5-tetrahydro-1,4-benzoxazepin-4-yl]-propionyl]-piperidin-4-yloxy]-essigsäureethylester, MS (ISP): 519 $(M+H)^+$.

Das Ausgangsmaterial kann wie folgt hergestellt werden:

a) Eine Lösung von 1 g D-Milchsäure-tert-butylester und 0,95 ml Triethylamin in 10 ml Dichlormethan wird bei 0 °C mit 1,93 g Trifluormethansulfonsäureanhydrid versetzt, 1 h bei 0 °C gerührt, nochmals mit 0,95 ml Triethylamin und mit 1,43 g O-tert-Butyl-dimethylsilyl-aminoethanol versetzt. Nach 2 h Rühren bei Raumtemperatur wird das Reaktionsgemisch eingeengt und der Rückstand an Kieselgel (Hexan/Essigester 2:1) chromatographiert. Man erhält 990 mg (S)-2-[2-(tert-Butyl-dimethyl-silanyloxy)-ethylamino]-propionsäure-tert-butylester, MS (EI): 246 (M-57).

b) In Analogie zu Beispiel 175 a) werden 5 g 4-Cyanosalicylsäure mit 4,36 g der Vorstufe umgesetzt und der Eindampfrückstand an Kieselgel (Hexan/tert-Butylmethylether 2:1) chromatographiert. Man erhält 1,19 g (S)-2-[2-(tert-Butyl-dimethyl-silanyloxy)-ethyl]-(4-cyano-2-hydroxy-benzoyl)-amino]-propionsäure-tert-butylester, MS (EI): 449 $(M+H)^+$.

c) Eine Lösung von 1,19 g der Vorstufe in 25 ml Ether wird mit 2,7 ml Tetrabutylammoniumfluorid (1 M in Tetrahydrofuran) versetzt, 16 h bei Raumtemperatur gerührt, mit 1M $H_3PO_4$-Lösung und ges. NaCl-Lösung gewaschen, getrocknet und eingeengt. Chromatographie des Rückstands an Kieselgel (Essigester) ergibt 704 mg (S)-2-[(4-Cyano-2-hydroxy-benzoyl)-(2-hydroxy-ethyl)-amino]-propionsäure-tert-butylester, MS (ISP): 335 $(M+H)^+$.

d) Die Umsetzung von 669 mg der Vorstufe in Analogie zu Beispiel 175 b) ergibt nach Chromatographie an Kieselgel (Hexan/Essigester 2:1) 345 mg (S)-2-(8-Cyano-5-oxo-2,3,4,5-tetrahydro-1,4-benzoxazepin-4-yl)-propionsäure--tert-butylester, MS (EI): 260 (M-56).

e) In Analogie zu Beispiel 175 c) werden 340 mg der Vorstufe mit 243 mg 4-Piperidinoxyessigsäureethylester umgesetzt. Nach Chromatographie des Rückstands an Kieselgel (Hexan/Essigester 1:3) erhält man 365 mg (S)-[1-[2-(8-Cyano-5-oxo-2,3,4,5-tetrahydro-1,4-benzoxazepin-4-yl)-propionyl]-piperidin-4-yloxy]-essigsäureethylester, $[\alpha]_D^{20}$ = - 18,8° (c = 0,6, Chloroform).

Beispiel 177

Die Reaktion von 80 mg (R,S)-4-[2-[4-(Amino-imino-methyl)-2-methoxy-benzoylamino]-propionyl]-phenoxy-essigsäureethylester-hydrojodid mit 22 mg Chlorameisensäureethylester nach Beispiel 176 B) ergibt nach Chromatographie an Kieselgel (Hexan/Essigester 1:3) 58 mg (R,S)-[4-[2-[4-(Amino-ethoxycarbonylimino-methyl)-2-methoxy-benzoylamino]-propionyl]-phenoxy]-essigsäureethylester als farbloses Öl, MS (ISP): 500 $(M+H)^+$.

Das Ausgangsmaterial kann wie folgt hergestellt werden:

a) Eine Lösung von 1,92 g 4-Cyanosalicylsäure und 1,49 g N-Hydroxysuccinimid in 30 ml Dichlormethan und 30 ml Dimethylformamid wird bei 0 °C mit 2,43 g Dicyclohexylcarbodiimid versetzt und während 16 h bei - 14 °C bis 15 °C gerührt. Der Niederschlag wird abgenutscht und die den 4-Cyanosalicylsäure-hydroxysuccinimidester enthaltende Mutterlauge direkt in die nächste Stufe eingesetzt.

b) Die Abspaltung der BOC-Gruppe an 4,1 g (S)-4-(2-tert-Butoxycarbonylamino-propionyl)-phenoxyessigsäureethylester (Beispiel 23 a) erfolgt mit 1,75 ml Trimethylsilyljodid bei 0 °C in 40 ml Dichlormethan. Nach Zugabe von 8 ml 4N HCl in Dioxan wird die Reaktionslösung eingeengt, der Rückstand in 40 ml Tetrahydrofuran gelöst und zusammen mit 2,6 ml 4-Methylmorpholin zu der in der Vorstufe erhaltenen, den 4-Cyanosalicylsäure-hydroxysuccinimidester enthaltenden Mutterlauge zugegeben. Nach 16 h bei Raumtemperatur wird das Gemisch eingeengt und der Rückstand an Kieselgel chromatographiert (Hexan/Essigester 3:1). Man erhält 2,98 g (S)-[4-[2-(4-Cyano-2-hydroxy-benzoylamino)-propionyl]-phenoxy]-essigsäure-ethylester, MS (ISP): 397 $(M+H)^+$.

c) Die Alkylierung von 792 mg der Vorstufe mit 0,19 ml Methyljodid in Gegenwart von 830 mg Kaliumcarbonat in 20 ml Dimethylformamid während 2 h bei Raumtemperatur ergibt nach Einengen der Lösung und Chromatographie des Rückstands an Kieselgel (Hexa/Essigester 3:1) 580 mg (R,S)-[4-[2-(4-Cyano-2-methoxy-benzoylamino)-propionyl]-phenoxy]-essigsäureethylester, MS (ISP): 411 $(M+H)^+$.

d) In Analogie zu Beispiel 31 werden 290 mg der Vorstufe mit Schwefelwasserstoff, Methyljodid und Ammoniumacetat umgesetzt, die Reaktionslösung auf die Hälfte eingeengt und das Produkt durch Zugabe von Ether gefällt. Man erhält 183 mg (R,S)-[4-[2-[4-(Amino-imino-methyl)-2-methoxy-benzoylamino]-propionyl]-phenoxy]-essigsäureethylester-hydrojodid, MS (ISP): 428 $(M+H)^+$.

Beispiel 178

Die Reaktion von 1,4 g (R,S)-[4-[2-(4-Cyano-phenyl)-1-methyl-2-oxo-ethylcarbamoyl]-phenoxy]-essigsäureethylester mit Schwefelwasserstoff, Methyljodid und Ammoniumacetat in Analogie zu Beispiel 31 ergibt nach Chromatographie des Eindampfrückstands an silyliertem Kieselgel RP18 (Wasser/Tetrahydrofuran-Gradient) 410 mg (R,S)-[4-[2-[4-(Amino-imino-methyl)-phenyl]-1-methyl-2-oxo-ethylcarbamoyl]-phenoxy]-essigsäureethylester-hydrojodid, MS (ISP): 398 $(M+H)^+$.

Das Ausgangsmaterial kann wie folgt hergestellt werden:

a) Eine Lösung von 10,92 g 4-Brombenzonitril in 80 ml Tetrahydrofuran wird bei -78 °C zu 37,5 ml 1,6H n-Butyllithium zugetropft. Die entstandene Suspension wird 30 min. bei -78 °C gerührt, bei derselben Temperatur zu 4,65 g (S)-2-tert-Butoxycarbonylamino-N-methoxy-N-methyl-propionamid in 80 ml Tetrahydrofuran zugegeben und 1 h bei -78 °C gerührt. Die rote Reaktionslösung wird zu 1M $H_3PO_4$ gegossen. Extraktion mit Ether, Waschen der organischen Phasen mit ges. NaCl-Lösung, Trocknen und Einengen der Lösung ergibt einen Rückstand, der nach Chromatographie an Kieselgel zu 3,68 g (S)-2-(4-Cyano-phenyl)-1-methyl-2-oxo-ethylcarbaminsäure-tert-butylester führt, $[\alpha]_D^{20}$ = - 5,7 ° (c = 1, Chloroform).

b) Die Spaltung der BOC-Gruppe an 1,92 g der Vorstufe erfolgt analog Beispiel 177 b). Das entstandene Amin hydrochlorid wird bei 0 °C zu 1,89 g 4-tert-Butyl-dimethyl-silanyloxy-benzoylchlorid in 20 ml Pyridin zugegeben und 16 h bei Raumtemperatur gerührt. Einengen der Lösung und Chromatographie des Rückstands an Kieselgel (Hexan/Essigester 2:1) ergibt 1,84 g (S)-4-(tert-Butyl-dimethyl-silanyloxy)-N-[2-(4-cyano-phenyl)-1-methyl-2-oxo-ethyl]-benzamid,$[\alpha]_D^{20}$ = + 45,9 ° (c = 0,7, Chloroform).

c) Entschützen von 1,81 g der Vorstufe ergibt 1,4 g (R,S)-N-[2-(4-Cyano-phenyl)-1-methyl-2-oxo-ethyl-4-hydroxy-benzamid, MS (ISP): 293 $(M-H)^+$.

d) Die Alkylierung von 1,4 g der Vorstufe mit 0,65 ml Bromessigsäureethylester in Gegenwart von $K_2CO_3$ in Dimethylformamid während 2 h bei Raumtemperatur ergibt nach Entfernen des Lösungsmittels und Chromatographie an Kieselgel (Hexan/Essigester 1:1) 1,23 g (R,S)-[4-[2-(4-Cyano-phenyl)-1-methyl-2-oxo-ethylcarbamoyl]-phenoxy]-essigsäureethylester, MS (ISP): 381 $(M+H)^+$.

Beispiel 179

Eine Lösung von 50 mg (R,S)-[4-[2-[4-[Amino-(tert-butyl-dimethyl-silanyloxyimino)-methyl]-phenyl]-1-methyl-2-oxo-ethylcarbamoyl]-phenoxy]-essigsäureethylester in 1 ml Ether wird mit 0,1 ml Tetrabutylammoniumfluorid (1M in Tetrahydrofuran) 1 h bei 0 °C gerührt, eingeengt und der Rückstand an Kieselgel chromatographiert (Hexan/Essigester 1:2). Man erhält 11 mg (R,S)-[4-[2-[4-(Amino-hydroxyimino-methyl)-phenyl]-1-methyl-2-oxo-ethylcarbamoyl]-phenoxy]-essigsäureethylester, MS (ISP): 414 $(M+H)^+$.

Das Ausgangsmaterial kann wie folgt hergestellt werden:

In Analogie zu Beispiel 31 werden 0.6 g (R,S)-[4-[2-(4-Cyano-phenyl)-1-methyl-2-oxo-ethylcarbamoyl]-phenoxy)-essigsäureethylester mit Schwefelwasserstoff und Methyljodid umgesetzt. Es werden 147 mg O-tert-Butyl-dimethyl-silyl-hydroxylamin mit dem entstandenen Methylthioimin in 6 ml Tetrahydrofuran 2 h bei Raumtemperatur gerührt. Chromatographie des Rückstands an Kieselgel (Hexan/Essigester 3:2) führt zu 50 mg (R,S)-[4-[2-[4-[Amino-(tert-butyl-dimethyl-silanyloxyimino)-methyl]-phenyl]-1-methyl-2-oxo-ethylcarbamoyl]-phenoxy]-essigsäureethylester, MS (ISP): 414 (M-113).

Beispiel 180

Aus 743 mg (S)-4-(2-Piperidin-4-yloxyacetylamino-propionyl)-phenoxy-essigsäureethylester-hydrochlorid in 20 ml Ethanol und 20 ml Acrylnitril erhält man nach 5 Tagen bei 80 °C nach Chromatographie des Eindampfrückstands an Kieselgel (Dichlormethan/Methanol 5%) 250 mg (S)-[4-[2-[1-(2-Cyano-ethyl)-piperidin-4-yloxyacetylamino]-propionyl]-phenoxy]-essigsäureethylester, MS (ISP): 446 $(M+H)^+$.

Eine Lösung von 250 mg (S)-[4-[2-[1-(2-Cyano-ethyl)-piperidin-4-yloxyacetylamino]-propionyl]-phenoxy]-essigsäureethylester in 10 ml Dichlormethan wird bei 0 °C mit 128 mg meta-Chlorperbenzoesäure versetzt und 1 h bei 0 °C gerührt. Die Reaktionslösung wird mit ges. $NaHCO_3$-Lösung und ges. NaCl-Lösung gewaschen, getrocknet und eingeengt. Chromatographie des Rückstands an Kieselgel ergibt 140 mg (S)-[4-[2-(1-Hydroxy-piperidin-4-yloxyacetylamino)-propionyl]-phenoxy]-essigsäureethylester, MS (ISP): 409 $(M+H)^+$.

Beispiel 181

Eine Lösung von 285 mg (S)-4-(2-Piperidin-4-yloxyacetylaminopropionyl)-phenoxyessigsäureethylester, 0,11 ml 4-Methylmorpholin und 217 mg Essigsäure-4-nitro-phenoxycarbonyloxymethylester wird 5 h bei Raumtemperatur gerührt, eingeengt und der Rückstand an Kieselgel (Hexan/Essigester 1:2) chromatographiert. Man erhält 205 mg (S)-4-[2-(4-Ethoxycarbonylmethoxy-phenyl)-1-methyl-2-oxo-ethylcarbamoylmethoxy]-piperidin-1-carbonsäure-acetoxymethylester, $[\alpha]_D^{20}$ = + 23.7 °(c = 0,35, Chloroform).

Beispiel 182

Die Abspaltung der BOC-Schutzgruppe an 3,45 g (S)-4-[1-tert-Butoxy-methyl-2-(4-ethoxycarbonylmethoxy-phenyl)-2-oxo-ethylcarbamoylmethoxy]-piperidin-1-carbonsäure-tert-butylester in 60 ml Dichlormethan und 30 ml Trifluoressigsäure ergibt nach Einengen des Lösungsmittels und Chromatographie des Rückstands an silyliertem Kieselgel RP18 (Wasser/Tetrahydrofuran-Gradient) 1,9 g (S)-[4-[3-Hydroxy-2-(2-piperidin-4-yloxy-acetylamino)-propionyl]-phenoxy]-essigsäureethylester-trifluoracetat, MS (ISP): 409 $(M+H)^+$.

Das Ausgangsmaterial kann wie folgt hergestellt werden:

a) In Analogie zu Beispiel 177 b) wird an 3,5 g (S)-4-[3-tert-Butoxy-2-tert-butoxycarbonylamino-propionyl]-phenoxyessigsäureethylester (Beispiel 7 d) die BOC-Gruppe gespalten und das entstandene Amin mit 2,57 g 1-tert-Butoxycarbonylpiperidin-4-yl-oxyessigsäure in Gegenwart von 2,95 g TPTU und 1,82 ml 4-Methylmorpholin in 60 ml Dichlormethan 16 h bei Raumtemperatur gerührt. Nach Entfernen des Lösungsmittels und Chromatographie des Rückstands an Kieselgel (Hexan/Essigester 1:2) erhält man 3,5 g (S)-4[1-tert-Butoxymethyl-2-(4-ethoxycarbonylmethoxy-phenyl)-2-oxo-ethylcarbamoylmethoxy]-piperidin-1-carbonsäure-tert-butylester als gelbes Öl, MS (ISP): 565 $(M+H)^+$.

Beispiel 183

Analog Beispiel 181 werden 480 mg (S)-[4-[3-Hydroxy-2-(2-piperidin-4-yloxy-acetylamino)-propionyl]-phenoxy]-essigsäureethylester mit 367 mg Essigsäure-4-nitro-phenoxycarbonyloxymethylester umgesetzt und der Rückstand an Kieselgel (Essigester) chromatographiert. Man erhält 161 mg (S)-[4-[2-[(1-Acetoxymethoxycarbonyl-piperidin-4-yloxy)-acetylamino]-3-hydroxy-propionyl]-phenoxy]-essigsäureethylester als farbloses Öl, MS (ISP): 525 $(M+H)^+$.

Beispiel 184

Eine Lösung von 800 mg (S)-4-[[2-(4-Ethoxycarbonylmethoxy-phenyl)-1-methyl-2-oxo-ethyl]-methyl-carbamoylmethoxy]-piperidin-1-carbonsäure-tert-butylester in 10 ml Dichlormethan und 5 ml Trifluoressigsäure wird 1 h bei Raumtemperatur gerührt und eingeengt. Chromatographie des Rückstands an silyliertem Kieselgel RP18 (Wasser/Tetrahydrofuran-Gradient) ergibt 509 mg (S)-4-[2-[Methyl-(piperidin-4-yloxy-acetyl)-amino]-propionyl]-phenoxy]-essigsäureethylester-trifluoracetat, MS (ISP): 407 $(M+H)^+$.

Das Ausgangsmaterial kann wie folgt hergestellt werden:

a) Die Kupplung von 5 g (S)-N-tert-Butoxycarbonyl-N-methyl-2-aminopropionsäure mit 2.88 g N,O-Dimethylhydroxylamin-hydrochlorid in 100 ml Dichlormethan in Gegenwart von 8,77 g TPTU und 5,96 ml 4-Methylmorpholin ergibt nach 16 h Rühren bei Raumtemperatur, Eingengen der Reaktionslösung und Chromatographie des Rückstands an Kieselgel (Hexan/Essigester 2:1)4,43 g (S)-[1-(Methoxy-methyl-carbamoyl)-ethyl]-methylcarbaminsaure-tert-butylester,$[\alpha]_D^{20}$ = - 62,3 ° (c = 1, Chloroform).

b) Nach der Umsetzung von 4,1 g der Vorstufe mit p-Brom-tert-butyl-dimethylsilylphenol analog Beispiel 3 a) und Chromatographie des Rückstands an Kieselgel (Hexan/Essigester 95:5) erhält man 2,82 g (S)-[2-(4-tert-Butyl-dimethyl-silanyloxy)-phenyl]-1-methyl-2-oxo-ethyl]-methylcarbaminsäure-tert-butylester als farbloses Öl, $[\alpha]_D^{20}$ = -127,0 ° (c = 0,5, Chloroform).

c) Entschützen von 2,2 g der Vorstufe analog Beispiel 3 b) ergibt 1,41 g (S)-[2-(4-Hydroxy-phenyl)-1-methyl-2-oxo-ethyl]-methylcarbaminsäure-tert-butylester,$[\alpha]_D^{20}$ = - 179,4 °(c = 0,7, Chloroform).

d) Die Alkylierung von 615 mg der Vorstufe analog Beispiel 3 c) und Chromatographie des Eindampfrückstands an Kieselgel (Hexan/ Essigester 3:1) ergibt 674 mg (S)-[4-[2-(tert-Butoxycarbonyl-methyl-amino)-propionyl]-phenoxy]-essigsäureethylester, $[\alpha]_D^{20}$ = - 135,0 ° (c = 0,5, Chloroform).

e) Die Abspaltung der BOC-Schutzgruppe an 810 mg der Vorstufe erfolgt analog Beispiel 177 b) bei -78 °C. Das entstandene Amin wird mit 498 mg 1-tert-Butexycarbonylpiperidin-4-yl-oxyessigsäure in Gegenwart von 570 mg TPTU und 0,39 ml 4-Methylmorpholin in 30 ml Dichlormethan 20 h bei Raumtemperatur gerührt. Nach Entfernen des Lösungsmittels und Chromatographie des Rückstands an Kieselgel erhält man 890 mg (S)-4-[[2-(4-Ethoxycarbonylmethoxy-phenyl)-1-methyl-2-oxoethyl]-methyl-carbamoylmethoxy]-piperidin-1-carbonsäure-tert-butylester als farbloses Öl, MS (ISP): 507 $(M+H)^+$.

Beispiel 185

Eine Lösung von 850 mg (S)-4-[2-[2-(4-Ethoxycarbonylmethoxy-phenyl)-1-methyl-2-oxo-ethylcarbamoyl]-ethyl]-piperidin-1-carbonsäure-tert-butylester in 5 ml Dichlormethan und 2,5 ml Trifluoressigsäure wird 1 h bei Raumtemperatur gerührt und eingeengt. Man erhält 760 mg (S)-[4-[2-(3-Piperidin-4-yl-propionylamino)-propionyl]-phenoxy]-essigsäureethylester-trifluoracetat als farbloses Öl, MS (ISP): 391 $(M+H)^+$.

Das Ausgangsmaterial kann wie folgt hergestellt werden:

Die Abspaltung der BOC-Schutzgruppe an 740 mg (S)-4-(2-tert-Butoxycarbonylamino-propionyl)-phenoxy-essigsäureethylester erfolgt analog zu Beispiel 177 b). Das entstandene Amin wird mit 500 mg 1-tert-Butoxycarbonylpiperidin-4yl-propionsäure in Gegenwart von 570 mg TPTU und 0,46 ml 4-Methylmorpholin in 10 ml Dichlormethan 16 h bei Raumtemperatur gerührt. Nach Entfernen des Lösungsmittels und Chromatographie des Rückstands an Kieselgel (Hexan/Essigester 2:3) erhält man 890 mg (S)-4-[2-[2-(4-Ethoxycarbonylmethoxy-phenyl)-1-methyl-2-oxo-ethylcarbamoyl]-ethyl]-piperidin-1-carbonsäure-tert-butylester als gelbes Öl, MS (ISP): 491 $(M+H)^+$.

Beispiel 186

Ausgehend von 300 mg (S)-[4-[2-(3-Piperidin-4-yl-propionylamino)-propionyl]-phenoxy]-essigsäureethylester-trifluoracetat erhält man analog Beispiel 181 nach Chromatographie an Kieselgel (Hexan/Essigester 1:2) 140 mg (S)-4-[2-[3-(1-Acetoxymethoxycarbonyl-piperidin-4-yl)-propionylamino]-propionyl]-phenoxy]-essigsäureethylester, MS (ISP): 507 $(M+H)^+$.

Beispiel 187

Eine Lösung von 100 mg (R)-3-[(S)-1-(4-Ethoxycarbonylmethoxy-benzoyl)-ethylcarbamoylmethoxy]-pyrrolidin-1-carbonsaure-tert-butylester in 2 ml Dichlormethan und 1 ml Trifluoressigsäure wird 2 h bei Raumtemperatur gerührt, eingeengt und der Rückstand an silyliertem Kieselgel RP18 (Wasser/Tetrahydrofuran-Gradient) chromatographiert. Man erhält 70 mg [4-[(S)-2-[(R)-2-Pyrrolidin-3-yloxy-acetylamino]-propionyl]-phenoxy]-essigsäureethylester-trifluoracetat, MS (ISP): 379 $(M+H)^+$.

Das Ausgangsmaterial kann wie folgt hergestellt werden:

a) Die Phasentransferreaktion von 4,1 g (R)-3-Hydroxy-pyrrolidin-1-carbonsäure-tert-butylester mit 3,85 ml Bromessigsäure-tert-butylester in 50 ml Toluol und 50 ml 50% NaOH in Gegenwart von 500 mg Tetrabutylammoniumhydrogensulfat ist nach 1 h beendet. Waschen der organischen Phase mit ges. NaCl-Lösung, Trocknen und Einengen ergibt einen Rückstand, der nach Chromatographie an Kieselgel (Hexan/ Essigester 3:1) zu 4,98 g (R)-3-tert-Butoxycarbonylmethoxy-pyrrolidin-1-carbonsäure-tert-butylester führt, MS (ISP): 302 $(M+H)^+$.

b) Eine Lösung von 4,95 g der Vorstufe in 50 mi Dichlormethan und 25 ml Trifluoressigsäure wird 3 h bei Raumtemperatur gerührt und eingeengt. Der Rückstand wird in 40 ml Dioxan und 40 ml 1N NaOH gelöst, bei Raumtemperatur mit 4,3 g Di-tert-butyldicarbonat in 40 ml Dioxan versetzt und 1,5 h gerührt. Das Reaktionsgemisch wird mit Ether verdünnt, die organische Phase mit 1N NaOH gewaschen und die wässerige Phase mit 3N HCl angesäuert. Extraktion der wässerigen Phase mit Ether, Waschen, Trocknen und Einengen der Etherphase ergibt 0,72 g (R)-3-Carboxymethoxy-pyrrolidin-1-carbonsäure-tert-butylester. Davon werden 360 mg mit nach Beispiel 177 b) entschütztem (S)-4-(2-tert-Butoxycarbonylamino-propionyl)-phenoxyessigsäureethylester in 15 ml Dichlormethan in Gegenwart von 446 mg TPTU und 0,33 ml 4-Methylmorpholin umgesetzt. Einengen der Reaktionslösung und Chromatographie des Rückstands ergibt 100 mg (R)-3-[(S)-1-(4-Ethoxycarbonylmethoxy-benzoyl)-ethylcarbamoylmethoxy]-pyrrolidin-1-carbonsäure-tert-butylester, MS (ISP): 479 $(M+H)^+$.

Beispiel 188

Eine Lösung von 290 mg (S)-6-[2-(4-Ethoxycarbonylmethoxy-phenyl)-1-methyl-2-oxo-ethylcarbamoyl]-1,2,3,4-tetrahydro-isochinolin-2-carbonsäure-tert-butylester in 4 ml Dichlormethan und 2 ml Trifluoressigsäure wird 1 h bei Raumtemperatur gerührt, eingeengt und der Rückstand an silyliertem Kieselgel RP18 (Wasser/Tetrahydrofuran-Gradient) chromatographiert. Man erhält 174 mg (S)-[4-[2-(1,2,3,4-Tetrahydro-isochinolin-6-ylcarbonylamino)-propionyl]-phenoxy]-essigesäureethylester-trifluoracetat,$[\alpha]_D^{20}$ = +51,7 °(c = 0,6, DMSO).

Das Ausgangsmaterial kann wie folgt hergestellt werden:

a) Zu einer Lösung von 1,46 g 6-Hydroxy-1,2,3,4-tetrahydro-isochinolin-hydrobromid in 10 ml Dioxan und 2 ml 1N NaOH werden bei 0 °C gleichzeitig 1,38 g Di-tert-butyldicarbonat in 10 ml Dioxan und 10 ml 1N NaOH zugegeben.

Nach 3 h Rühren bei Raumtemperatur wird das Reaktionsgemisch mit 1N HCl auf pH 5 gestellt, mit Ether extrahiert, die Etherphasen mit ges. NaCl-Lösung gewaschen, getrocknet und eigeengt. Chromatographie des Rückstands an Kieselgel ergibt 1,07 g 6-Hydroxy-1,2,3,4-tetrahydro-isochinolin-2-carbonsaure-tert-butylester, MS (EI): 192 (M-57).

b) Bei -15 °C wird eine Lösung von 918 mg der Vorstufe in 10 ml Dichlormethan mit 1,2 ml Triethylamin und anschliessend mit 0,7 ml Trifluormethansulfonsäureanhydrid versetzt und 2 h bei Raumtemperatur gerührt. Der Niederschlag wird abgenutscht, das Filtrat eingeengt und der Rückstand an Kieselgel (Hexan/Essigester 7:1) chromatographiert. Man erhält 1,02 g 6-Trifluormethylsulfonyloxy-1,2,3,4-tetrahydro-isochinolin-2-carbonsäure-tert-butylester, MS (EI): 324 (M-57).

c) Von der Vorstufe werden 950 mg mit CO in Gegenwart von 17 mg Palladiumacetat, 32 mg 1,3-Bis-(diphenylphosphino)-propan und 0,4 ml Triethylamin in 4 ml DMSO und 2,6 ml Methanol während 2 h bei 80 °C umgesetzt. Die Reaktionslösung wird mit ges. NaCl-Lösung verdünnt, mit Ether extrahiert, die Etherphasen mit ges. NaCl-Lösung gewaschen, getrocknet und eingeengt. Chromatographie des Rückstands an Kieselgel ergibt 443 mg 1,2,3,4-Tetrahydro-isochinolin-2,6-dicarbonsäure-2-tert-butylester-6-methylester, MS (ISP): 292 $(M+H)^+$.

d) Die Verseifung von 387 mg der Vorstufe mit 213 mg NaOH in 9 ml Methanol und 1 ml Wasser erfolgt während 6 h bei Raumtemperatur. Die Reaktionslösung wird konzentriert, mit Wasser verdünnt. Die wässerige Phase wird mit Ether extrahiert, mit 1N HCl auf pH 5 gestellt und der Niederschlag abgenutscht. Nach dem Waschen der Kristalle mit Wasser und Trocknen erhält man 274 mg 1,2,3,4-Tetrahydro-isochinolin-2,6-dicarbonsäure-2-tert-butylester, MS (EI): 220 (M-57).

e) Die Abspaltung der BOC-Schutzgruppe an 313 mg (S)-4-(2-tert-Butoxycarbonylamino-propionyl)-phenoxyessigsäureethylester analog Beispiel 177b) ergibt das Amin. Dieses wird mit 247 mg der Vorstufe, 264 mg TPTU und 0,20 ml 4-Methylmorpholin in 10 ml Dichlormethan während 48 h bei Raumtemperatur gerührt. Nach Chromatographie des Eindampfrückstands an Kieselgel (Hexan/Essigester 1:1) erhält man 444 mg (S)-6-[2-(4-Ethoxycarbonylmethoxy-phenyl)-1-methyl-2-oxo-ethylcarbamoyl]-1,2,3,4-tetrahydro-isochinolin-2-carbonsäure-tert-butylester, $[\alpha]_D^{20}$ = + 51,3 ° (c = 0,4, Chloroform).

Beispiel 189

Eine Lösung von 106 mg 1-[8-(Amino-imino-methyl)-5-oxo-2,3,4,5-tetrahydro-1,4-benzoxazepin-4-ylacetyl]-piperidin-4-yloxyessigsäuremethylesteracetat (Beispiel 175) und 18 mg Lithiumthydroxyd in 5 ml Methanol und 0,5 ml Wasser wird 16 h bei Raumtemperatur gerührt, mit 1N HCl auf pH 3 gestellt und eingeengt. Chromatographie des Rückstands an silyliertem Kieselgel RP18 (Wasser/Tetrahydrofuran-Gradient) ergibt 60 mg 1-[8-(Amino-imino-methyl)-5-oxo-2,3,4,5-tetrahydro-1,4-benzoxazepin-4-ylacetyl]-piperidin-4-yloxyessigsäure als weisse Kristalle, MS (ISP): 405 $(M+H)^+$.

Beispiel 190

Eine Lösung von 200 mg (R)-3-[(S)-1-(4-tert-Butoxycarbonylmethoxy-benzoyl)-ethylcarbamoylmethoxy]-pyrrolidin-1-carbonsäure-tert-butylester in 2 ml Dichlormethan und 1 ml Trifluoressigsäure wird 4 h bei Raumtemperatur gerührt, eingeengt und der Rückstand an silyliertem Kieselgel RP18 (Wasser/Tetrahydrofuran-Gradient) chromatographiert. Man erhält 113 mg [4-[(S)-2-[(R)-2-Pyrrolidin-3-yloxy-acetylamino]-propionyl]-phenoxy]-essigsäure-trifluoracetat, MS (ISP): 351 $(M+H)^+$.

Das Ausgangsmaterial kann wie folgt hergestellt werden:

Die Kupplung von 360 mg (R)-3-Carboxymethoxy-pyrrolidin-1-carbonsäure-tert-butylester (Beispiel 187 b) mit 569 mg nach Beispiel 177 b) entschütztem (S)-4-(2-tert-Butoxycarbonylamino-propionyl)-phenoxyessigsäure-tert-butylester in 15 ml Dichlormethan in Gegenwart von 446 mg TPTU und 0,33 ml 4-Methylmorpholin ergibt nach Einengen der Reaktionslösung und Chromatographie des Rückstands an Kieselgel (Hexan/Essigester 1:3) 200 mg (R)-3-[(S)-1-(4-tert-Butoxycarbonylmethoxy-benzoyl)-ethylcarbamoylmethoxy]-pyrrolidin-1-carbonsäure-tert-butylester, MS (ISP): 507 $(M+H)^+$.

Beispiel 191

In Analogie zu Beispiel 185 werden 231 mg (S)-4-[[2-(4-tert-Butoxycarbonylmethoxy-phenyl)-1-methyl-2-oxo-ethyl]-methyl-carbamoylmethoxy]-piperidin-1-carbonsäure-tert-butylester entschützt und an silyliertem Kieselgel

RP18 (Wasser/Tetrahydrofuran-Gradient) chromatographiert. Man erhält 113 mg (S)-4-[2-[Methyl-(piperidin-4-yloxy-acetyl)-amino]-propionyl]-phenoxy]-essigsäure-trifluoracetat, MS (ISP): 379 $(M+H)^+$.

Das Ausgangsmaterial kann wie folgt hergestellt werden:

a) Die Alkylierung von 723 mg (S)-[2-(4-Hydroxy-phenyl)-1-methyl-2-oxo-ethyl]-methylcarbaminsäure-tert-butylester analog Beispiel 3 c) und Chromatographie des Eindampfrückstands an Kieselgel (Hexan/ Essigester 5:1) ergibt 990 mg (S)-[4-[2-(tert-Butoxycarbonyl-methyl-amino)-propionyl]-phenoxy]-essigsäure-tert-butylester, $[\alpha]_D^{20}$ = - 124,5 ° (c = 0,4, Chloroform).

b) Die Abspaltung der BOC-Schutzgruppe an 394 mg der Vorstufe erfolgt analog Beispiel 177 b) bei -78 °C. Das entstandene Amin wird mit 311 mg 1-tert-Butoxycarbonylpiperidin-4-yl-oxyessigsäure in Gegenwart von 356 mg TPTU und 222 mg 4-Methylmorpholin in 45 ml Dichlormethan 20 h bei Raumtemperatur gerührt. Nach Entfernen des Lösungsmittels und Chromatographie des Rückstands an Kieselgel (tert-Butylmethylether/Hexan 4:1) erhält man 179 mg (S)-4-[[2-(4-tert-Butoxycarbonylmethoxy-phenyl)-1-methyl-2-oxo-ethyl]-methyl-carbamoylmethoxy]-piperidin-1-carbonsäure-tert-butylester als hellgelbes Öl, $[\alpha]_D^{20}$ = - 120,7° (c = 0,3, Chloroform).

Beispiel A

Eine Verbindung der Formel I kann man in an sich bekannter Weise als Wirkstoff zur Herstellung von Tabletten folgender Zusammensetzung verwenden:

| | pro Tablette |
|---|---|
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

Beispiel B

Eine Verbindung der Formel I kann man in an sich bekannter Weise als Wirkstoff zur Herstellung von Kapseln folgender Zusammensetzung verwenden:

| | pro Kapsel |
|---|---|
| Wirkstoff | 100,0 mg |
| Maisstärke | 20,0 mg |
| Milchzucker | 95,0 mg |
| Talk | 4,5 mg |
| Magnesiumstearat | 0,5 mg |
| | 220,0 mg |

**Patentansprüche**

**1.** Essigsäurederivate der Formel

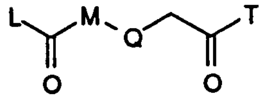

I

worin

L eine Gruppe der Formeln $L^1$ bis $L^5$:

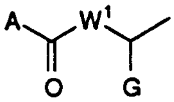

$L^1$

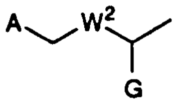

$L^2$

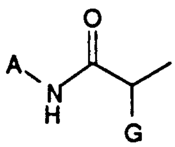

$L^3$

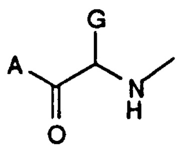

$L^4$

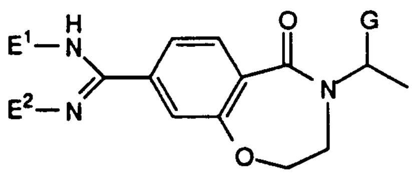

$L^5$

wobei eine in der Gruppe L und/oder zwischen den Gruppen L und M enthaltene nicht amidartig gebundene Carbonylgruppe auch als Oxim vorliegen kann,

A eine Gruppe der Formeln $A^1$ bis $A^4$:

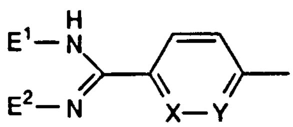

$A^1$

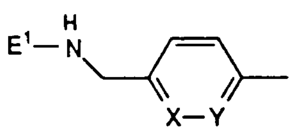

$A^2$

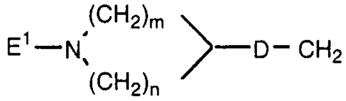

$A^3$

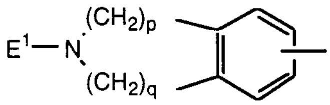

$A^4$

| | |
|---|---|
| $E^1$ und $E^2$ | H, nieder-Alkyl, OH, nieder-Alkoxy, nieder-Alkoxy-nieder alkyl, Carboxy-niederalkyl, P(O)(O-nieder-Alkyl)$_2$, C(O)OR$^1$, OC(O)R$^1$, OC(O)OR$^1$ oder C(O)SR$^1$, wobei zumindest eins von $E^1$ und $E^2$ H ist oder |
| $E^1$ und $E^2$ | zusammen mit den N-Atomen, an die sie gebunden sind, eine (5,5-Dimethyl- oder 5-Oxo)-4,5-dihydro-1,2,4-oxadiazol-3-ylgruppe sind, |
| $R^1$ | nieder-Alkoxy-niederalkyl, nieder-Alkyl, durch OH, COOH, nieder-Alkoxycarbonyl, nieder-Alkanoyloxy, nieder-Alkenoyl oxy, durch gegebenenfalls (durch nieder-Alkanoyloxy) substituiertes Benzoyloxy oder durch nieder-Alkyl-CONH substituiertes nieder-Alkyl, oder gegebenenfalls (durch Halogen oder niederAlkoxy) substituiertes und gegebenenfalls über nieder-Alkylen gebundenes Phenyl, oder gegebenenfalls durch O unterbrochenes Cycloalkyl, |
| eins von X und Y | CH und das andere CH, C-nieder-Alkyl, C-nieder-Alkoxy oder N, |
| D | eine Gruppe $(CH_2)_s$ oder $(CH_2)_tO$, |
| s | 1 bis 4, |
| m und n | 0 bis 5 und |
| t | 0 bis 3, jedoch m + n 1 bis 5 und jedes von m + t und n + t mindestens 1, |
| p und q | 0 bis 5, jedoch p + q 2 bis 5, |
| $W^1$ | $CH_2$, nieder Alkyl-CH, nieder-Alkyl-OC(O)CH, NH, nieder-Alkyl-N oder nieder-Alkoxy-nieder-alkyl-N, |
| $W^2$ | O, NH, Acyl-N oder nieder Alkyl-OC(O)-N, |
| G | H oder gegebenenfalls durch OH, SH, $CONH_2$, CONH-nieder-Alkyl, nieder-Alkylthio, Aryl, $NH_2$, NH-$R^a$, N($R^a$,$R^b$) oder O$R^a$ substitutiertes nieder-Alkyl, wobei $R^a$ und $R^b$ nieder-Alkyl, nieder Alkoxy-niederalkyl, Acyl oder nieder-Alkoxycarbonyl sind, |
| M | über das N-Atom an die Ketogruppe gebundenes 1,4-Piperidinylen oder gegebenenfalls durch nieder-Alkyl, nieder-Alkoxy, $OCH_2COOH$ oder $OCH_2COO$-nieder-Alkyl substituiertes 1,4-Phenylen, |
| Q | O, $CH_2$, NH, Acyl-N oder nieder-Alkyl OC(O)N ist |
| T | $NH_2$, NH-nieder-alkyl, , NH-nieder-Alkyl-COOH, NH-nieder-Alkyl-COO-niederalkyl, durch nieder-Alkoxy, COOH, COO-nieder-Alkyl, nieder-Alkyl-COO oder nieder-Alkyl-OCOO substituiertes nieder-Alkyloxy oder nieder-Alkenyloxy oder eine Gruppe OT', |
| T' | H, nieder-Alkyl, gegebenenfalls über nieder-Alkylen gebundenes Phenyl oder Pyridyl oder |

gegebenenfalls über nieder-Alkylen gebundenes und gegebenenfalls durch O, NH oder NCOO-nieder-Alkyl unterbrochenes Cycloalkyl ist, mit den Massgaben, dass

a)

| | |
|---|---|
| T' | von H, nieder-Alkyl und Phenyl-niederalkyl verschieden ist, falls |
| L | eine Gruppe der Formel |

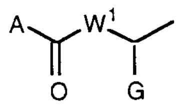

$L^1$

| | |
|---|---|
| A | eine Gruppe der Formel |

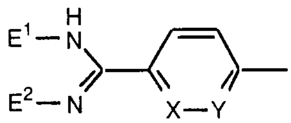

$A^1$

| | |
|---|---|
| eins von $E^1$ und $E^2$ | Wasserstoff und das andere Wasserstoff, tert-Butoxycarbonyl oder Benzyloxycarbonyl, |
| eins von X und Y | CH und das andere CH oder N und |
| $W^1$ | NH, nieder-Alkyl-N oder nieder-Alkoxy-niederalkyl-N ist, |
| G | die obige Bedeutung hat, |
| M | über das N-Atom an die Ketogruppe gebundenes 1,4-Piperidinylen, und |
| Q | O ist und dass |

b)

| | |
|---|---|
| T' | von H, nieder-Alkyl, Phenyl und Phenyl-niederalkyl, verschieden ist, falls |
| L | eine Gruppe der Formel $L^{11}$, $L^{31}$ oder $L^{41}$: |

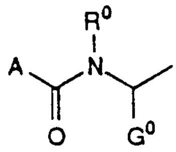

$L^{11}$

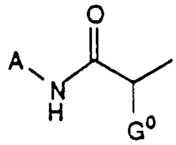

$L^{31}$

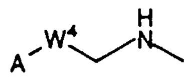

$L^{41}$

| | |
|---|---|
| A | eine Gruppe der Formel $A^1$ |

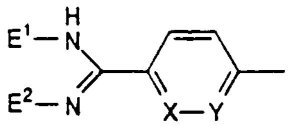

$A^1$

| | |
|---|---|
| eins von $E^1$ und $E^2$ | Wasserstoff und das andere Wasserstoff, tert-Butoxycarbonyl oder Benzyloxycarbonyl, |
| eins von X und Y | CH und das andere CH, C-nieder-Alkyl, C-nieder-Alkoxy oder N, |
| $R^o$ und $G^o$ | H oder nieder-Alkyl, |
| $W^4$ | C=O oder C=NOH, |
| M | gegebenenfalls durch nieder-Alkyl, nieder-Alkoxy, $OCH_2COOH$ oder $OCH_2COO$-nieder-Alkyl substituiertes 1,4-Phenylen und |
| Q | O, $CH_2$ oder NH ist, |
| c) | A nur dann eine Gruppe der Formel $A^3$ sein kann, wenn L eine Gruppe der Formel $L^1$ und M 1,4-Phenylen ist, und |
| d) | G nicht Wasserstoff ist, wenn L eine Gruppe der Formel $L^1$ und A eine Gruppe der Formel $A^3$ oder $A^4$ ist, |

sowie Hydrate oder Solvate und physiologisch verwendbare Salze davon, wobei "nieder" geradkettige oder verzweigte Gruppen mit 1 bis 6 C-Atom bezeichnet.

**2.** Verbindungen nach Anspruch 1, worin L eine Gruppe der Formel

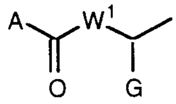

$L^1$

A eine Gruppe $A^1$, $A^2$ oder $A^{30}$:

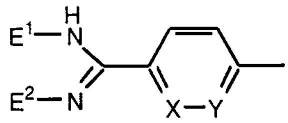

$A^1$

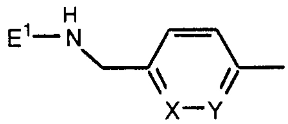

$A^2$

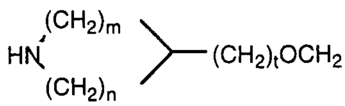

$A^{30}$

| | |
|---|---|
| eins von $E^1$ und $E^2$ | H und das andere H, nieder-Alkyl, OH, nieder-Alkoxy, nieder-Alkoxy-niederalkyl, Carboxy-niederalkyl, $PO(O\text{-nieder-Alkyl})_2$, $C(O)OR^1$ oder $OC(O)OR^1$, |
| $R^1$ | nieder-Alkoxy-niederalkyl, nieder-Alkyl, durch OH, COOH oder nieder-Alkanoyloxy substituiertes nieder-Alkyl, oder gegebenenfalls substituiertes und gegebenenfalls über nieder-Alkylen gebundenes Phenyl, oder gegebenenfalls durch O unterbrochenes Cycloalkyl, |
| eins von X und Y | CH und das andere CH oder N, |
| m und n | 0 bis 5 und t 0 bis 3, jedoch m + n 1 bis 5 und jedes von m + t und n + t mindestens 1, |
| $W^1$ | $CH_2$, nieder-Alkyl-OCOCH, NH, nieder-Alkyl-N oder nieder-Alkoxy-niederalkyl-N, |
| G und M | die gleiche Bedeutung wie im Anspruch 1 haben, |
| Q | Sauerstoff, |
| T | eine Gruppe OT" und |
| T" | H, nieder-Alkyl, nieder-Alkoxy-niederalkyl oder gegebenenfalls über nieder-Alkylen gebundenes und gegebenenfalls durch O unterbrochenes Cycloalkyl ist, mit den Massgaben, dass |
| a) | |
| T" | von H, nieder-Alkyl und Phenyl-niederalkyl verschieden ist, falls |
| A | eine Gruppe der Formel |

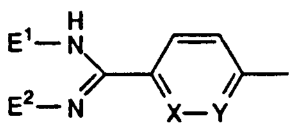

$A^1$

| | |
|---|---|
| $E^1$ und $E^2$ | Wasserstoff, tert-Butoxycarbonyl oder Benzyloxycarbonyl sind, |
| X, Y, G und Q | die obige Bedeutung haben, |
| $W^1$ | NH, nieder-Alkyl-N oder nieder-Alkoxy-niederalkyl-N und |
| M | über das N-Atom an die Ketogruppe gebundenes 1,4-Piperidinylen ist und dass |

b)

| | |
|---|---|
| T" | von H, nieder-Alkyl, Phenyl und Phenyl-niederalkyl, verschieden ist, falls |
| L | eine Gruppe der Formel $L^{11}$: |

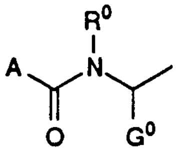

$L^{11}$

| | |
|---|---|
| A | eine Gruppe der Formel |

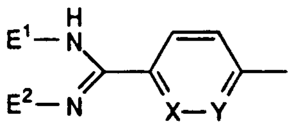

$A^1$

| | |
|---|---|
| $E^1$ und $E^2$ | Wasserstoff, tert-Butoxycarbonyl oder Benzyloxycarbonyl sind, |
| X, Y und Q | die obige Bedeutung haben, |
| $R^o$ und $G^o$ | H oder nieder-Alkyl und |
| M | gegebenenfalls durch nieder-Alkyl, nieder-Alkoxy, $OCH_2COOH$ oder $OCH_2COO$-nieder-Alkyl substituiertes 1,4-Phenylen ist, |
| c) | A nur dann eine Gruppe der Formel $A^{30}$ sein kann, wenn M 1,4-Phenylen ist, |

und physiologisch verwendbare Salze davon.

**3.** Essigsäurederivate nach Anspruch 1, worin L eine Gruppe $L^1$, in der A eine Gruppe $A^1$ ist, nämlich der Formel

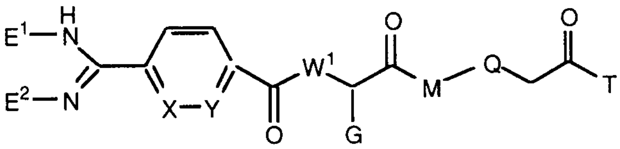

I-A

worin $E^1$, $E^2$, X, Y, $W^1$, G, M, Q und T die gleiche Bedeutung wie in Anspruch 1 haben.

**4.** Essigsäurederivate nach Anspruch 1 oder 3, worin eins von $E^1$ und $E^2$ H und das andere H, OH, $C(O)OR^1$ oder $OC(O)OR^1$ ist.

**5.** Essigsäurederivate nach Anspruch 4, worin $R^1$ nieder-Alkyl, wie Aethyl, Butyl oder Isobutyl, nieder-Alkoxy-nieder-alkyl, wie Methoxyäthyl, durch Benzoyloxy oder nieder-Alkanoyloxy substituiertes nieder-Alkyl, wie Benzoyloxymethyl, Acetoxymethyl, Acetoxyäthyl oder Pivaloyloxymethyl, oder Phenyl ist.

**6.** Essigsäurederivate nach Anspruch 1, 3, 4 oder 5, worin eins von X und Y CH und das andere CH oder N und/oder worin $W^1$ NH oder $CH_2$ und/oder worin Q O oder $CH_2$ ist.

**7.** Essigsäurederivate nach einem der Ansprüche 1 und 3-6, worin G H, nieder-Alkyl, wie Methyl oder Aethyl, oder nieder-Alkoxycarbonylaminoniederalkyl, wie Aethoxycarbonylaminopropyl ist.

**8.** Essigsäurederivate nach einem der Ansprüche 1 und 3-7, worin M über das N-Atom an die Ketogruppe gebundenes 1,4-Piperidinylen, 1,4-Phenylen oder durch $OCH_2COO$-nieder-Alkyl, wie Methoxycarbonylmethoxy, substituiertes 1,4-Phenylen ist.

**9.** Essigsäurederivate nach einem der Ansprüche 1 und 3-8, worin T nieder-Alkoxy, wie Methoxy, Aethoxy, Isopropoxy, Isobutoxy, tert-Butoxy oder Hexyloxy, nieder-Alkoxy-niederalkoxy, wie Methoxyäthoxy, durch COO-nieder-Alkyl substituiertes nieder-Alkenyloxy, wie 2-Isobutoxycarbonyl-2-pentenyloxy, durch nieder-Alkyl-COO substituiertes nieder-Alkoxy, wie Pivaloyloxymethoxy, durch nieder-Alkyl-OCOO substituiertes nieder-Alkoxy, wie 1-Isopropoxycarbonyloxy-äthoxy, gegebenenfalls durch O unterbrochenes Cycloalkyloxy, wie Tetrahydropyranyloxy, über nieder-Alkylenoxy gebundenes Pyridyl, wie 3- oder 4-Pyridylmethoxy, oder über nieder-Alkylenoxy gebundenes und gegebenenfalls durch NCOO-nieder-Alkyl unterbrochenes Cycloalkyl, wie 1-tert-Butoxycarbonyl-3 oder 4-piperidylmethoxy.

**10.** Essigsäurederivate nach einem der Ansprüche 1-9 aus der Gruppe der folgenden:

(S)-4-[2-[4-[Imino-2-(methoxy-ethoxycarbonylamino)-methyl]-benzoylamino]-propionyl]-phenoxyessigsäure-ethylester,
(Z)-(R,S)-4-[2-[4-[Amino-hydroxyimino-methyl]-benzoylamino]-propionyl]-phenoxyessigsäureethylester,
(S)-4-[2-[4-(Ethoxycarbonylamino-imino-methyl)-benzoylamino]-propionyl]-phenoxyessigsäure-tetrahydropyran-4-yl-ester,
(Z)-(R,S)-4-[2-[4-[Amino-ethoxycarbonyloximino-methyl]-benzoylamino]-propionyl]-phenoxyessigsäureethylester,
(S)-4-[2-[4-(Imino-phenoxycarbonylamino-methyl)-benzoylamino]-propionyl]-phenoxyessigsäureethylester,
(S)-4-[2-[4-[Imino-(2-methoxy-ethoxy-carbonylamino)-methyl]-benzoylamino]-propionyl]-phenoxyessigsäure-2-methoxy-ethylester,
(Z)-(S)-4-[2-[4-(Amino-hydroxyimino-methyl)-benzoylamino]-propionyl]-phenoxyessigsäureethylester,
(E/Z)-(S)-1-[2-[4-(Amino-ethoxycarbonylimino-methyl)-benzoylamino]-propionyl]-piperidin-4-yloxyessigsäure-isopropylester,
(E/Z)-(S)-1-[2-[4-(Amino-hydroxyimino-methyl)-benzoylamino]-propionyl]-piperidin-4-yloxyessigsäure-isopropylester,
[1-[4-[4-(Ethoxycarbonylamino-imino-methyl)-phenyl]-4-oxo-butyryl]-piperidin-4-yloxy]-essigsäureisopropylester,
(RS)-[1-[4-[4-(Isobutoxycarbonylamino-imino-methyl)-phenyl]-2-methyl-4-oxo-butyryl]-piperidin-4-yloxy]-essigsäure-isopropylester und insbesondere
(E/Z)-(S)-1-[2-[4-(Amino-hydroxyimino-methyl)-benzoylamino)-propionyl]-piperidin-4-yloxyessigsäureethylester.

**11.** Essigsäurederivate nach einem der Ansprüche 1 und 3-9 aus der Gruppe der folgenden:

(Z)-(S)- [1-[2-[4-(Amino-hydroxyimino-methyl)-benzoylamino]-propion yl]-piperidin-4-yloxy]essigsäure-(R/S)-1-isopropoxycarbonyloxy-ethylester,
(R)-(E)/(Z)-[1-[4-[4-(Amino-hydroxyimino-methyl)-phenyl]-2-methyl-4-oxo-butyryl]-piperidin-4-yloxy] essigsäure-pyridin-3-ylmethyl-ester,
(R)-(E)/(Z)- [1-[4-[4-(Amino-hydroxyimino-methyl)-phenyl]-2-methyl-4-oxo-butyryl]-piperidin-4-yloxy]-essigsäure-pyridin-4-ylmethyl-ester,
(E)- oder (Z)-(RS)-3-[1-[(R)-4-[4-(amino-hydroxyimino-methyl)-phenyl]-2-methyl-4-oxo-butyryl]-piperidin-4-yloxyacetoxymethyl]-piperidin-1-carbonsäure-tert-butylester,
(R)-[1-[4-[4-(Benzoyloxymethoxycarbonylamino-imino-methyl)-phenyl]-2-methyl-4-oxo-butyryl]-piperidin-4-yloxy]-essigsäure-ethylester,
(R)-[1-[4-[4-(Imino-pivaloyloxymethoxy carbonylamino-methyl)-phenyl]-2-methyl-4-oxo-butyryl]-piperidin-4-yloxy] -essigsäure-ethylester,
(E)- oder (Z)-(R)-4-[1-[4-[4-(Amino-hydroxyimino-methyl)-phenyl]-2-methyl-4-oxo-butyryl]-piperidin-4-yloxyacetoxymethyl]-piperidin-1-carbonsäure-tert-butylester,
(S)-[4-[2-[4-[(2-Acetoxy-ethoxy-carbonylimino)-amino-methyl]-benzoyl-amino]-propionyl]-phenoxy]-essigsäure-ethylester.

**12.** Essigsäurederivate nach Anspruch 1, worin L eine Gruppe $L^1$, in der A eine Gruppe $A^3$ ist, nämlich der Formel

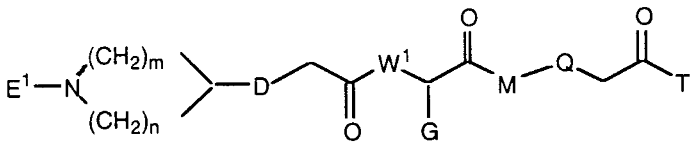

I-B

worin $E^1$, m, n, D, $W^1$, G, Q und T die gleiche Bedeutung wie in Anspruch 1 haben und M 1,4-Phenylen ist.

**13.** Essigsäurederivate nach Anspruch 1 oder 12, worin $E^1$ H, OH oder $C(O)OR^1$, m und n die Zahl 2 und/oder D und Q O und/oder $W^1$ NH und/oder G nieder-Alkyl, wie Methyl, und/oder T nieder-Alkoxy, wie Aethoxy, sind.

**14.** Essigsäurederivate nach Anspruch 13, worin $E^1$ nieder-Alkanoyloxyniederalkoxycarbonyl, wie Acetoxymethoxycarbonyl, ist.

**15.** Verbindung nach Anspruch 12, 13 oder 14,

(S)-4-[2-(4-Ethoxycarbonylmethoxy-phenyl)-1-methyl-2-oxo-ethylcarbamoylmethoxy]-piperidin-1-carbonsäure-acetoxymethylester.

**16.** Essigsäurederivate nach Anspruch 1, worin L eine Gruppe $L^1$, in der A eine Gruppe $A^2$ ist, nämlich der Formel

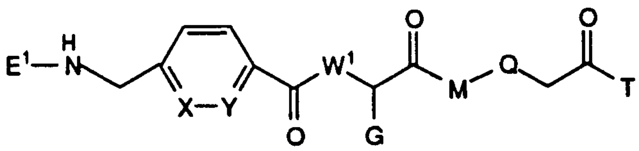

I-C

worin $E^1$, X, Y, $W^1$, G, M, Q und T die gleiche Bedeutung wie in Anspruch 1 haben, insbesondere worin Q O und T OH oder nieder-Alkoxy ist.

**17.** Essigsäurederivate nach Anspruch 1, worin L eine Gruppe $L^1$, in der A eine Gruppe $A^4$ ist, nämlich der Formel

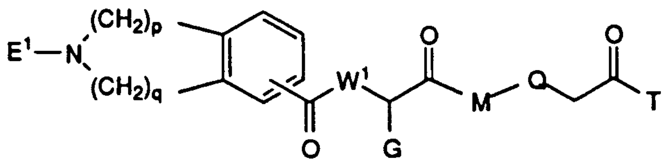

I-D

worin $E^1$, p, q, $W^1$, G, M, Q und T die gleiche Bedeutung wie in Anspruch 1 haben, insbesondere worin M 1,4-Phenylen, Q O und T nieder-Alkoxy ist.

**18.** Essigsäurederivate nach Anspruch 1, worin L eine Gruppe $L^2$, in der A eine Gruppe $A^1$ ist, nämlich der Formel

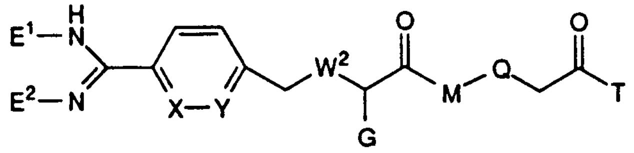

I-E

worin $E^1$, $E^2$, X, Y, $W^2$, G, M, Q und T die gleiche Bedeutung wie in Anspruch 1 haben, insbesondere worin M über das N-Atom an die Ketogruppe gebundenes 1,4-Piperidinylen, Q O und T nieder-Alkoxy ist.

**19.** Essigsäurederivate nach Anspruch 1, worin L eine Gruppe $L^3$, in der A eine Gruppe $A^1$ ist, nämlich der Formel

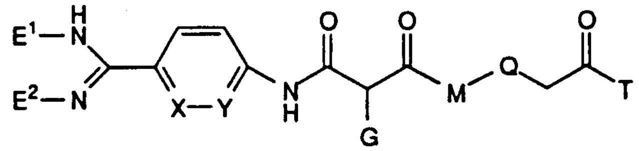

I-F

worin $E^1$, $E^2$, X, Y, G, M, Q und T die gleiche Bedeutung wie in Anspruch 1 haben, insbesondere worin M über das N-Atom an die Ketogruppe gebundenes 1,4-Piperidinylen, Q O und T nieder-Alkoxy ist.

**20.** Essigsäurederivate nach Anspruch 1, worin L eine Gruppe $L^4$, in der A eine Gruppe $A^1$ ist, nämlich der Formel

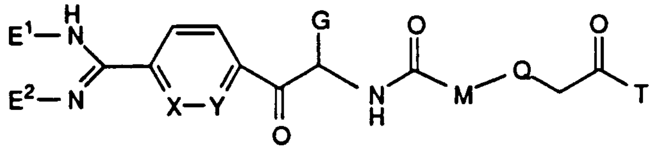

I-G

worin $E^1$, $E^2$, X, Y, G, M, Q und T die gleiche Bedeutung wie in Anspruch 1 haben, insbesondere worin M 1,4-Phenylen, Q O und T nieder-Alkoxy ist.

**21.** Essigsäurederivate nach Anspruch 1, worin L eine Gruppe $L^5$ ist, nämlich der Formel

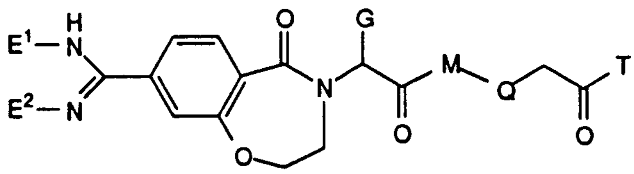

I-H

worin $E^1$, $E^2$, G, M, Q und T die gleiche Bedeutung wie in Anspruch 1 haben, insbesondere worin M über das N-Atom an die Ketogruppe gebundenes 1,4-Piperidinylen, Q O und T nieder-Alkoxy ist.

**22.** Verbindungen nach einem der Ansprüche 1-21 zur Verwendung als pharmazeutische Wirkstoffe.

**23.** Verfahren zur Herstellung der Verbindungen nach Anspruch 1, dadurch gekennzeichnet, dass man

a) in einer Verbindung der Formel II

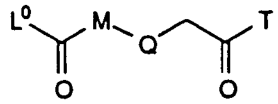

II

worin $L^o$ eine Gruppe einer der Formeln $L^{10}$ bis $L^{50}$

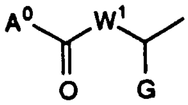

$L^{10}$

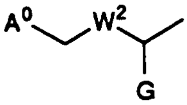

$L^{20}$

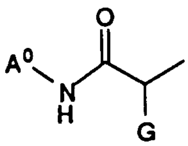

$L^{30}$

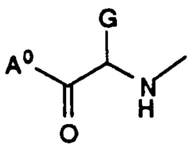

$L^{40}$

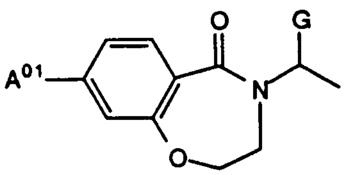

$L^{50}$

in der $A^o$ eine eine geschützte Amino- oder Amidinogruppe enthaltende Gruppe A ist und A, $W^1$, $W^2$, G, M, Q und T die gleiche Bedeutung wie in Anspruch 1 haben und $A^{01}$ eine geschützte Amidinogruppe ist, die geschützte Amino- oder Amidinogruppe spaltet, oder

b) in einer Verbindung der Formel III

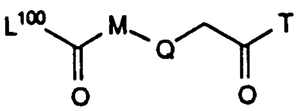

III

worin $L^{100}$ eine Gruppe einer der Formeln $L^{101}$ bis $L^{501}$

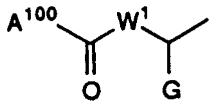

$L^{101}$

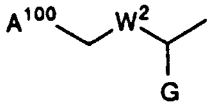

$L^{201}$

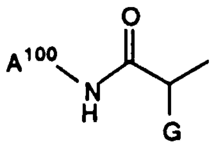

$L^{301}$

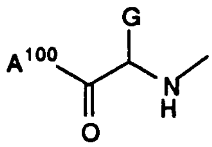

$L^{401}$

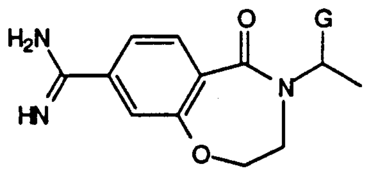

$L^{501}$

in der $A^{100}$ eine eine freie Amidinogruppe enthaltende Gruppe A ist und A, $W^1$, $W^2$, G, M, Q und T die gleiche Bedeutung wie in Anspruch 1 haben,
oder in einem Salz davon die freie Amidinogruppe in eine durch eine Gruppe $E^1$ oder $E^2$ substituierte Amidinogruppe umwandelt oder

c) in einer Verbindung der Formel IV

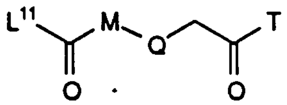

IV

worin $L^{11}$ eine Gruppe einer der Formeln $L^{111}$ bis $L^{115}$

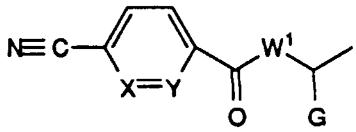

$L^{111}$

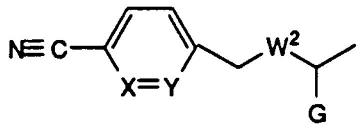

$L^{112}$

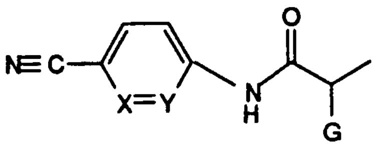

$L^{113}$

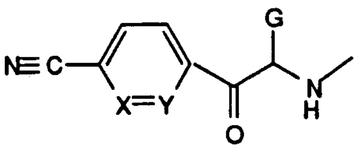

$L^{114}$

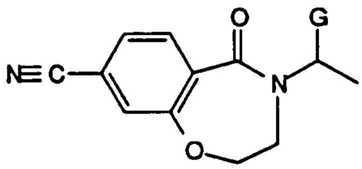

$L^{115}$

in der X, Y, $W^1$, $W^2$, G, M, Q und T die gleiche Bedeutung wie in Anspruch 1 haben, die in $L^{11}$ enthaltene Cyangruppe in eine gegebenenfalls durch $E^1$ oder $E^2$ substituierte Amidinogruppe umwandelt oder

d) ein Amin der Formel

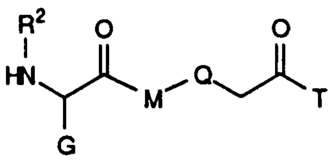

V

worin $R^2$ H, nieder-Alkyl oder nieder-Alkoxy-niederalkyl ist und G, M, Q und T die gleiche Bedeutung wie in Anspruch 1 haben,

mit einer Säure A-COOH oder einem funktionellen Derivat davon umsetzt, und

e) gewünschtenfalls eine in einer Verbindung der Formel I enthaltene reaktionsfähige Gruppe funktionell

abwandelt und

f) gewünschtenfalls eine Verbindung der Formel I in ein physiologisch verträgliches Salz oder ein Salz einer Verbindung der Formel I in die freie Säure oder Base überführt.

**24.** Pharmazeutische Präparate, insbesondere für die Behandlung oder Prophylaxe von Krankheiten, die durch die Bindung von adhäsiven Proteinen an Blutplättchen, sowie durch die Blutplättchenaggregation und die Zell-Zell-Adhäsion verursacht sind, enthaltend eine Verbindung nach einem der Ansprüche 1-21 als Wirkstoff.

**25.** Verwendung einer Verbindung nach einem der Ansprüche 1-21 zur Herstellung von Arzneimitteln zur Behandlung oder Prophylaxe von Krankheiten, die durch die Bindung von adhäsiven Proteinen an Blutplättchen, sowie durch die Blutplättchenaggregation und die Zell-Zell-Adhäsion verursacht sind, insbesondere zur Behandlung oder Prophylaxe von Blutplättchenthromben, Thrombose, Hirnschlag, Herzinfarkt, Entzündung, Arteriosklerose oder Osteoporose, oder als Antitumor-Mittel oder als Mittel für die Wundheilung.

**26.** Essigsäurederivat nach einem der Ansprüche 1-9,

(E/Z)-(S)-1-[2-[4-(Amino-hydroxyimino-methyl)-benzoylamino]-propionyl]-piperidin-4-yloxyessigsäure-ethylester.

## Claims

**1.** Acetic acid derivatives of the formula

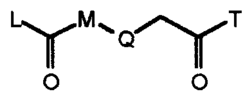

I

wherein

L is a group of formulae $L^1$ to $L^5$:

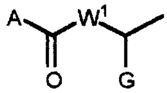

L[1]

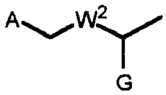

L[2]

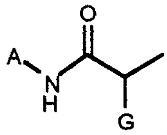

L[3]

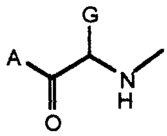

L[4]

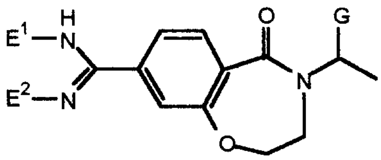

L[5]

in which a carbonyl group present in the L group and/or between the L and M groups which is not bonded in the form of an amide can also be present as an oxime,

A is a group of formulae $A^1$ to $A^4$:

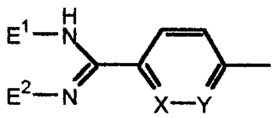

$A^1$

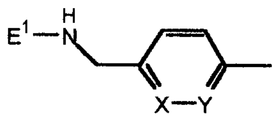

$A^2$

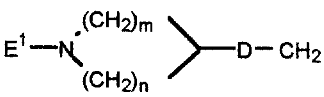

$A^3$

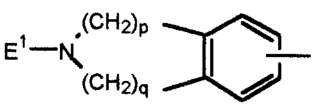

$A^4$

| | |
|---|---|
| $E^1$ and $E^2$ | are H, lower-alkyl, OH, lower-alkoxy, lower-alkoxy-lower-alkyl, carboxy-lower-alkyl, P(O)(O-lower-alkyl)$_2$, C(O)OR$^1$, OC(O)R$^1$, OC(O)OR$^1$ or C(O)SR$^1$, with at least one of $E^1$ and $E^2$ being H, or |
| E' and $E^2$ | together with the N atoms to which they are attached are a (5,5-dimethyl or 5-oxo)-4,5-dihydro-1,2,4-oxadiazol-3-yl group, |
| $R^1$ | is lower-alkoxy-lower-alkyl, lower-alkyl, lower-alkyl substituted by OH, COOH, lower-alkoxycarbonyl, lower-alkanoyloxy, lower-alkenoyloxy, benzoyloxy optionally substituted (by lower-alkanoyloxy) or lower-alkyl-CONH, or phenyl optionally substituted (by halogen or lower-alkoxy) and optionally bonded via lower-alkylene, or cycloalkyl optionally interupted by O, |
| one of X and Y | is CH and the other is CH, C-lower-alkyl, C-lower-alkoxy or N, |
| D | is a group (CH2)$_s$ or $(CH_2)_t$O, |
| s | is 1 to 4, |
| m and n | are 0 to 5 and |
| t | is 0 to 3, but m + n is 1 to 5 and each of m + t and n + t is at least 1, |
| p and q | are 0 to 5, but p + q is 2 to 5, |
| $W^1$ | is $CH_2$, lower alkyl-CH, lower-alkyl-OC(O)CH, NH, lower-alkyl-N or lower-alkoxy-lower-alkyl-N, |
| $W^2$ | is O, NH, acyl-N or lower-alkyl-OC(O)-N, |
| G | is H or lower-alkyl optionally substituted by OH, SH, $CONH_2$, CONH-lower-alkyl, lower-alkylthio, aryl, $NH_2$, NH-$R^a$, N($R^a$,$R^b$) or OR$^a$, with $R^a$ and $R^b$ being lower-alkyl, lower-alkoxy-lower-alkyl, acyl or lower-alkoxycarbonyl, |
| M | is 1,4-piperidinylene bonded via the N atom to the keto group or 1,4-phenylene optionally substituted by lower-alkyl, lower-alkoxy, $OCH_2COOH$ or $OCH_2COO$-lower-alkyl, |
| Q | is O, $CH_2$, NH, acyl-N or lower-alkyl OC(O)N, |
| T | is $NH_2$, NH-lower-alkyl, NH-lower-alkyl-COOH, NH-lower-alkyl-COO-lower-alkyl, lower-alkoxy or lower-alkenyloxy substituted by lower-alkoxy, COOH, COO-lower-alkyl, lower-alkyl-COO or lower-alkyl-OCOO, or a group OT', |
| T' | is H, lower-alkyl, phenyl or pyridyl optionally bonded via lower-alkylene or cycloalkyl optionally bonded via lower-alkylene and optionally interrupted by O, NH or NCOO-lower-alkyl, with the provisos that |
| a) | |
| T' | is different from H, lower-alkyl and phenyl-lower-alkyl where |
| L | is a group of the formula |

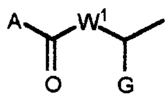

$L^1$

A is a group of the formula

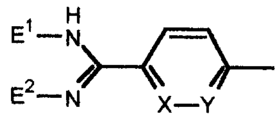

$A^1$

one of $E^1$ and $E^2$ is hydrogen and the other is hydrogen, tert-butoxycarbonyl or benzyloxycarbonyl,

one of X and Y is CH and the other is CH or N and

$W^1$ is NH, lower-alkyl-N or lower-alkoxy-lower-alkyl-N,

G has the above significance,

M is 1,4-piperidinylene bonded via the N atom to the keto group and

Q is O, and

b)

T' is different from H, lower-alkyl, phenyl and phenyl-lower-alkyl where

L is a group of formula $L^{11}$, $L^{31}$ or $L^{41}$;

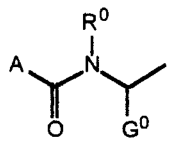

$L^{11}$

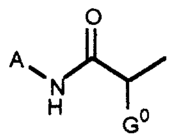

$L^{31}$

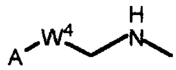

$L^{41}$

A is a group of formula $A^1$

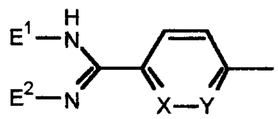

$A^1$

one of $E^1$ and $E^2$ is hydrogen and the other is hydrogen, tert-butoxycarbonyl or benzyloxycarbonyl,

| | |
|---|---|
| one of X and Y | is CH and the other is CH, C-lower-alkyl, C-lower-alkoxy or N, |
| $R^o$ and $G^o$ | are H or lower-alkyl, |
| $W^4$ | is C=O or C=NOH, |
| M | is 1,4-phenylene optionally substituted by lower-alkyl, lower-alkoxy, $OCH_2COOH$ or $OCH_2COO$-lower-alkyl and |
| Q | is O, $CH_2$ or NH, |

c)

| | |
|---|---|
| A | can only be a group of formula $A^3$ when L is a group of formula $L^1$ and M is 1,4-phenylene, and |

d)

| | |
|---|---|
| G | Is not hydrogen when L is a group of formula $L^1$ and A is a group of formula $A^3$ or $A^4$, |

as well as hydrates or solvates and physiologically usable salts thereof, whereby 'lower' denotes straight-chain or branched groups with 1 to 6 C atoms.

**2.** Compounds according to claim 1, wherein L is a group of the formula

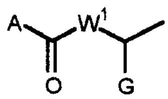

$L^1$

| | |
|---|---|
| A | is a group $A^1$ , $A^2$ or $A^{30}$: |

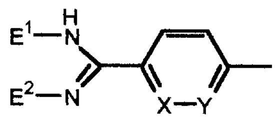

$A^1$

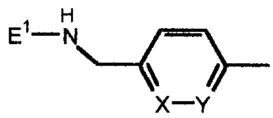

$A^2$

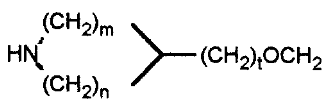

$A^{30}$

| | |
|---|---|
| one of $E^1$ and $E^2$ | is H and the other is H, lower-alkyl, OH, lower-alkoxy, lower-alkoxy-lower-alkyl, carboxy-lower-alkyl, PO(O-lower-alkyl)$_2$, C(O)OR$^1$ or OC(O)OR$^1$, |
| $R^1$ | is lower-alkoxy-lower-alkyl, lower-alkyl, lower-alkyl substituted by OH, COOH or lower-alkanoyloxy, or phenyl optionally substituted and optionally bonded via lower-alkylene, or cycloalkyl optionally interrupted by O, |

| | |
|---|---|
| one of X and Y | is CH and the other is CH or N, |
| m and n | are 0 to 5 and t is 0 to 3, but m + n is 1 to 5 and each of m + t and n + t is at least 1, |
| $W^1$ | is $CH_2$, lower-alkyl-OCOCH, NH, lower-alkyl-N or lower-alkoxy-lower-alkyl-N, |
| G and M | have the same significance as in claim 1, |
| Q | is oxygen, |
| T | is a group OT" and |
| T" | is H, lower-alkyl, lower-alkoxy-lower-alkyl or cycloalkyl optionally bonded via lower-alkylene and optionally interrupted by O, with the provisos that |
| a) | |
| T" | is different from H, lower-alkyl and phenyl-lower-alkyl where |
| A | is a group of the formula |

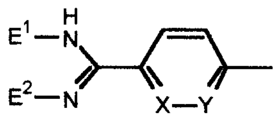

$A^1$

| | |
|---|---|
| $E^1$ and $E^2$ | are hydrogen, tert-butoxycarbonyl or benzyloxycarbonyl, |
| X, Y, G and Q | have the above significance, |
| $W^1$ | is NH, lower-alkyl-N or lower-alkoxy-lower-alkyl-N and |
| M | is 1,4-piperidinylene bonded via the N atom to the keto group, and |
| b) | |
| T" | is different from H, lower-alkyl, phenyl and phenyl-lower-alkyl where |
| L | is a group of formula $L^{11}$: |

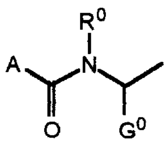

$L^{11}$

| | |
|---|---|
| A | is a group of the formula |

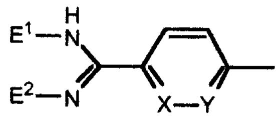

A1

| | |
|---|---|
| $E^1$ and $E^2$ | are hydrogen, tert-butoxycarbonyl or benzyloxycarbonyl, |
| X, Y and Q | have the above significance, |
| $R^o$ and $G^o$ | are H or lower-alkyl and |
| M | is 1,4-phenylene optionally substituted by lower-alkyl, lower-alkoxy, $OCH_2COOH$ or $OCH_2COO$-lower-alkyl, |

c)

| | |
|---|---|
| A | can only be a group of formula $A^{30}$ when M is 1,4-phenylene, |

and physiologically usable salts thereof.

**3.** Acetic acid derivatives according to claim 1, wherein L is a group $L^1$ in which A is a group $A^1$, namely of the formula

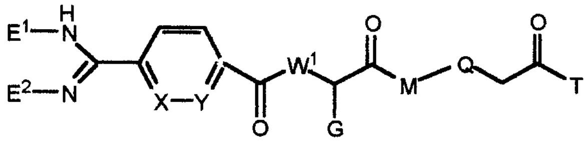

I-A

wherein $E^1$, $E^2$, X, Y, $W^1$, G, M, Q and T have the same significance as in claim 1.

**4.** Acetic acid derivatives according to claim 1 or 3, wherein one of $E^1$ and $E^2$ is H and the other is H, OH, C(O)OR$^1$ or OC(O)OR$^1$.

**5.** Acetic acid derivatives according to claim 4, wherein $R^1$ is lower-alkyl, such as ethyl, butyl or isobutyl, lower-alkoxy-lower-alkyl, such as methoxyethyl, lower-alkyl substituted by benzoyloxy or lower-alkanoyloxy, such as benzoyloxymethyl, acetoxymethyl, acetoxyethyl or pivaloyloxymethyl, or phenyl.

**6.** Acetic acid derivatives according to claim 1, 3, 4 or 5, wherein one of X and Y is CH and the other is CH or N and/or wherein $W^1$ is NH or $CH_2$ and/or wherein Q is O or $CH_2$.

**7.** Acetic acid derivatives according to any one of claims 1 and 3-6, wherein G is H, lower-alkyl, such as methyl or ethyl, or lower-alkoxycarbonylamino-lower-alkyl, such as ethoxycarbonylaminopropyl.

**8.** Acetic acid derivatives according to any one of claims 1 and 3-7, wherein M is 1,4-piperidinylene bonded via the N atom to the keto group, 1,4-phenylene or 1,4-phenylene substituted by $OCH_2COO$-lower-alkyl, such as methoxycarbonylmethoxy.

**9.** Acetic acid derivatives according to any one of claims 1 and 3-8, wherein T is lower-alkoxy, such as methoxy, ethoxy, isopropoxy, isobutoxy, tert-butoxy or hexyloxy, lower-alkoxy-lower-alkoxy, such as methoxyethoxy, lower-alkenyloxy substituted by COO-lower-alkyl, such as 2-isobutoxycarbonyl-2-pentenyloxy, lower-alkoxy substituted by lower-alkyl-COO, such as pivaloyloxymethoxy, lower-alkoxy substituted by lower-alkyl-OCOO, such as 1-isopropoxycarbonyloxy-ethoxy, cycloalkoxy optionally interrupted by O, such as tetrahydropyranyloxy, pyridyl bonded via lower-alkyleneoxy, such as 3- or 4-pyridylmethoxy, or cycloalkyl bonded via lower-alkyleneoxy and optionally interrupted by NCOO-lower-alkyl, such as 1-tert-butoxycarbonyl-3 or 4-piperidylmethoxy.

**10.** Acetic acid derivatives according to any one of claims 1-9 from the following group:

Ethyl (S)-4-[2-[4-[imino-2-(methoxy-ethoxycarbonylamino)-methyl]-benzoylamino]-propionyl]-phenoxyacetate,
ethyl (Z)-(R,S)-4-[2-[4-[amino-hydroxyimino-methyl]-benzoylamino]-propionyl]-phenoxyacetate,
tetrahydropyran-4-yl (S)-4- [2-[4-(ethoxycarbonylamino-imino-methyl)-benzoylamino]-propionyl]-phenoxyacetate,
ethyl (Z)-(R,S)-4-[2-[4-[amino-ethoxycarbonyloximino-methyl]-benzoylamino]-propionyl]-phenoxyacetate,
ethyl (S)-4-[2-[4-(imino-phenoxycarbonylamino-methyl)-benzoylamino]-propionyl]-phenoxyacetate,
2-methoxy-ethyl (S)-4-[2-[4-[imino-(2-methoxyethoxy-carbonylamino)-methyl]-benzoylamino]-propionyl]-phenoxyacetate,
ethyl (Z)-(S)-4-[2-[4-(amino-hydroxyimino-methyl)-benzoylamino]-propionyl]-phenoxyacetate,
isopropyl (E/Z)-(S)-1-[2-[4-(amino-ethoxycarbonylimino-methyl)-benzoylamino]-propionyl]-piperidin-4-yloxyacetate,
isopropyl (E/Z)-(S)-1-[2-[4-(amino-hydroxyimino-methyl)-benzoylamino]-propionyl]-piperidin-4-yloxyacetate,
isopropyl [1-[4-[4-(ethoxycarbonylamino-imino-methyl)-phenyl]-4-oxo-butyryl]-piperidin-4-yloxy]-acetate,
isopropyl (RS)-[1-[4-[4-(isobutoxycarbonylamino-imino-methyl)-phenyl]-2-methyl-4-oxo-butyryl]-piperidin-4-yloxy]-acetate and especially
ethyl (E/Z)-(S)-1-[2- [4-(amino-hydroxyimino-methyl)-benzoylamino]-propionyl]-piperidin-4-yloxyacetate.

**11.** Acetic acid derivatives according to any one of claims 1 and 3-9 from the following group:

(R/S)-1-Isopropoxycarbonyloxy-ethyl (Z)-(S)-[1-[2-[4-(amino-hydroxyimino-methyl)-benzoylamino]-propionyl]-piperidin-4-yloxy]acetate,
pyridin-3-ylmethyl (R)-(E)/(Z)-[1-[4-[4-(amino-hydroxyimino-methyl)-phenyl]-2-methyl-4-oxo-butyryl]-piperidin-4-yloxy]-acetate,
pyridin-4-ylmethyl (R)-(E)/(Z)-[1-[4-[4-(amino-hydroxyimino-methyl)-phenyl]-2-methyl-4-oxo-butyryl]-piperidin-4-yloxy]-acetate,
tert-butyl (E)- or (Z)-(RS)-3-[1-[(R)-4-[4-(amino-hydroxyimino-methyl)-phenyl]-2-methyl-4-oxo-butyryl]-piperidin-4-yloxyacetoxymethyl]-piperidine-1-carboxylate,
ethyl (R)-[1- [4-[4-(benzoyloxymethoxycarbonylamino-imino-methyl)-phenyl]-2-methyl-4-oxo-butyryl]-piperidin-4-yloxy]-acetate,
ethyl (R)-[1-[4-[4-(imino-pivaloyloxymethoxycarbonylamino-methyl)-phenyl]-2-methyl-4-oxo-butyryl]-piperidin-4-yloxy]-acetate,
tert-butyl (E)- or (Z)-(R)-4-[1-[4-[4-(amino-hydroxyimino-methyl)-phenyl]-2-methyl-4-oxo-butyryl]-piperidin-4-yloxyacetoxymethyl]-piperidine-1-carboxylate,
ethyl (S)- [4-[2-[4-[(2-acetoxy-ethoxy-carbonylimino)-amino-methyl]-benzoylamino]-propionyl]-phenoxy]-acetate.

**12.** Acetic acid derivatives according to claim 1, wherein L is a group $L^1$ in which A is a group $A^3$, namely of the formula

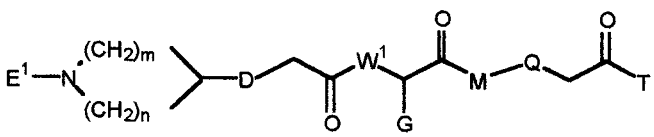

I-B

wherein $E^1$, m, n, D, $W^1$, G, Q and T have the same significance as in claim 1 and M is 1,4-phenylene.

**13.** Acetic acid derivatives according to claim 1 or 12, wherein $E^1$ is H, OH or $C(O)OR^1$, m and n are the number 2 and/or D and Q are O and/or $W^1$ is NH and/or G is lower-alkyl, such as methyl, and/or T is lower-alkoxy, such as ethoxy.

**14.** Acetic acid derivatives according to claim 13, wherein $E^1$ is lower-alkanoyloxy-lower-alkoxycarbonyl, such as acetoxymethoxycarbonyl.

**15.** The compound according to claim 12, 13 or 14, acetoxymethyl (S)-4-[2-(4-ethoxycarbonylmethoxy-phenyl)-1-methyl-2-oxo-ethylcarbamoylmethoxy]-piperidine-1-carboxylate.

**16.** Acetic acid derivatives according to claim 1, wherein L is a group $L^1$ in which A is a group $A^2$, namely of the formula

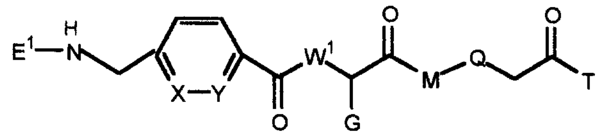

I-C

wherein $E^1$, X, Y, $W^1$, G, M, Q and T have the same significance as in claim 1, especially wherein Q is O and T is OH or lower-alkoxy.

**17.** Acetic acid derivatives according to claim 1, wherein L is a group $L^1$ in which A is a group $A^4$, namely of the formula

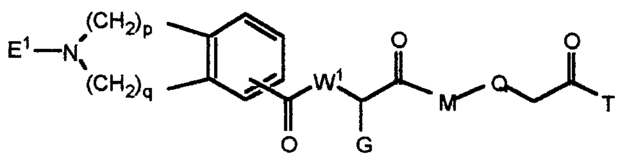

I-D

wherein $E^1$, p, q, $W^1$, G, M, Q and T have the same significance as in claim 1, especially wherein M is 1,4-phenylene, Q is O and T is lower-alkoxy.

**18.** Acetic acid derivatives according to claim 1, wherein L is a group $L^2$ in which A is a group $A^1$, namely of the formula

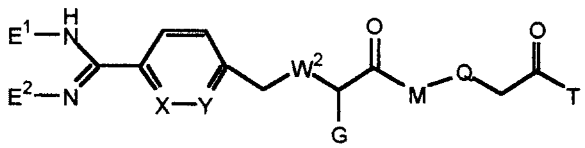

I-E

wherein $E^1$, $E^2$, X, Y, $W^2$, G, M, Q and T have the same significance as in claim 1, especially wherein M is 1,4-piperidinylene bonded via the N atom to the keto group, Q is O and T is lower-alkoxy.

**19.** Acetic acid derivatives according to claim 1, wherein L is a group $L^3$ in which A is a group $A^1$, namely of the formula

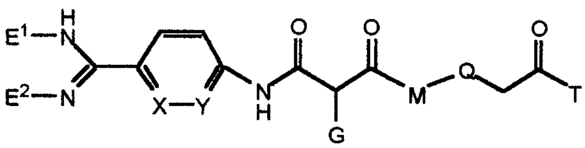

I-F

wherein $E^1$, $E^2$, X, Y, G, M, Q and T have the same significance as in claim 1, especially wherein M is 1,4-piperidinylene bonded via the N atom to the keto group, Q is O and T is lower-alkoxy.

**20.** Acetic acid derivatives according to claim 1, wherein L is a group $L^4$ in which A is a group $A^1$, namely of the formula

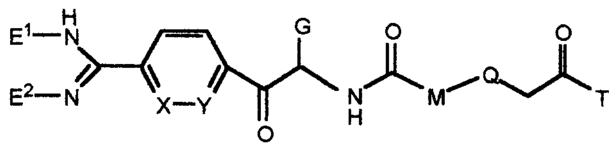

I-G

wherein $E^1$, $E^2$, X, Y, G, M, Q and T have the same significance as in claim 1, especially wherein M is 1,4-phenylene, Q is O and T is lower-alkoxy.

**21.** Acetic acid derivatives according to claim 1, wherein L is a group $L^5$, namely of the formula

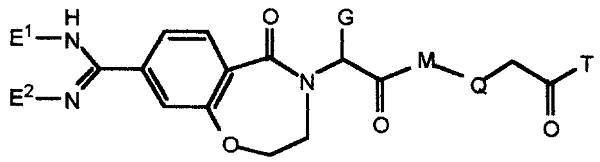

I-H

wherein $E^1$, $E^2$, G, M, Q and T have the same significance as in claim 1, especially wherein M is 1,4-piperidinylene bonded via the N atom to the keto group, Q is O and T is lower-alkoxy.

**22.** Compounds acccording to any one of claims 1-21 for use as pharmaceutically active substances.

**23.** A process for the manufacture of the compounds according to claim 1, characterized by

a) cleaving the protected amino or amidino group in a compound of the formula

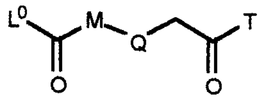

II

wherein $L^o$ is a group of one of formulae $L^{10}$ to $L^{50}$

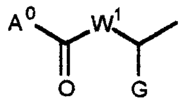

$L^{10}$

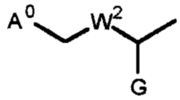

$L^{20}$

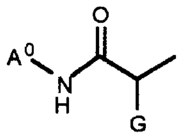

$L^{30}$

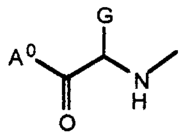

$L^{40}$

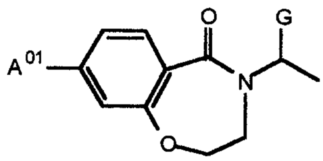

$L^{50}$

in which $A^o$ is a group A containing a protected amino or amidino group, and A, $W^1$, $W^2$, G, M, Q and T have the same significance as in claim 1 and $A^{01}$ is a protected amidino group,

or

b) converting the free amidino group in a compound of formula III

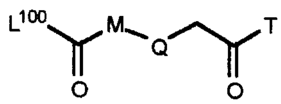

III

wherein $L^{100}$ is a group of one of formulae $L^{101}$ to $L^{501}$

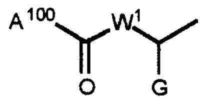

$L^{101}$

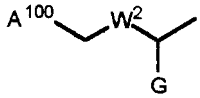

$L^{201}$

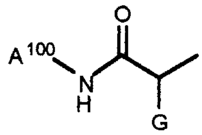

$L^{301}$

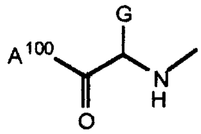

$L^{401}$

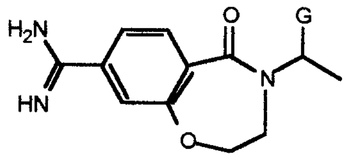

$L^{501}$

in which $A^{101}$ is a group A containing a free amidino group and A, $W^1$, $W^2$, G, M, Q and T have the same significance as in claim 1,
or in a salt thereof into an amidino group substituted by a group $E^1$ or $E^2$, or

c) converting the cyano group present in $L^{11}$ in a compound of formula IV

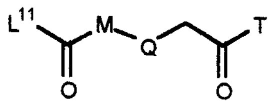

IV

wherein $L^{11}$ is a group of one of formulae $L^{111}$ to $L^{115}$

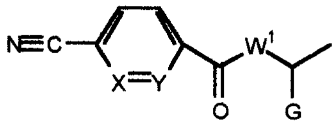

$L^{111}$

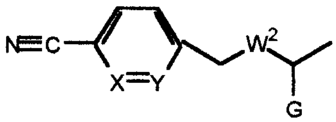

$L^{112}$

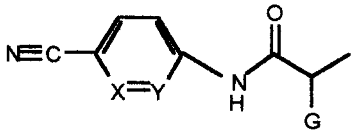

$L^{113}$

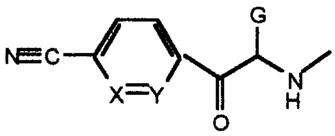

$L^{114}$

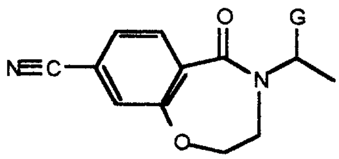

$L^{115}$

in which X, Y, $W^1$, $W^2$, G, M, Q and T have the same significance as in claim 1, into an amidino group optionally substituted by $E^1$ or $E^2$, or

d) reacting an amine of the formula

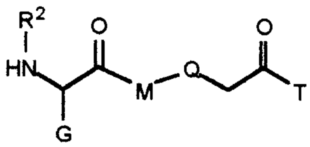

V

wherein $R^2$ is H, lower-alkyl or lower-alkoxy-lower-alkyl and G, M, Q and T have the same significance as in claim 1,

with an acid A-COOH or a functional derivative thereof, or

e) if desired, functionally modifying a reactive group present in a compound of formula I, and

f) if desired, converting a compound of formula I into a physiologically compatible salt or converting a salt of a compound of formula I into the free acid or base.

24. Pharmaceutical preparations, especially for the treatment or prophylaxis of illnesses which are caused by the binding of adhesive proteins to blood platelets as well as by blood platelet aggregation and cell-cell adhesion, containing a compound according to any one of claims 1-21 as the active substance.

25. The use of a compound according to any one of claims 1-21 for the production of medicaments for the treatment or prophylaxis of illnesses which are caused by the binding of adhesive proteins to blood platelets as well as by blood platelet aggregation and cell-cell adhesion, especially for the treatment or prophylaxis of blood platelet thrombi, thrombosis, stroke, cardiac infarct, inflammation, arteriosclerosis or osteoporosis, or as antitumour medicaments or as medicaments for wound healing.

26. The acetic acid derivatives according to any one of claims 1-9,

ethyl (E/Z)-(S)-1-[2-[4-(amino-hydroxyimino-methyl)-benzoylamino]-propionyl]-piperidin-4-yloxyacetate.

## Revendications

1. Dérivés de l'acide acétique de formule

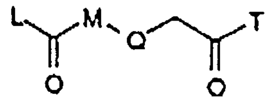

I

dans laquelle

L est un groupe de formules $L^1$ à $L^5$ :

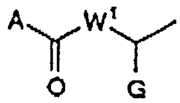

$L^1$

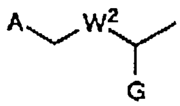

$L^2$

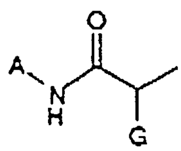

$L^3$

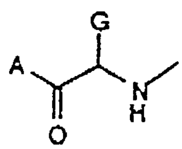

$L^4$

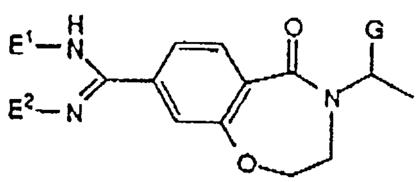

$L^5$

dans laquelle un groupe carbonyle contenu dans le groupe L et/ou entre les groupes L et M, et lié par une

liaison autre qu'amide, peut aussi se présenter sous forme d'une oxime,
A est un groupe de formules $A^1$ à $A^4$ :

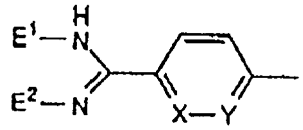

$A^1$

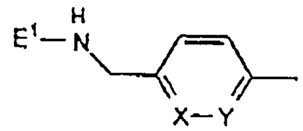

$A^2$

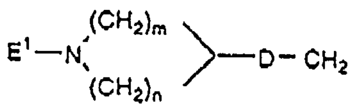

$A^3$

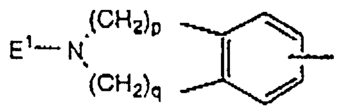

$A^4$

$E^1$ et $E^2$ sont des atomes d'hydrogène, des groupes alkyle inférieur, OH, alcoxy inférieur, (alcoxy inférieur)-(alkyle inférieur), carboxy(alkyle inférieur), P(O)(O-alkyle inférieur)$_2$, C(O)O$R^1$, OC(O)$R^1$, OC(O)O$R^1$ ou C(O)S$R^1$, au moins l'un des deux radicaux $E^1$ et $E^2$ étant H, ou
$E^1$ et $E^2$, avec les atomes d'azote auxquels ils sont liés, forment ensemble un groupe (5,5-diméthyl- ou 5-oxo-)-4,5-dihydro-1,2,4-oxadiazole-3-yle, et
$R^1$ est un groupe (alcoxy inférieur)(alkyle inférieur), alkyle inférieur, alkyle inférieur substitué par OH, COOH, (alcoxy inférieur)-carbonyle, alcanoyloxy inférieur, alcénoyloxy inférieur, un groupe alkyle inférieur substitué par un (alkyle inférieur)-CONH ou par un benzoyloxy éventuellement substitué par un alcanoyloxy inférieur, ou phényle éventuellement substitué par halogéno ou alcoxy inférieur et éventuellement lié par l'intermédiaire de groupe alkyle inférieur, ou cycloalkyle éventuellement interrompu par O,
l'un des radicaux X et Y est CH, et l'autre est CH ou un groupe C-alkyle inférieur, C-alcoxy inférieur, ou N,
D est un groupe $(CH_2)_s$ ou $(CH_2)_tO$,
s vaut de 1 à 4,
m et n valent de 0 à 5, et
t vaut de 0 à 3, mais m + n vaut de 1 à 5 et tant m + t que n + t valent au moins 1,
p et q valent de 0 à 5, mais p + q vaut de 2 à 5,
$W^1$ est $CH_2$, un groupe (alkyle inférieur)-CH, (alkyle inférieur)-OC(O)CH, NH, (alkyle inférieur)-N ou (alcoxy inférieur)(alkyle inférieur)-N,
$W^2$ est O, NH, un groupe acyl-N ou (alkyle inférieur)-OC(O)-N,
G est H ou un groupe alkyle inférieur éventuellement substitué par OH, SH, $CONH_2$, CONH-(alkyle inférieur), alkylthio inférieur, aryle, $NH_2$, NH-$R^a$, N($R^a$,$R^b$) ou O$R^a$, $R^a$ et $R^b$ étant des groupes alkyle inférieur, (alcoxy inférieur)(alkyle inférieur), acyle, ou (alcoxy inférieur)-carbonyle,
M est un groupe 1,4-pipéridinylène lié au groupe céto par l'intermédiaire de l'atome d'azote, ou un groupe 1,4-phénylène éventuellement substitué par alkyle inférieur, alcoxy inférieur, $OCH_2COOH$ ou $OCH_2COO$-(alkyle inférieur),
Q est O, $CH_2$, NH, acyl-N ou (alkyle inférieur)-OC(O)N,
T est $NH_2$, un groupe NH-(alkyle inférieur), NH-(alkyle inférieur)-COOH, NH-(alkyle inférieur)-COO-(alkyle inférieur), alkyloxy inférieur ou alcényloxy inférieur substitué alcoxy inférieur, COOH, COO-(alkyle inférieur), (alkyle inférieur)-COO ou (alkyle inférieur)-OCOO ; ou encore un groupe OT',
T' est H ou un groupe alkyle inférieur, phényle ou pyridyle éventuellement lié par l'intermédiaire d'un groupe alkylène inférieur, ou cycloalkyle éventuellement lié par l'intermédiaire d'un groupe alkylène inférieur et éventuellement interrompu par O, NH ou un groupe NCOO-(alkyle inférieur), à la condition que :
a)

T' soit différent de H, d'un groupe alkyle inférieur ou d'un groupe pnényl(alkyle inférieur), quand L est un groupe de formule

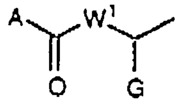

$L^1$

A est un groupe de formule

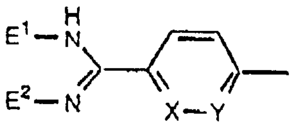

$A^1$

l'un des radicaux $E^1$ et $E^2$ est un atome d'hydrogène et l'autre est un atome d'hydrogène ou le groupe tert-butoxycarbonyle ou benzyloxycarbonyle,
l'un des radicaux X et Y est CH et l'autre est CH ou N, et
$W^1$ est NH, un groupe (alkyle inférieur)-N ou (alcoxy inférieur)-(alkyle inférieur)-N,
G a les significations données ci-dessus,
M est un groupe 1,4-pipéridinylène lié par l'intermédiaire d'un atome d'azote au groupe céto, et
Q est O,
et que
b)
T' soit différent de H ou d'un groupe alkyle inférieur, phényle ou phényl(alkyle inférieur), quand
L est un groupe de formule $L^{11}$, $L^{31}$ ou $L^{41}$ :

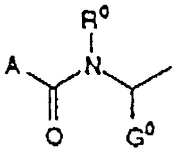

$L^{11}$

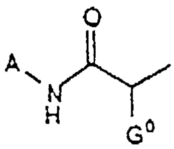

$L^{31}$

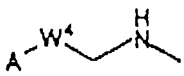

$L^{41}$

A est un groupe de formule $A^1$

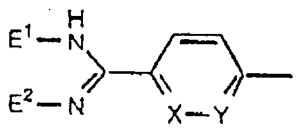

$A^1$

l'un des radicaux $E^1$ et $E^2$ est un atome d'hydrogène et l'autre est un atome d'hydrogène ou le groupe tert-butoxycarbonyle ou benzyloxycarbonyle,
l'un des radicaux X et Y est CH, et l'autre est CH ou un groupe C-(alkyle inférieur), C-(alcoxy inférieur) ou N,
$R^0$ et $G^0$ sont des atomes d'hydrogène ou des groupes alkyle inférieur,
$W^4$ est C=O ou C=NOH,
M est un groupe 1,4-phénylène éventuellement substitué par alkyle inférieur, alcoxy inférieur, $OCH_2COOH$, ou $OCH_2COO$-(alkyle inférieur), et
Q est O, $CH_2$ ou NH,
c)
que A ne puisse être un groupe de formule $A^3$ que quand L est un groupe de formule $L^1$ et M est le groupe 1,4-phénylène, et que
d)
G ne soit pas un atome d'hydrogène quand L est un groupe de formule L' et A est un groupe de formule $A^3$ ou $A^4$,

ainsi que leurs produits d'hydratation et de solvatation et leurs sels acceptables d'un point de vue physiologique, "inférieur" désignant des groupes à chaîne droite ou ramifée ayant de 1 à 6 atomes de carbone.

**2.** Composés selon la revendication 1, dans laquelle L est un groupe de formule

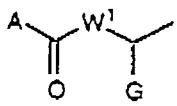

$L^1$

A est un groupe $A^1$, $A^2$ ou $A^{30}$ :

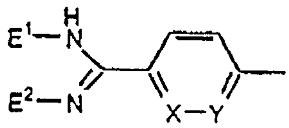

$A^1$

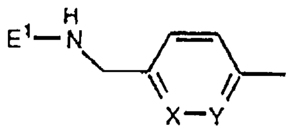

$A^2$

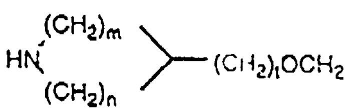

$A^{30}$

l'un des radicaux $E^1$ et $E^2$ est H et l'autre est H ou un groupe alkyle inférieur, OH, alcoxy inférieur, (alcoxy inférieur)(alkyle inférieur), carboxy(alkyle inférieur), PO(O-alkyle inférieur)$_2$, C(O)O$R^1$ ou OC(O)O$R^1$,

$R^1$ est un groupe (alcoxy inférieur)(alkyle inférieur), alkyle inférieur, alkyle inférieur substitué par OH, COOH ou alcanoyloxy inférieur, ou phényle éventuellement substitué et éventuellement lié par l'intermédiaire d'un groupe alkylène inférieur, ou cycloalkyle éventuellement interrompu par O,

l'un des radicaux X et Y est CH et l'autre est CH ou N,

m et n valent de 0 à 5 et t vaut de 0 à 3, mais m + n vaut de 1 à 5 et tant m + t que n + t vaut au moins 1,

$W^1$ est $CH_2$ ou un groupe (alkyle inférieur)-OCOCH, NH, (alkyle inférieur)-N ou (alcoxy inférieur)(alkyle inférieur)-N,

G et M ont les mêmes significations que dans la revendication 1,

Q est un atome d'oxygène,

T est un groupe OT", et

T" est H ou un groupe alkyle inférieur, (alcoxy infé-rieur)(alkyle inférieur), ou cycloalkyle éventuellement lié par l'intermédiaire d'un groupe alkylène inférieur et éventuellement interrompu par O, à la condition que

a)

T" soit différent de H ou d'un groupe alkyle inférieur ou phényl(alkyle inférieur), quand

A est un groupe de formule

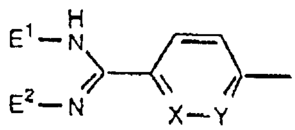

$A^1$

$E^1$ et $E^2$ sont des atomes d'hydrogène ou des groupes tert-butoxycarbonyle ou benzyloxycarbonyle,

X, Y, G et Q ont les significations données ci-dessus,

$W^1$ est NH ou un groupe (alkyle inférieur)-N ou (alcoxy inférieur)(alkyle inférieur)-N, et

M est un groupe 1,4-pipéridinylène lié par l'intermédiaire de l'atome d'azote au groupe céto, et que

b)

T" soit différent de H ou d'un groupe alkyle inférieur, phényle ou phényl(alkyle inférieur), quand

L est un groupe de formule $L^{11}$ :

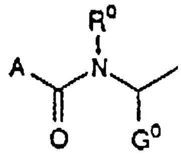

$L^{11}$

A est un groupe de formule

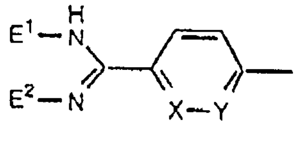

$A^1$

$E^1$ et $E^2$ sont des atomes d'hydrogène ou des groupes tert-butoxycarbonyle ou benzyloxycarbonyle,

X, Y et Q ont les significations données ci-dessus,

$R^0$ et $G^0$ sont des atomes d'hydrogène ou des groupes alkyle inférieur, et

M est un groupe 1,4-phénylène éventuellement substitué par des substituants alkyle inférieur, alcoxy inférieur, $OCH_2COOH$ ou $OCH_2COO$-(alkyle inférieur),

c)

que A ne puisse être un groupe de formule $A^{30}$ que quand M est un groupe 1,4-phénylène,

et leurs sels utilisables d'un point de vue physiologique.

**3.** Dérivés de l'acide acétique selon la revendication 1, dans lesquels L est un groupe $L^1$ dans lequel A est un groupe $A^1$, plus précisément de formule

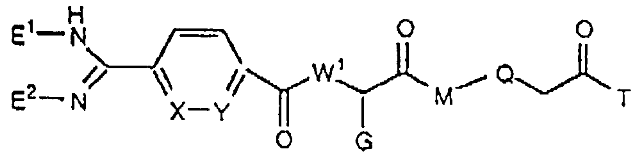

I-A

dans laquelle $E^1$, $E^2$, X, Y, $W^1$, G, M, Q et T ont les mêmes significations que dans la revendication 1.

**4.** Dérivés de l'acide acétique selon la revendication 1 ou 3, dans lesquels l'un des radicaux $E^1$ et $E^2$ est H et l'autre est H, OH, $C(O)OR^1$ ou $OC(O)OR^1$.

**5.** Dérivés de l'acide acétique selon la revendication 4, dans lesquels $R^1$ est un groupe alkyle inférieur tel qu'éthyle, butyle ou isobutyle, un groupe (alcoxy inférieur)(alkyle inférieur), tel que méthoxyéthyle, un groupe alkyle inférieur substitué par benzoyloxy ou alcanoyloxy inférieur, tel que benzoyloxyméthyle, acétoxyméthyle, acétoxyéthyle ou pivaloyloxy-méthyle, ou encore le groupe phényle.

**6.** Dérivés de l'acide acétique selon la revendication 1, 3, 4 ou 5, dans lesquels l'un des radicaux X et Y est CH et l'autre est CH ou N, et/ou dans lesquels $W^1$ est NH ou $CH_2$, et/ou dans lesquels Q est O ou $CH_2$.

**7.** Dérivés de l'acide acétique selon l'une des revendications 1 et 3 à 6, dans lesquels G est H ou un groupe alkyle inférieur tel que méthyle ou éthyle, ou un groupe (alcoxy inférieur)carbonylamino(alkyle inférieur), tel qu'éthoxycarbonylaminopropyle.

**8.** Dérivés de l'acide acétique selon l'une des revendications 1 et 3 à 7, dans lesquels M est un groupe 1,4-pipéridinylène, 1,4-phénylène ou 1,4-phénylène substitué par $OCH_2COO$-(alkyle inférieur), tel que le groupe méthoxycarbonylméthoxy, et lié au groupe céto par l'inter-médiaire de l'atome d'azote.

**9.** Dérivés de l'acide acétique selon l'une des revendications 1 et 3 à 8, dans lesquels T est un groupe alcoxy inférieur, tel que méthoxy, éthoxy, isopropoxy, isobutoxy, tert-butoxy ou hexyloxy, un groupe (alcoxy inférieur) (alcoxy inférieur), tel que méthoxyéthoxy, un groupe alcényloxy inférieur substitué par COO-alkyle inférieur, tel que 2-isobutoxycarbonyl-2-penténoyloxy, un groupe alcoxy inférieur substitué par (alkyle inférieur)-COO, tel que pivaloyloxyméthoxy, un groupe alcoxy inférieur substitué par (alkyle inférieur)-OCOO, tel que 1-isopropoxycarbonyloxyéthoxy, un groupe cycloalkyloxy éventuelle-ment interrompu par O, tel que tétrahydropyrranyloxy, un groupe pyridyle lié par l'intermédiaire d'un groupe alkylénoxy inférieur, tel que 3- ou 4-pyridylméthoxy, ou un groupe cycloalkyle lié par l'intermédiaire d'un groupe alkylénoxy inférieur et éventuellement interrompu par un groupe NCOO-(alkyle inférieur), tel que 1-tert-butoxycarbonyle-3 ou 4-pipéridylméthoxy.

**10.** Dérivés de l'acide acétique selon l'une des revendications 1 à 9, choisis dans le groupe des composés suivants :

Ester éthylique de l'acide (S)-4-[2-[4-[imino-2-(méthoxyéthoxycarbonylamino)-méthyl]-benzoylamino]-propionyl]-phénoxyacétique,
Ester éthylique de l'acide (Z)-(R,S)-4-[2-[4-[amino-hydroxyimino-méthyl]-benzoylamino]-propionyl]-phénoxyacétique,
Ester tétrahydropyranne-4-ylique de l'acide (S)-4-[2-[4-(éthoxycarbonylaminoiminométhyl]-benzoylamino]-propionyl]-phénoxyacétique,
Ester éthylique de l'acide (Z)-(R,S)-4-[2-[4-[aminoéthoxycarbonyl-oximinométhyl]-benzoylamino]-propionyl]-phénoxyacétique,

Ester éthylique de l'acide (S)-4-[2-[4-[iminophénoxycarbonylaminométhyl]-benzoylamino]-propionyl]-phénoxyacétique,

Ester 2-méthoxyéthylique de l'acide (S)-4-[2-[4-[imino-(2-méthoxyéthoxycarbonylamino)-méthyl]-benzoylamino]-propionyl]-phénoxyacétique,

Ester éthylique de l'acide (Z)-(S)-4-[2-[4-(aminohydroxyiminométhyl[-benzoylamino]-propionyl]-phénoxyacétique,

Ester isopropylique de l'acide (E/Z)-(S)-1-[2-[4-(aminoéthoxycarbonyliminométhyl]-benzoylamino]-propionyl]-pipéridine-4-yloxyacétique,

Ester isopropylique de l'acide (E/Z)-(S)-1-[2-[4-(aminohydroxyiminométhyl]-benzoylamino]-propionyl]-pipéridine-4-yloxyacétique,

Ester isopropylique de l'acide [1-[4-[4-(éthoxycarbonylaminoiminométhyl)-phényl]-4-oxobutyryl]-pipéridine-4-yloxy]-acétique,

Ester isopropylique de l'acide (RS)-[1-[4-[4-(isobutoxycarbonylaminoiminométhyl)-phényl]-2-méthyl-4-oxobutyryl]-pipéridine-4-yoxy]-acétique, et en particulier

Ester éthylique de l'acide (E/Z)-(S)-1-[2-[4-(aminohydroxyiminométhyl)-benzoylamino]-propionyl]-pipéridine-4-yloxyacétique

**11.** Dérivés de l'ester acétique selon l'une des revendications 1 et 3 à 9, choisis dans le groupe des composés suivants :

Ester (R/S)-1-isopropoxycarbonyloxyéthylique de l'acide (Z)-(S)-[1-[2-[4-(aminohydroxyiminométhyl)-benzoylamino]-propionyl]-pipéridine-4-yloxy]-acétique,

Ester pipéridine-3-ylméthylique de l'acide (R)-(E)/(Z)-[1-[4-[4-(aminohydroxyiminométhyl)-phényl]-2-méthyl-4-oxobutyryl]-pipéridine-4-yloxy]-acétique,

Ester pipéridine-4-ylméthylique de l'acide (R)-(E)/(Z)-[1-[4-[4-(aminohydroxyiminométhyl)-phényl]-2-méthyl-4-oxobutyryl]-pipéridine-4-yloxy]-acétique,

Ester tert-butylique de l'acide (E)- ou (Z)-(RS)-3-[1-[(R)-4-[4-(aminohydroxyiminométhyl)-phényl]-2-méthyl-4-oxobutyryl]-pipéridine-4-yloxy-acétoxyméthyl]-pipéridine-1-carboxylique,

Ester éthylique de l'acide (R)-[1-[4-[4-(benzoyloxyméthoxycarbonylaminoiminométhyl)-phényl]-2-méthyl-4-oxobutyryl]-pipéridine-4-yloxy]-acétique,

Ester éthylique de l'acide (R)-[1-[4-[4-(iminopivaloyloxyméthoxycarbonylaminométhyl)-phényl]-2-méthyl-4-oxobutyryl]-pipéridine-4-yloxy]-acétique,

Ester tert-butylique de l'acide (E)- ou (Z)-(R)-4-[1-[4-[4-(aminohydroxyiminométhyl)-phényl]-2-méthyl-4-oxobutyryl]-pipéridine-4-yloxyacétoxyméthyl]-pipéridine-1-carboxylique,

Ester éthylique de l'acide (S)-[4-[2-[4-[(2-acétoxyéthoxycarbonylamino)-aminométhyl]-benzoylamino]-propionyl]-phénoxy]-acétique.

**12.** Dérivés de l'acide acétique selon la revendication 1, dans lesquels L est un groupe $L^1$ dans lequel A est un groupe $A^3$, plus précisément de formule

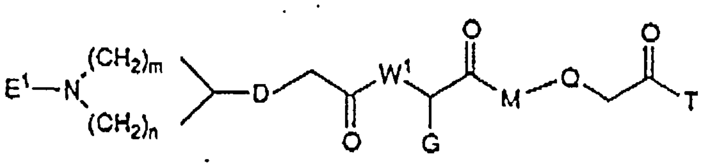

I-B

où $E^1$, m, n, D, $W^1$, G, M, Q et T ont les mêmes significations que dans la revendication 1.

**13.** Dérivés de l'acide acétique selon la revendication 1 ou 12, dans lesquels $E^1$ est H, OH ou $C(O)OR^1$, m et n valent chacun 2 et/ou D et Q sont O et/ou $W^1$ est NH et/ou G est un groupe alkyle inférieur tel que méthyle, et/ou M est un groupe 1,4-phénylène, et/ou T est un groupe alcoxy inférieur, tel qu'éthoxy.

**14.** Dérivés de l'acide acétique selon la revendication 13, dans lesquels $E^1$ est un groupe (alcanoyloxy inférieur)(alcoxy inférieur)carbonyle, tel qu'acétoxyméthoxycarbonyle.

**15.** Composé selon la revendication 12, 13 ou 14,

l'ester acétoxyméthylique de l'acide (S)-4-[2-(4-éthoxycarbonylméthoxyphényl)-1-méthyl-2-oxoéthylcarbamoylméthoxy]-pipéridine-1-carboxylique.

**16.** Dérivés de l'acide acétique selon la revendication 1, dans lesquels L est un groupe $L^1$ dans lequel A est un groupe $A^2$, plus précisément de formule

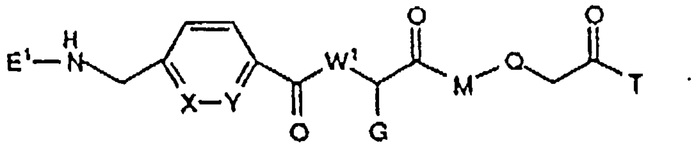

I-C

où $E^1$, X, Y, $W^1$, G, M, Q et T ont les mêmes signifcations que dans la revendication 1, en particulier où Q est O et T est OH ou un groupe alcoxy inférieur.

**17.** Dérivés de l'acide acétique selon la revendication 1, dans lesquels L est un groupe $L^1$, dans lequel A est un groupe $A^4$, plus précisément de formule

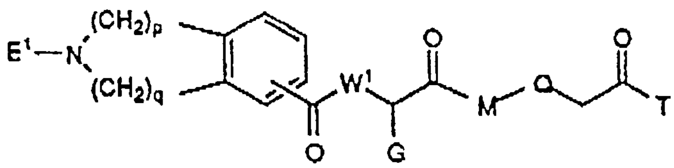

I-D

où $E^1$, p, q, $W^1$, G, M, Q et T ont les mêmes significations que dans la revendication 1, en particulier où M est un groupe 1,4-phénylène, Q est O et T est un groupe alcoxy inférieur.

**18.** Dérivés de l'acide acétique selon la revendication 1, dans lesquels L est un groupe $L^2$ dans lequel A est un groupe $A^1$, plus précisément de formule

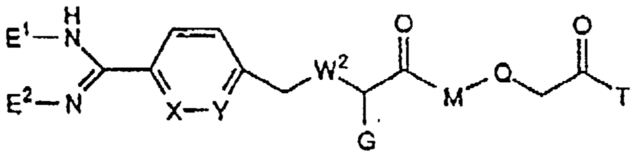

I-E

où $E^1$, $E^2$, X, Y, $W^2$, G, M, Q et T ont les mêmes significations que dans la revendication 1, en particulier où M est un groupe 1,4-pipéridinylène lié au groupe céto par l'intermédiaire de l'atome d'azote, Q est O et T est un groupe alcoxy inférieur.

**19.** Dérivés de l'acide acétique selon la revendication 1, dans lesquels L est un groupe $L^3$ dans lequel A est un groupe $A^1$, plus précisément de formule

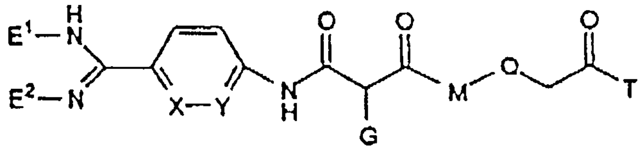

I·F

où $E^1$, $E^2$, X, Y, G, M, Q et T ont les mêmes significations que dans la revendication 1, en particulier où M est un groupe 1,4-pipéridinylène lié au groupe céto par l'intermédiaire de l'atome d'azote, Q est O et T est un groupe alcoxy inférieur.

**20.** Dérivés de l'acide acétique selon la revendication 1, dans lesquels L est un groupe $L^4$ dans lequel A est un groupe $A^1$, plus précisément de formule

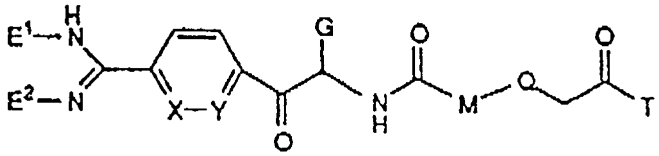

I·G

dans laquelle $E^1$, $E^2$, X, Y, G, M, Q et T ont les mêmes significations que dans la revendication 1, en particulier où M est un groupe 1,4-phénylène, Q est O et T est un groupe alcoxy inférieur.

**21.** Dérivés de l'acide acétique selon la revendication 1, dans lesquels L est un groupe $L^5$, plus précisément de formule

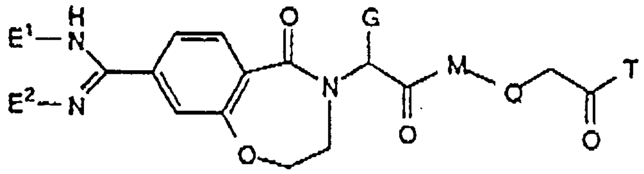

I·H

où $E^1$, $E^2$, G, M, Q et T ont les mêmes significations que dans la revendication 1, en particulier où M est un groupe 1,4-pipéridinylène lié au groupe céto par l'intermédiaire de l'atome d'azote, Q est O et T est un groupe alcoxy inférieur.

**22.** Composés selon l'une des revendications 1 à 21, pour utilisation en tant que principes actifs pharmaceutiques.

**23.** Procédé de préparation des composés selon la revendication 1, caractérisé en ce que

a) dans un composé de formule II

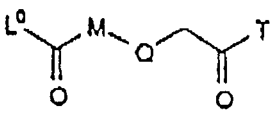

II

dans laquelle $L^0$ est un groupe ayant l'une des formules $L^{10}$ à $L^{50}$

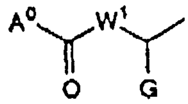

$L^{10}$

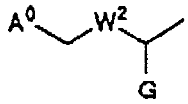

$L^{20}$

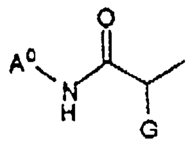

$L^{30}$

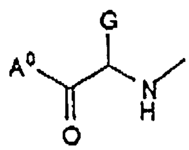

$L^{40}$

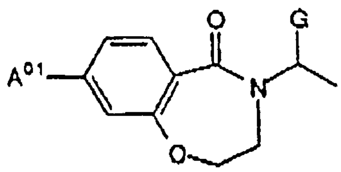

$L^{50}$

dans lesquelles $A^0$ est un groupe A contenant un groupe amino ou amidino protégé, et A, $W^1$, $W^2$, G, M, Q et T ont les mêmes signifcations que dans la revendication 1, et $A^{01}$ est un groupe amidino protégé, on dissocie le groupe amino ou amidino protégé, ou

b) dans un composé de formule III

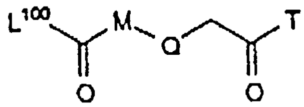

III

dans laquelle $L^{100}$ est un groupe ayant l'une des formules $L^{101}$ à $L^{501}$

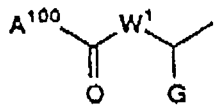

L101

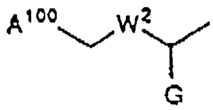

L201

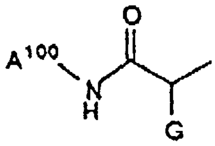

L301

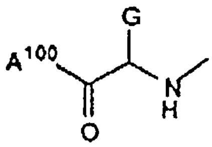

L401

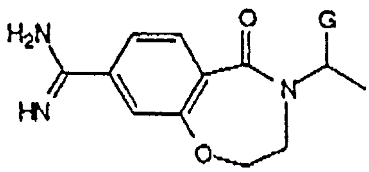

L501

dans lesquelles $A^{100}$ est un groupe A contenant un groupe amidino libre, et A, $W^1$, $W^2$, G, M, Q et T ont les mêmes significations que dans la revendication 1,
ou dans un sel de ce composé, on convertit le groupe amidino libre en un groupe amidino substitué par un groupe $E^1$ ou $E^2$, ou

c) dans un composé de formule IV

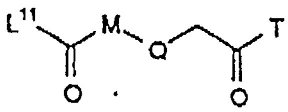

IV

dans laquelle $L^{11}$ est un groupe ayant l'une des formules $L^{111}$ à $L^{115}$

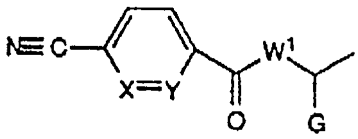

$L^{111}$

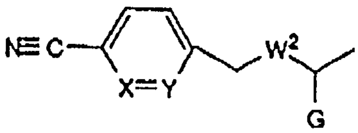

$L^{112}$

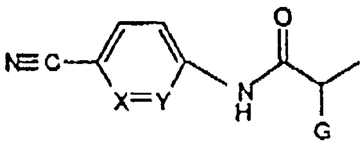

$L^{113}$

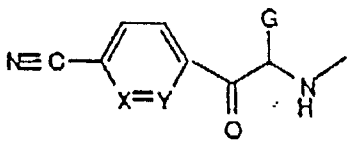

$L^{114}$

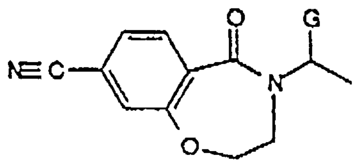

$L^{115}$

dans lesquelles X, Y, $W^1$, $W^2$, G, M, Q et T ont les mêmes significations que dans la revendication 1, on convertit le groupe cyano contenu dans $L^{11}$ en un groupe amidino éventuellement substitué par $E^1$ ou $E^2$, ou

d) on fait réagir une amine de formule

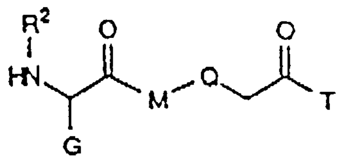

V

dans laquelle $R^2$ est H ou un groupe alkyle inférieur ou (alcoxy inférieur)(alkyle inférieur), et G, M, Q et T ont les mêmes significations que dans la revendication 1,
avec un acide A-COOH ou un dérivé fonctionnel de ce dernier, et

e) si on le souhaite, on soumet à une conversion fonctionnelle un groupe réactif contenu dans un composé de formule I, et
f) si on le souhaite, on convertit un composé de formule I en un sel compatible d'un point de vue physiologique,

ou on convertit un sel d'un composé de formule I en l'acide ou la base libre.

**24.** Préparations pharmaceutiques, en particulier pour le traitement ou la prophylaxie de maladies qui sont provoquées par la liaison de protéines adhésives à des plaquettes sanguines, ainsi que par une agrégation plaquettaire et une adhésion cellules-cellules, contenant en tant que principe actif un composé selon l'une des revendications 1 à 21.

**25.** Utilisation d'un composé selon l'une des revendications 1 à 21 pour préparer des médicaments destinés au traitement ou à la prophylaxie de maladies provoquées par la liaison de protéines adhésives à des plaquettes sanguines, et aussi par une agrégation plaquettaire et par une adhésion cellules-cellules, en particulier pour le traitement ou la prophylaxie de thromboses plaquettaires, de la thrombose, de l'accident vasculaire cérébral, de l'infarctus du myocarde, des inflammations, de l'artériosclérose ou de l'ostéoporose, ou encore en tant qu'agent antitumoral ou en tant qu'agent de cicatrisation.

**26.** Dérivé de l'acide acétique selon l'une des revendications 1 à 9, à savoir l'ester éthylique de l'acide (E/Z)-(S)-1-[2-[4-(aminohydroxyiminométhyl)-benzoylamino]-propionyl]-pipéridine-4-yloxyacétique.